(19) Europäisches Patentamt
European Patent Office
Office européen des brevets

(11) EP 2 170 903 B1

(12) **EUROPEAN PATENT SPECIFICATION**

(45) Date of publication and mention of the grant of the patent:
**05.09.2012 Bulletin 2012/36**

(21) Application number: **08773561.9**

(22) Date of filing: **20.06.2008**

(51) Int Cl.:
***C07D 495/20*** *(2006.01)* ***A61K 31/438*** *(2006.01)*
***A61P 25/18*** *(2006.01)*

(86) International application number:
**PCT/EP2008/005011**

(87) International publication number:
**WO 2008/155132 (24.12.2008 Gazette 2008/52)**

(54) **SPIRO[PIPERIDINE-4,4' -THIENO[3, 2-C]PYRAN]DERIVATIVES AND RELATED COMPOUNDS AS INHIBITORS OF THE SIGMA RECEPTOR FOR THE TREATMENT OF PSYCHOSIS**

SPIRO[PIPERIDIN-4,4'-THIENO[3,2-C]PYRAN]DERIVATE UND VERWANDTE VERBINDUNGEN ALS INHIBITOREN DES SIGMA-REZEPTORS ZUR BEHANDLUNG VON PSYCHOSEN

DÉRIVÉS DE SPIRO[PIPERIDIN-4,4'-THIENO[3,2-C]PYRAN] ET COMPOSÉS ASSOCIÉS UTILISÉS COMME INHIBITEURS DU RÉCEPTEUR SIGMA POUR LE TRAITEMENT DE LA PSYCHOSE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **20.06.2007 EP 07384027**

(43) Date of publication of application:
**07.04.2010 Bulletin 2010/14**

(73) Proprietor: **Laboratorios del. Dr. Esteve, S.A.**
**08041 Barcelona (ES)**

(72) Inventors:
- **OBERDORF, Christoph**
  **48161 Münster (DE)**
- **SCHEPMANN, Dirk**
  **48147 Münster (DE)**
- **WÜNSCH, Bernhard**
  **48161 Münster (DE)**
- **ZAMANILLO-CASTANEDO, Daniel**
  **E-08041 Barcelona (ES)**

(74) Representative: **Peters, Hajo et al**
**ZACCO GmbH**
**Bayerstrasse 83**
**80335 München (DE)**

(56) References cited:
**WO-A-01/12630 WO-A-97/11697**
**US-A- 4 021 451**

- **DATABASE CA [Online] CHEMICAL ABSTRACTS SERVICE, COLUMBUS, OHIO, US; 1988, SAUTER, FRITZ ET AL: "Thienospirans. V. Thienospirans via directed lithiations" XP002448343 retrieved from STN Database accession no. 1988:221581 & HETEROCYCLES , 26(10), 2639-56 CODEN: HTCYAM; ISSN: 0385-5414, 1987,**
- **DATABASE CA [Online] CHEMICAL ABSTRACTS SERVICE, COLUMBUS, OHIO, US; 1975, DOBSON, THOMAS A. ET AL: "Pyrano heterocycles. I. Syntheses of isochromans and the novel thieno[3,2-c]pyran, benzothieno[3,2-c] pyran, benzothieno[2,3- c]pyran, and pyrano[4,3-b]benzofuran systems" XP002448344 retrieved from STN Database accession no. 1975:514254 & JOURNAL OF HETEROCYCLIC CHEMISTRY , 12 (3), 591-4 CODEN: JHTCAD; ISSN: 0022-152X, 1975,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 170 903 B1

## Description

### FIELD OF THE INVENTION

The present invention relates to compounds having pharmacological activity towards the sigma (σ) receptor, and more particularly to some thieno-pyrano-pyrazole derivatives, to processes of preparation of such compounds, to pharmaceutical compositions comprising them, and to their use in therapy and prophylaxis, in particular for the treatment of psychosis.

### BACKGROUND OF THE INVENTION

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad Sci., 1996, 93:8072-8077). The existence of sigma receptors in the CNS, immune and endocrine systems have suggested a likelihood that it may serve as link between the three systems.

There is still a need to find compounds that have pharmacological activity towards the sigma receptor, being both effective and selective, and having good "drugability" properties, i.e. good pharmaceutical properties related to administration, distribution, metabolism and excretion.

The closest technique known comprises benzimidazoles of WO2003035065 for the inhibition of kinases. Tetrahydro-pyranopyrazole compounds displaying cannabinoid modulating activity are disclosed in W02007001939 and FR2875230. None of the them presents spiro-pyrano-pyrazole variants or analogues.

Spiropiperidines are known as potent ligands to sigma receptors (Maier et al, J Med Chem, 2002, 45, 438-448 and Maier et al, J Med Chem, 2002, 45, 4923-4930). However, such spiropiperidines show benzofuran and benzopyran rings.

### SUMMARY OF THE INVENTION

We have now found a family of structurally distinct thieno-pyrano-pyrazole derivatives which are particularly selective inhibitors of the sigma receptor.

The invention is directed to compounds of general formula (I) or (Ia),

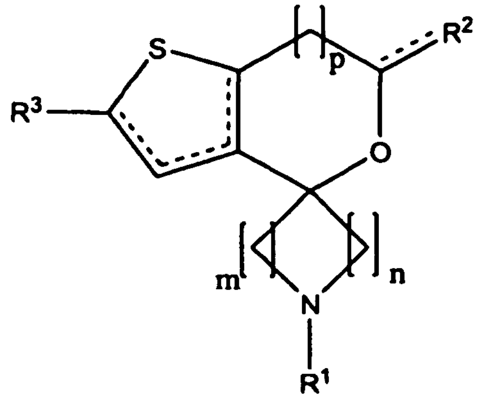

or

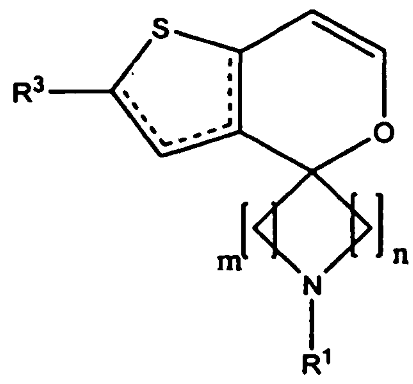

(I) (Ia)

wherein
m is selected from 1, 2 or 3, n is selected from 1, 2 or 3, and m + n is either 3, 4 or 5;
p is selected from 0 or 1;
the dotted lines are either a double or a single bond;
R’ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
$R^2$ is selected from hydrogen; an optionally at least monosubstituted, linear or branched $C_{1-6}$-aliphatic group; $(CH_2)_qCN$ with q being 0, 1, 2, 3 or 4; O-R with R being H or an optionally at least monosubstituted, linear or branched $C_{1-6}$- aliphatic group; =O; $(CH_2)_sCOR^*$; $(CH_2)_sNR^*R^{**}$; $CONR^*R^{**}$; $(CH_2)_sCO_2R^*$; CH=NOR*; $(CH_2)_sOR^*$:
with
R* being H; or an optionally at least monosubstituted, linear or branched $C_{1-6}$- aliphatic group;
R** being an optionally at least monosubstituted, linear or branched $C_{1-6}$- aliphatic group;
s being 0, 1, or 2;
$R^3$ is selected from hydrogen; an optionally at least monosubstituted, linear or branched $C_{1-18}$-aliphatic group; $NO_2$; $SO_3H$; COR"’; $(CH_2)_rNR'R''$; $CO_2R'$; an optionally at least monosubstituted alkyl-aryl with alkyl optionally being substituted by OH; an optionally at least monosubstituted alkyl-heterocyclyl with alkyl optionally being substituted by OH; or an optionally at least monosubstituted alkyl-cycloalkyl with alkyl optionally being substituted by OH;
with
R’ being H; or an optionally at least monosubstituted, linear or branched $C_{1-6}$-aliphatic group;
R" being an optionally at least monosubstituted, linear or branched $C_{1-6}$-aliphatic group;
R"’ being H; an optionally at least monosubstituted, linear or branched $C_{1-6}$-aliphatic group; or an optionally at least monosubstituted aryl, an optionally at least monosubstituted heterocyclyl, or an optionally at least monosubstituted cycloalkyl;
r being 0, 1, or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another embodiment the invention is also directed to compounds of general formula (I) or (Ia),

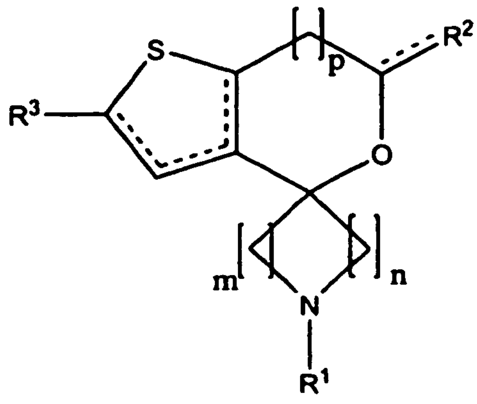

or

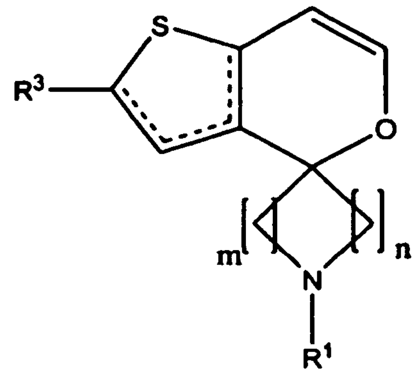

(I) (Ia)

wherein

m is selected from 1, 2 or 3, n is selected from 1, 2 or 3, and m + n is either 3, 4 or 5;

p is selected from 0 or 1;

the dotted lines are either a double or a single bond;

R' is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;

$R^2$ is selected from hydrogen; an optionally at least monosubstituted, linear or branched $C_{1-6}$-aliphatic group; $(CH_2)_qCN$ with q being 0, 1, 2, 3 or 4; O-R with R being H or an optionally at least monosubstituted, linear or branched $C_{1-6}$-aliphatic group; $(CH_2)_sCOR^*$; $(CH_2)_sNR^*R^*$.; $CONR^*R^{**}$; $(CH_2)_sCO_2R^*$; $CH{=}NOR^*$; $(CH_2)_sOR^*$;

with

R* being H; or an optionally at least monosubstituted, linear or branched $C_{1-6}$-aliphatic group;

R** being an optionally at least monosubstituted, linear or branched $C_{1-6}$-aliphatic group; or an optionally at least monosubstituted aryl or alkyl-aryl;

s being 0, 1, or 2;

$R^3$ is selected from hydrogen; halogen; an optionally at least monosubstituted, linear or branched $C_{1-18}$-aliphatic group; CN; CH(=NOH); $NO_2$; $SO_3H$; COR'''; $(CH_2)_rNR'R''$; CH(OR')(OR''); $CO_2R'$; an optionally at least monosubstituted alkyl-aryl with alkyl optionally being substituted by OH; an optionally at least monosubstituted alkyl-heterocyclyl with alkyl optionally being substituted by OH or forming a C(O)-keto-group; or an optionally at least monosubstituted alkyl-cycloalkyl with alkyl optionally being substituted by OH;

with

R' being H; or an optionally at least monosubstituted, linear or branched $C_{1-6}$-aliphatic group;

R'' being an optionally at least monosubstituted, linear or branched $C_{1-6}$-aliphatic group;

R''' being H; an optionally at least monosubstituted, linear or branched $C_{1-6}$-aliphatic group; or an optionally at least monosubstituted aryl, an optionally at least monosubstituted heterocyclyl, or an optionally at least monosubstituted cycloalkyl;

r being 0, 1, or 2;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;

with the following proviso applying:

- if $R^2$ and $R^3$ are both hydrogen, R' may not be methyl.

In the context of this invention, alkyl radical or group is understood as meaning saturated and unsaturated, linear or branched hydrocarbons, which can be unsubstituted or mono- or polysubstituted. Thus unsaturated alkyl is understood to encompass alkenyl and alkinyl groups, like e.g. $-CH{=}CH-CH_3$ or $-C-C-CH_3$, while saturated alkyl encompasses e.g. $-CH_3$ and $-CH_2-CH_3$. In these radicals, $C_{1-2}$-alkyl represents C1- or C2-alkyl, $C_{1-3}$-alkyl represents C1-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents C1-, C2-, C3-, C4-, C5-, C6- or C7-alkyl, $C_{1-8}$-alkyl

represents C1-, C2-, C3-, C4-, C5-, C6-, C7- or C8-alkyl, $C_{1-10}$-alkyl represents C1-, C2-, C3-, C4-, C5-, C6-, C7-, C8-, C9-or C10-alkyl and $C_{1-18}$-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also $CHF_2$, $CF_3$ or $CH_2OH$ etc.

In the context of this invention aliphatic group or radical includes alkyl (saturated), alkenyl (unsaturated alkyl) and alkinyl (unsaturated alkyl) and thus is synonymous for: saturated or unsaturated alkyl (see above).

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, $C_{3-4}$-cycloalkyl represents C3- or C4-cycloalkyl, $C_{3-5}$-cycloalkyl represents C3-, C4- or C5-cycloalkyl, $C_{3-6}$-cycloalkyl represents C3-, C4-, C5- or C6-cycloalkyl, $C_{3-7}$-cycloalkyl represents C3-, C4-, C5-, C6- or C7-cycloalkyl, $C_{3-8}$-cycloalkyl represents C3-, C4-, C5-, C6-, C7- or C8-cycloalkyl, $C_{4-5}$-cycloalkyl represents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{5-6}$-cycloalkyl represents C5- or C6-cycloalkyl and $C_{5-7}$-cycloalkyl represents C5-, C6- or C7-cycloalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

In the context of this invention alkyl-cycloalkyl is understood as meaning a cycloalkyl group (see above) being connected to another atom through a $C_{1-6}$-alkyl group (see above), whereas the $C_{1-6}$-alkyl-group is always saturated and unsubstituted, and linear or branched.

In connection with alkyl or aliphatic group - unless defined otherwise - the term substituted in the context of this invention is understood as meaning replacement of at least one hydrogen radical by F, Cl, Br, I, $NH_2$, SH or OH, "polysubstituted" radicals being understood as meaning that the replacement takes effect both on different and on the same atoms several times with the same or different substituents, for example three times on the same C atom, as in the case of $CF_3$, or at different places, as in the case of e.g. $-CH(OH)-CH=CH-CHCl_2$.

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$. $(CH_2)_{4-5}$ is to be understood as meaning $-CH_2-CH_2-CH_2-CH_2-$ and $-CH_2-CH_2-CH_2-CH_2-CH_2-$, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through a $C_{1-6}$-alkyl-group (see above), whereas the $C_{1-6}$-alkyl-group is always saturated and unsubstituted, and linear or branched.

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through a $C_{1-6}$-alkyl group (see above), whereas the $C_{1-6}$-alkyl-group is always saturated and unsubstituted, and linear or branched.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, $NO_2$, COOH; $NR_xR_y$, with $R_x$ and $R_y$ independently being either H or a saturated or unsaturated, linear or branched, substituted or unsubstituted $C_{1-6}$-alkyl; a saturated or unsaturated, linear or branched, substituted or unsubstituted $C_{1-6}$-alkyl; a saturated or unsaturated, linear or branched, substituted or unsubstituted $-O-C_{1-6}$-alkyl (alkoxy); a saturated or unsaturated, linear or branched, substituted or unsubstituted $-S-C_{1-6}$-alkyl; a saturated or unsaturated, linear or branched, substituted or unsubstituted - $C(O)-C_{1-6}$-alkyl-group; a saturated or unsaturated, linear or branched, substituted or unsubstituted $-C(O)-O-C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl.

The term "salt" is to be understood as meaning any form of the active compound used according to the invention

in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono or (di)sodium, (mono or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates.

Another preferred embodiment of the invention refers to a compound of general formula Ic,

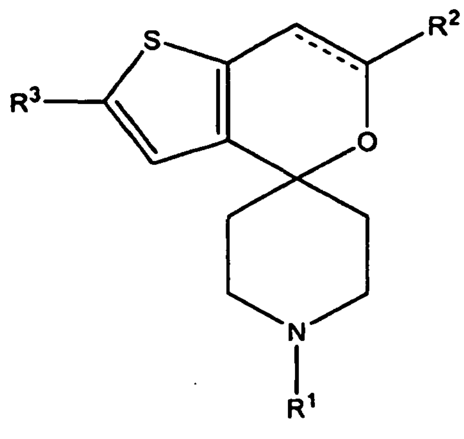

**(Ic)**

wherein

m is selected from 1, 2 or 3, n is selected from 1, 2 or 3, and m + n is either 3, 4 or 5;

p is selected from 0 or 1;

the dotted lines are either a double or a single bond;

R' is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;

$R^2$ is selected from hydrogen; an optionally at least monosubstituted, linear or branched $C_{1-6}$-aliphatic group; $(CH_2)_qCN$ with q being 0, 1, 2, 3 or 4; O-R with R being H or an optionally at least monosubstituted, linear or branched $C_{1-6}$-aliphatic group; $(CH_2)_sCOR^*$; $(CH_2)_sNR^*R^{**}$; $CONR^*R^{**}$; $(CH_2)_sCO_2R^*$; CH=NOR*; $(CH_2)_sOR^*$;

with

R* being H; or an optionally at least monosubstituted, linear or branched $C_{1-6}$-aliphatic group;

R** being an optionally at least monosubstituted, linear or branched $C_{1-6}$-aliphatic group; or an optionally at least monosubstituted aryl or alkyl-aryl;

s being 0, 1, or 2;

$R^3$ is selected from hydrogen; halogen; an optionally at least monosubstituted, linear or branched $C_{1-18}$-aliphatic group; CN; CH(=NOH); $NO_2$; $SO_3H$; COR'''; $(CH_2)_rNR'R''$; CH(OR')(OR''); $CO_2R'$; an optionally at least monosubstituted alkyl-aryl with alkyl optionally being substituted by OH; an optionally at least monosubstituted alkyl-heterocyclyl with alkyl optionally being substituted by OH or forming a C(O)-keto-group; or an optionally at least monosubstituted alkyl-cycloalkyl with alkyl optionally being substituted by OH;

with

R' being H; or an optionally at least monosubstituted, linear or branched $C_{1-6}$-aliphatic group;

R'' being an optionally at least monosubstituted, linear or branched $C_{1-6}$-aliphatic group;

R''' being H; an optionally at least monosubstituted, linear or branched $C_{1-6}$-aliphatic group; or an optionally at least monosubstituted aryl, an optionally at least monosubstituted heterocyclyl, or an optionally at least monosubstituted cycloalkyl;

r being 0, 1, or 2;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In one embodiment, one or both of the following provisos apply:

- if $R^2$ and $R^3$ are both hydrogen, R' may not be methyl; and /or
- if $R^2$ and $R^3$ are both hydrogen, R' may not be hydrogen.

In a preferred embodiment of the compound according to the invention according to general formula (I), (Ia) or (Ic) R' is selected from H; an optionally at least monosubstituted, linear or branched $C_{1-18}$-aliphatic group; an optionally at least monosubstituted alkyl-aryl; an optionally at least monosubstituted alkyl-heterocyclyl; or an optionally at least monosubstituted alkyl-cycloalkyl.

In another preferred embodiment of the compound according to the invention according to general formula (I) or (Ia) $R^2$ is selected from H; $(CH_2)_qCN$ with q being 0, 1, 2, 3 or 4; or OR with R being H or an optionally at least monosubstituted, linear or branched $C_{1-6}$-aliphatic group; preferably $R^2$ is selected from H; CN; $CH_2CN$; or OR with R being H or a linear or branched $C_{1-4}$-alkyl group, more preferably $R^2$ is selected from H, CN; $CH_2CN$; OH or $OCH_3$.

In another preferred embodiment of the compound according to the invention according to general formula (I), (Ia) or (Ic) $R^2$ is selected from hydrogen; an optionally at least monosubstituted, linear or branched $C_{1-6}$-aliphatic group; or OR with R being H or an optionally at least monosubstituted, linear or branched $C_{1-6}$-aliphatic group; $(CH_2)_q$-CN, $(CH_2)_q$-NH-benzyl; $(CH_2)_q$-$N(R^{22})_2$; $(CH_2)_q$-C(O)-$N(R^{22})_2$; $(CH_2)_q$-C(O)-$R^{22}$ or $(CH_2)_qC(O)$-O-$R^{22}$, with q being 0, or 1 and $R^{22}$ being H; or an optionally at least monosubstituted, linear or branched $C_{1-6}$-aliphatic group;.

In another preferred embodiment of the compound according to the invention according to general formula (I) or (Ia) $R^3$ is selected from H; or an optionally at least monosubstituted, linear or branched $C_{1-6}$-aliphatic group; especially $R^3$ is selected from H; or an optionally at least monosubstituted, linear or branched $C_{1-6}$-alkyl group.

In another preferred embodiment of the compound according to the invention according to general formula (I), (Ia) or (Ic) $R^3$ is selected from hydrogen; halogen; CN; CH(=NOH); an optionally at least monosubstituted, linear or branched $C_{1-10}$-aliphatic group; $CH_2$-$R^{33}$; C(O)-$R^{33}$; C(O)O-$R^{33}$; CHOH-$R^{33}$; $CH_2N(R^{33})_2$ with $R^{33}$ being H; or an optionally at least monosubstituted, linear or branched $C_{1-6}$-aliphatic group; or an optionally at least monosubstituted aryl.

In another preferred embodiment of the compound according to the invention according to general formula (I) or (Ia) m and n are selected from 1 or 2 and m+n are selected from 3 or 4.

In another very preferred embodiment of the compound according to the invention the compounds are compounds according to general formula (Id)

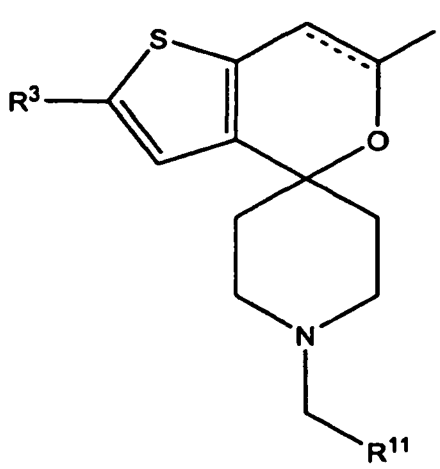

(Id)

wherein

the bond with the dotted line ........ either is a double or a single bond;

R" is selected from an optionally at least monosubstituted, linear or branched $C_{1-9}$-aliphatic group; an optionally at least monosubstituted aryl or alkyl-aryl; an optionally at least monosubstituted heterocyclyl or alkyl-heterocyclyl; an optionally at least monosubstituted cycloalkyl or alkyl-cycloalkyl;

$R^2$ is selected from hydrogen; an optionally at least monosubstituted, linear or branched $C_{1-6}$-aliphatic group; or OR with R being H or an optionally at least monosubstituted, linear or branched $C_{1-6}$-aliphatic group; $(CH_2)_q$-CN, $(CH_2)_q$-NH-benzyl; $(CH_2)_q$-$N(R^{22})_2$; $(CH_2)_q$-C(O)-$N(R^{22})_2$; $(CH_2)_q$-C(O)-$R^{22}$ or $(CH_2)_q$-C(O)-$OR^{22}$, with q being 0, or 1 and $R^{22}$ being H; or an optionally at least monosubstituted, linear or branched $C_{1-6}$-aliphatic group;

$R^3$ is selected from hydrogen; halogen; CN; CH(=NOH); an optionally at least monosubstituted, linear or branched $C_{1-10}$-aliphatic group; $CH_2$-$R^{33}$; C(O)-$R^{33}$; C(O)O-$R^{33}$; CHOH-$R^{33}$; $CH_2N(R^{33})_2$ with $R^{33}$ being H; an optionally at least monosubstituted, linear or branched $C_{1-6}$-aliphatic group; or an optionally at least monosubstituted aryl;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a preferred embodiment of the compounds according to general formula (Id) R" is selected from an optionally at least monosubstituted, linear or branched $C_{1-9}$-aliphatic group; an optionally at least monosubstituted aryl or alkyl-aryl; an optionally at least monosubstituted heterocyclyl; or an optionally at least monosubstituted cycloalkyl; preferably is phenyl, p-methoy-phenyl, cyclohexyl, thiophene, benzyl; branched or linear $C_{3-7}$-alkyl; or branched or linear $C_{3-7}$-alkenyl.

In a preferred embodiment of the compounds according to general formula (Id) $R^2$ is selected from hydrogen; an unsubstituted or by at least one of OH, F, or Cl substituted, linear or branched $C_{1-6}$-aliphatic group; or OR with R being H or CH3; $(CH_2)_q$-CN, $(CH_2)_q$-NH-benzyl; $(CH_2)_q$-$N(R^{22})_2$; $(CH_2)_q$-C(O)-$N(R^{22})_2$; $(CH_2)_4$-C(O)-$R^{22}$ or $(CH_2)_q$-C(O)-O-$R^{22}$, with q being 0, or 1 and $R^{22}$ being H, $CH_3$, or $C_2H_5$.

In a preferred embodiment of the compounds according to general formula (Id) $R^3$ is selected from hydrogen; halogen; CN; CH(=NOH); a linear or branched, unsubstituted or by at least one of OH, Cl, or F substituted $C_{1-6}$-aliphatic group; $CH_2$-$R^{33}$; C(O)-$R^{33}$; C(O)O-$R^{33}$; CHOH-$R^{33}$; $CH_2N(R^{33})_2$ with $R^{33}$ being H; $CH_3$; $C_2H_5$; or phenyl.

In another very preferred embodiment of the compound according to the invention the compounds are compounds according to general formula (Ib)

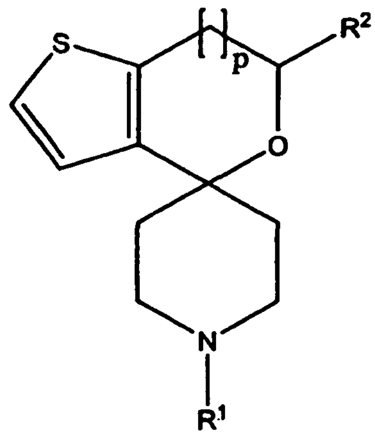

(Ib)

wherein
p is selected from 0 or 1;
$R^1$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; an optionally at least monosubstituted alkyl-cycloalkyl; or COOR' with R' being either H or $C_{1-4}$-alkyl;
$R^2$ is selected from hydrogen; an optionally at least monosubstituted, linear or branched $C_{1-6}$-aliphatic group; $(CH_2)_qCN$ with q being 0, or 1; or OR with R being H or an optionally at least monosubstituted, linear or branched $C_{1-6}$-aliphatic group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the compound according to the invention according to general formula (Ib) $R^2$ is selected from H; $CH_2CN$; CN; or OR with R being H or an optionally at least monosubstituted, linear or branched $C_{1-6}$-alkyl group; especially $R^2$ is selected from H; $CH_2CN$; CN; OH or a linear or branched $OC_{1-4}$-alkyl group; more especially $R^2$ is selected from H, CN, $CH_2CN$, OH or $OCH_3$.

In another preferred embodiment of the compound according to the invention according to general formula (Ib) R' is selected from hydrogen; an optionally at least monosubstituted, linear or branched $C_{1-18}$-aliphatic group; an optionally at least monosubstituted alkyl-aryl; an optionally at least monosubstituted alkyl-heterocyclyl; or an optionally at least monosubstituted alkyl-cycloalkyl.

In another preferred embodiment of the compound according to the invention according to general formula (Ib) R' is selected from
hydrogen, $C_{1-10}$-alkyl, linear or branched; $C_{1-10}$-alkenyl, linear or branched; optionally at least mono-substituted $C_{1-6}$-alkyl-aryl; optionally at least mono-substituted $C_{1-6}$-alkyl-heterocyclyl; or optionally at least monosubstituted $C_{1-6}$-alkyl-cycloalkyl;
preferably R' is selected from hydrogen; $C_{1-10}$-alkyl, linear or branched; $C_{2-8}$-alkenyl, linear or branched; optionally at least mono-substituted $C_{16}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl.

In another highly preferred embodiment of the compound according to the invention the compounds are compounds according to general formula (Ib)

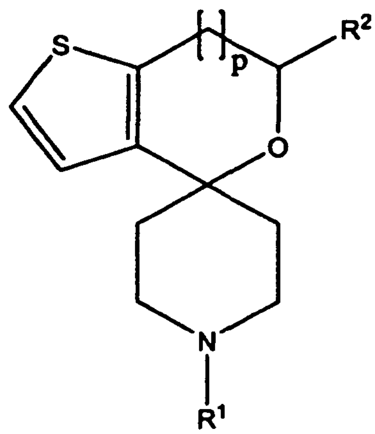

**(Ib)**

wherein
p is selected from 0 or 1;
R' is selected from hydrogen; $C_{1-10}$-alkyl, linear or branched; $C_{2-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl;
$R^2$ is selected from H; CN; $CH_2CN$; OH or a linear or branched $OC_{1-4}$-alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the compound according to the invention the compounds according to general formula (I) or (Ia) are selected from:

- Ethyl-6'-methoxy-6',7'-dihydrospiro[piperidin-4,4'-thieno[3,2-c]pyran]-1-carboxylate;
- 6'-Methoxy-6',7'-dihydrospiro[piperidin-4,4'-thieno[3,2-c]pyran;
- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyt)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];
- 3'-Methoxy-1-(4-phenylbutyl)-6',7'-dihydrospiro[piperidin-4,4'-thieno[3.2-c]pyran];
- 1-(4-Fluorbenzyl)-6'-Methoxy -6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'-dihydrospiro[piperidin-4,4'-thieno[3.2-c]pyran];
- 1-Benzylspiro[piperidin-4,4'-thieno[3,2-c]pyran];
- 1-Benzyl-6',7'-dihydrospiro[piperidin-4,4'-thieno[3,2-c]pyran]-6'-carbonitrile;
- {1-Benzyl-6',7'-dihyrospiro[piperidin-4,4'-thieno[3,2-c]pyran]-6'-yl}acetonitrile;
- 1-Benzyl-6',7'-dihydrospiro[piperidin4,4'-thieno[3.2-c]pyran]; or
- 1-Benzyl-6',7'-dihydrospiro[piperidin-4,4'-thieno[3,2-c]pyran]-6'-ol;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
preferably from

- 1-Benzyl-6'-methoxy-6',7'-dihydrospiro[piperidin-4,4'-thieno[3.2-c]pyran];

- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];
- 3'-Methoxy-1-(4-phenylbutyl)-6',7'-dihydrospiro[piperidin-4,4'-thieno[3.2-c]pyran];
- 1-(4-Fluorbenzyl)-6'-Methoxy -6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-clpyran];
- 6'-Methoxy-1-(thien-2-ylmethyl)-6',7'-dihydrospiro[piperidin-4,4'-thieno[3.2-clpyran];
- 1-Benryl-6',7'-dihydrospiro[piperidin-4,4'-thieno[3,2-c]pyran]-6'-carbonitrile;
- {1-Benzyl-6',7'-dihyrospiro[piperidin-4,4'-thieno[3,2-c]pyran]-6'-yl}acetonitrile;
- 1-Benzyl-6',7'-dihydrospiro[piperidin-4,4'-thieno[3,2-c]pyran]; or
- 1-Benzyl-6',7'-dihydrospiro[piperidin-4,4'-thieno[3,2-c]pyran]-6'-ol;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the compound according to the invention the compounds according to general formula (I), (Ia), (Ic) or (Id) are selected from:

- 6'-Methoxy-6',7'-dihydrospiro[piperidin-4,4'-thieno[3,2-c]pyran;
- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4,4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'-thieno[3.2-c]pyran];

- 3'-Methoxy-1-(4-phenylbutyl)-6',7'-dihydrospiro[piperidin-4,4'-thieno[3.2-c]pyran];
- 1-(4-Fluorbenzyl)-6'-Methoxy -6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'-dihydrospiro[piperidin-4,4'-thieno[3.2-c]pyran];
- 1-Benzylspiro[piperidin-4,4'-thieno[3,2-c]pyran];
- 1-Benzyl-6',7'-dihydrospiro[piperidin-4,4'-thieno[3,2-c]pyran]-6'-carbonitril;
- {1-Benzyl-6',7'-dihyrospiro[piperidin-4,4'-thieno[3,2-c]pyran]-6'-yl}acetonitril;
- 1-Benzyl-6',7'-dihydrospiro[piperidin-4,4'-thieno[3,2-c]pyran];
- 1-Benzyl-6',7'-dihydrospirolpiperidin-4,4'-thieno[3,2-c]pyran]-6'-ol
- 1-(Cyclohexylmethyl)spiro[piperidine-4,4'-thieno[3,2-c]pyrano]
- 1-(Cyclohexylmethyl)-6',7'-dihydrospiro[piperidine-4,4'-thieno[3,2-c]pyrano]-6'-carbonitrile
- 1-(1-Benzyl-6',7'-dihydrospiro[piperidine-4,4'-thieno[3,2-c]pyrano]-6'-yl)acetone
- Methyl-2-(1-benzyl-6',7'-dihyrospiro[piperidin-4.4'-thieno[3,2-c]pyrano]-6'-yl)-acetate
- Ethyl-2-(1-benzyl-6',7'-dihyrospiro[piperidine-4,4'-thieno[3,2-c]pyrano]-6'-yl)acetate
- 1-(Cyclohexylmethyl)-6',7'-dihydrospiro[piperidine-4,4'-thieno[3,2-c]pyrano]
- 2-(1-Benzyl-6',7'-dihyrospiro[piperidine-4,4'-thieno[3,2-c]pyrano]-6'-yl)ethanol
- 1-Benzyl-6',7'-dihyrospiro[piperidine-4,4'-thieno[3,2-c]pyrano]-6'-carboxamide
- 1-Benzyl-6',7'-dihyrospiro[piperidine-4,4'-thieno[3,2-c]pyrano]-6'-carbaldehyde
- (1-Benzyl-6',7'-dihydrospiro[piperidine-4,4'-thieno[3,2-c]pyrano]-6'-yl)methanamine
- Methyl-1-benzyl-6',7'-dihyrospiro[piperidine-4,4'-thieno[3,2-c]pyrano]-6'-carboxylate
- Etyhl-1-benzyl-6',7'-dihyrospiro[piperidine-4,4'-thieno[3,2-c]pyrano]-6'-carboxylate
- (1-Benzyl-6',7'-dihyrospiro[piperidine-4,4'-thieno[3,2-c]pyrano]-6'-yl)methanol
- (1-Benzyl-6',7'-dihydrospiro[piperidine-4,4'-thieno[3,2-c]pyrano]-6'-yl)-N,N-dimethylmethanamine
- (1-Benzyl-6',7'-dihydrospiro[piperidine-4,4'-thieno[3,2-c]pyrano]-6'-yl)-N-benzyl-methanamine
- (1-Benzyl-6'-methoxy-6',7'-dihydrospiro[piperidine-4,4'-thieno[3.2-c]pyrano]-2-carbaldehyde-dimethylacetal
- 1-Benzyl-2'-chloro-6'-methoxy-6',7'-dihydrospiro[piperidine-4,4'-thieno[3.2-c]-pyrano]
- 1,2'-Dibenzyl-6'-methoxy-6',7'-dihydrospiro[piperidine-4,4'-thieno[3.2-c]pyrano]
- (1-Benzyl-6'-methoxy-6',7'-dihydrospiro[piperidine-4,4'-thieno[3.2-c]pyrano]-2-carbaldehyde
- (1-Benzyl-6'-methoxy-6',7'-dihydrospiro[piperidine-4,4'-thieno[3.2-c]pyrano]-2'-yl)-methanol

- (1-Benzyl-6'-methoxy-6',7'-dihydrospiro[piperidine-4,4'-thieno[3.2-c]pyrano]-2'-yl)-N,N-dimethylmethanamine
- (E/Z)-1-Benzyl-6'-methoxy-6',7'-dihydrospiro[piperidine-4,4'-thieno[3,2-c]pyrano]-2-carbaldehydoxim
- 1-Benzyl-6'-methoxy-6',7'-dihydrospiro[piperidine-4,4'-thieno[3,2-c]pyrano]-2-carbonitrile
- Methyl-1-benzyl-6'-methoxy-6',7'-dihydrospiro[piperidine-4,4'-thieno[3,2-c]pyrano] -2-carboxylate
- 1-(1-Benzyl-6'-methoxy-6',7'-dihydrospiro[piperidine-4,4'-thieno[3.2-c]pyrano]-2-yl)-1-phenylmethanol
- 1-(1-Benzyl-6'-methoxy-6',7'-dihydrospiro[piperidine-4,4'-thieno[3.2-c]pyrano]-2-yl)-1-phenylmethanone;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

In general the processes are described below in the experimental part. The starting materials are commercially available or can be prepared by conventional methods.

A preferred aspect of the invention is also a process for the production of a compound according to the invention according to general formula (I), wherein a compound of formula (III)

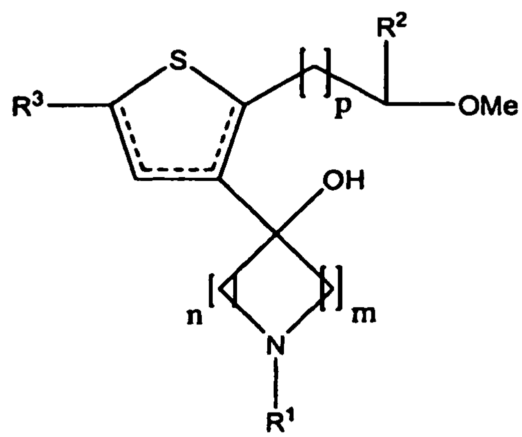

**(III)**

, wherein $R^1$, $R^2$, $R^3$, m, n and p are as defined above, is reacted with p-toluol sulphonic acid to form acompound according the formula I. It is preferred if in this process

- $R^2$ is $OCH_3$; and/or
- $R^3$ is H and/or
- p is H.

A preferred aspect of the invention is also a process for the production of a compound according to the invention according to general formula (Ib), wherein a compound of formula (IIIb)

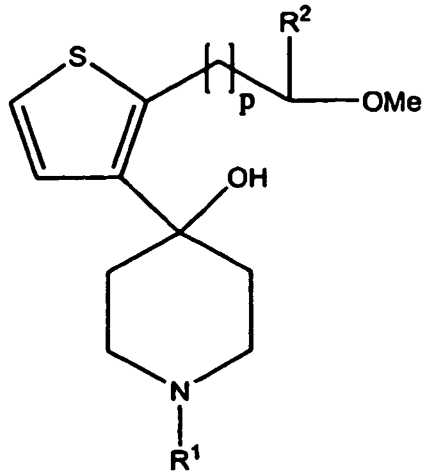

(IIIb)

, wherein $R^1$, $R^2$ and p are as defined above is reacted with p-toluol sulphonic acid to form acompound according the formula (Ib). It is preferred if

- $R^2$ is $OCH_3$ and/or
- p is 1.

It is further preferred if in the process above as a next step a compound according to formula (I) or (Ib) in which $R^3$ is C(O)OR' is reacted with a alkaline solution in water to form a compound according to formulas (I) or (Ib) with $R^1$ being H.

The obtained reaction products may, if desired, be purified by conventional methods, such as crystallisation and chromatography. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

Another aspect of the invention refers to a pharmaceutical composition which comprises a compound according to the invention or a pharmaceutically acceptable salt, isomer or solvate thereof, and a pharmaceutically acceptable carrier, adjuvant or vehicle. The present invention thus provides pharmaceutical compositions comprising a compound of this invention, or a pharmaceutically acceptable salt, derivative, prodrug or stereoisomers thereof together with a pharmaceutically acceptable carrier, adjuvant, or vehicle, for administration to a patient. This aspect also refers to a pharmaceutical composition which comprises a compound according to the invention or a pharmaceutically acceptable salt or solvate thereof, and a pharmaceutically acceptable carrier, adjuvant or vehicle. The present invention thus also provides pharmaceutical compositions comprising a compound of this invention, or a pharmaceutically acceptable salt thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation

and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to the use of a compound according to the invention in the manufacture of a medicament.

Another aspect of the invention refers to the use of a compound according to the invention in the manufacture of a medicament for the treatment or prophylaxis of a sigma receptor mediated disease or condition. A parallel aspect of the invention refers to the use of a compound according to the invention for the treatment or prophylaxis of a sigma receptor mediated disease or condition. A preferred embodiment of this is this use wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer.

A preferred embodiment of this is this use wherein the disease is pain, especially neuropathic pain, inflammatory pain or other pain conditions, allodynia and/or hyperalgesia, especially mechanical allodynia.

The compounds of the invention may be synthesized following one of the Reaction Schemes A, B, C, D, E or F set out below:

**Reaction Scheme A:** (with EX meaning „Example")

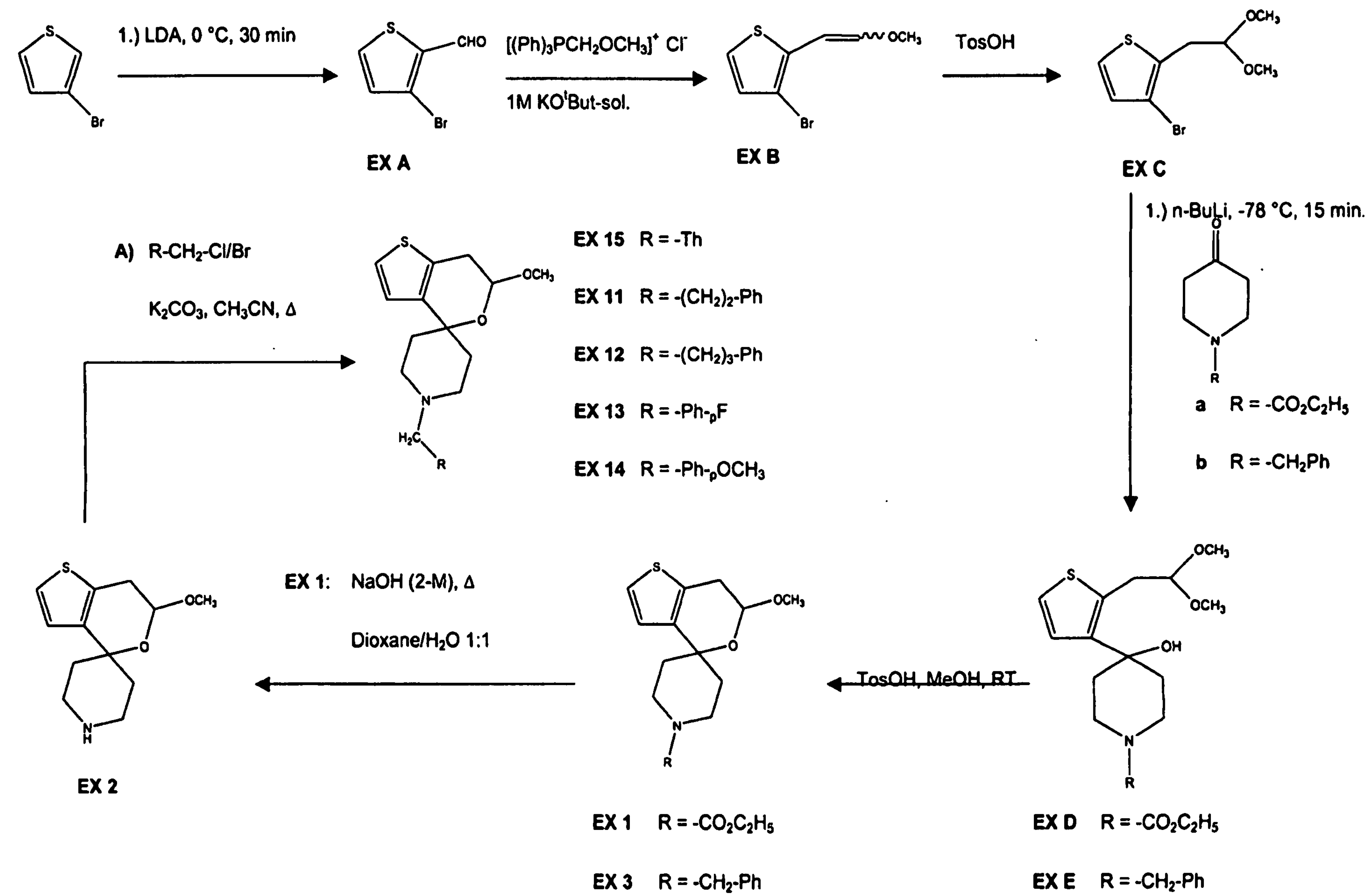

**Reaction Scheme B:** (with EX meaning „Example")

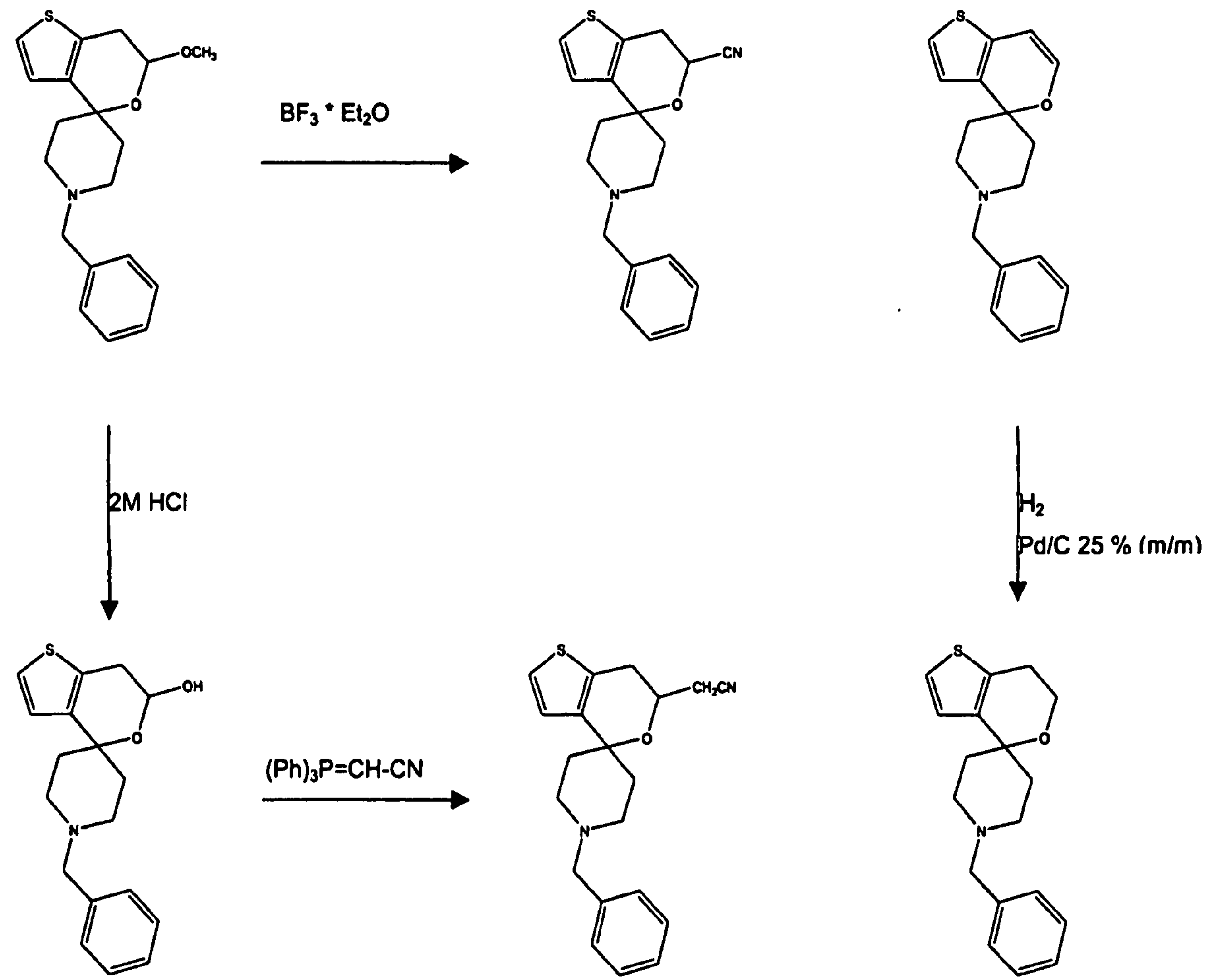

**Synthese Thienopyrane C** (with EX meaning „Example“)

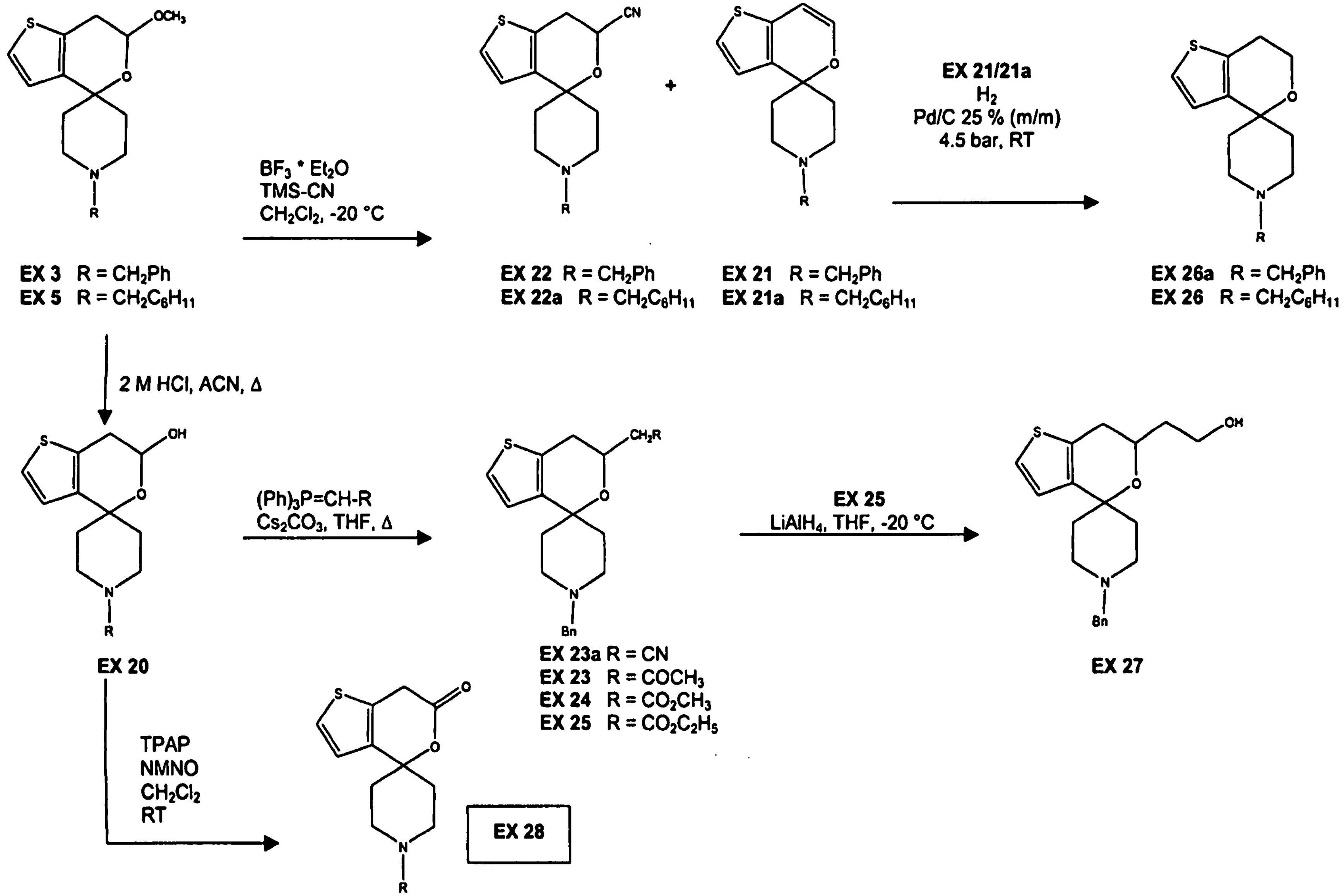


**EX 3** R = $CH_2Ph$
**EX 5** R = $CH_2C_6H_{11}$

**EX 22** R = $CH_2Ph$
**EX 22a** R = $CH_2C_6H_{11}$

+

**EX 21** R = $CH_2Ph$
**EX 21a** R = $CH_2C_6H_{11}$

**EX 26a** R = $CH_2Ph$
**EX 26** R = $CH_2C_6H_{11}$

**EX 20**

**EX 23a** R = CN
**EX 23** R = $COCH_3$
**EX 24** R = $CO_2CH_3$
**EX 25** R = $CO_2C_2H_5$

**EX 27**

**EX 28**

**Synthese Thienopyrane D** (with EX meaning „Example“)

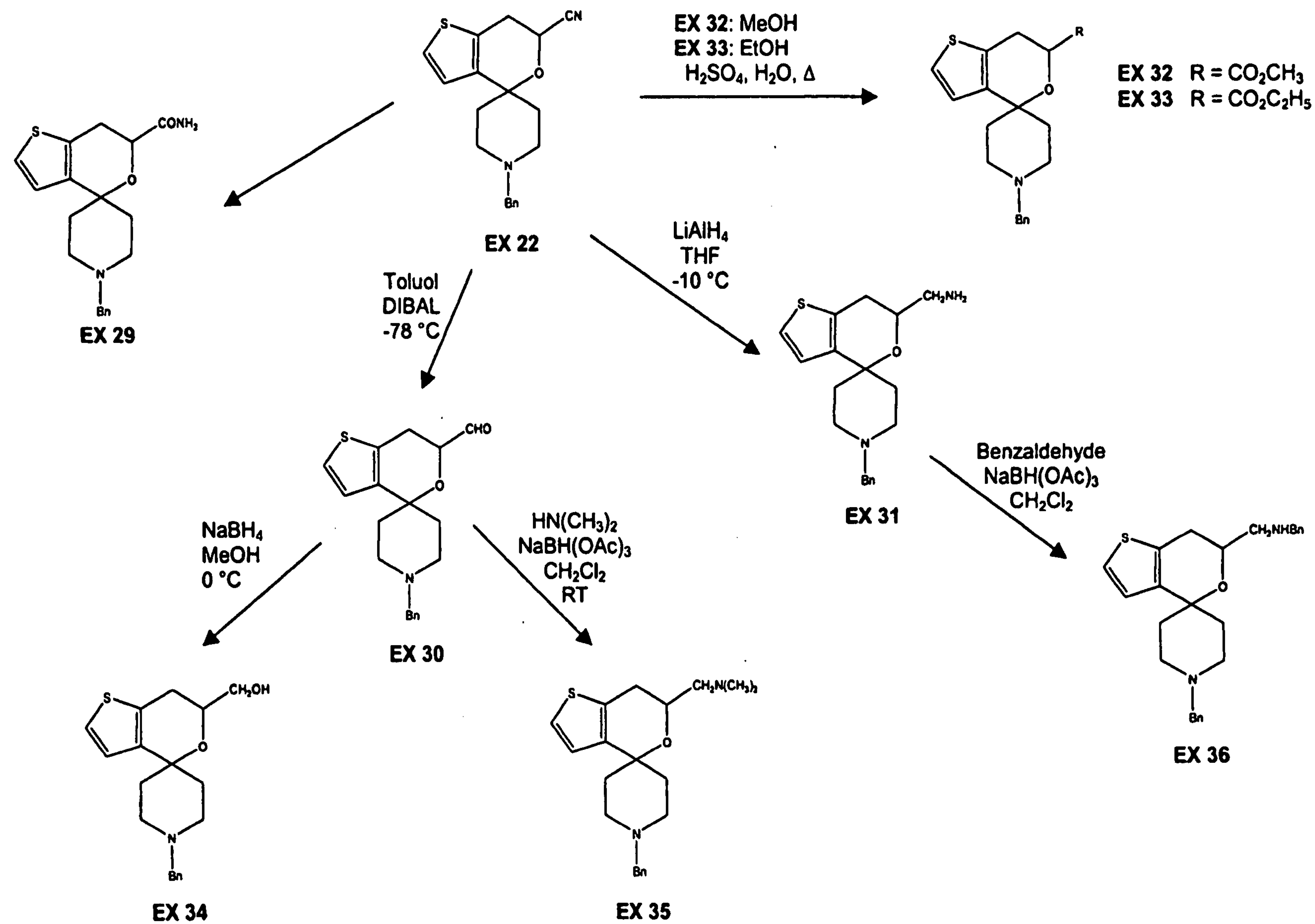

**Synthese Thienopyrane E** (with EX meaning „Example")

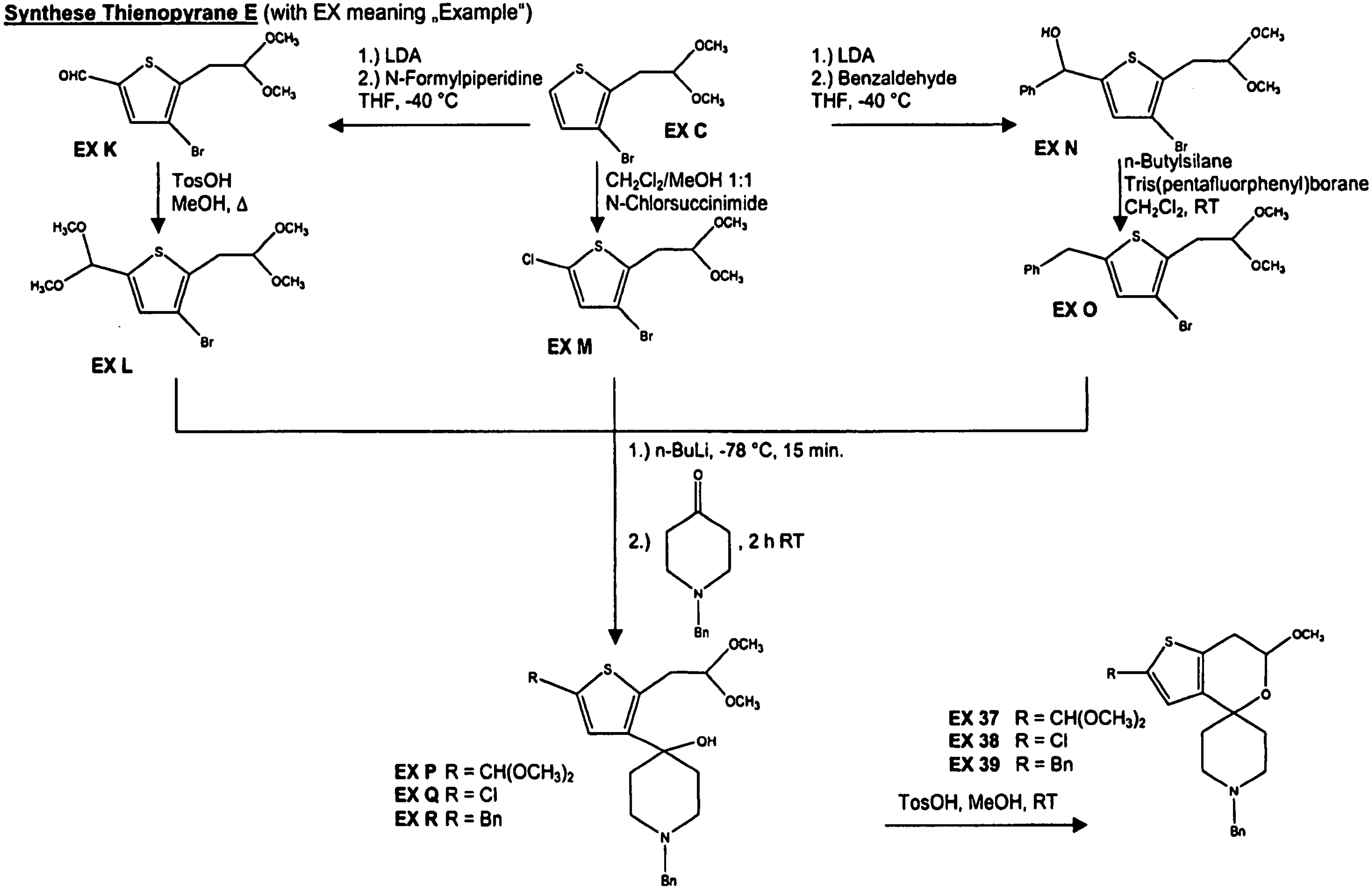

**Synthese Thienopyrane F** (with EX meaning „Example")

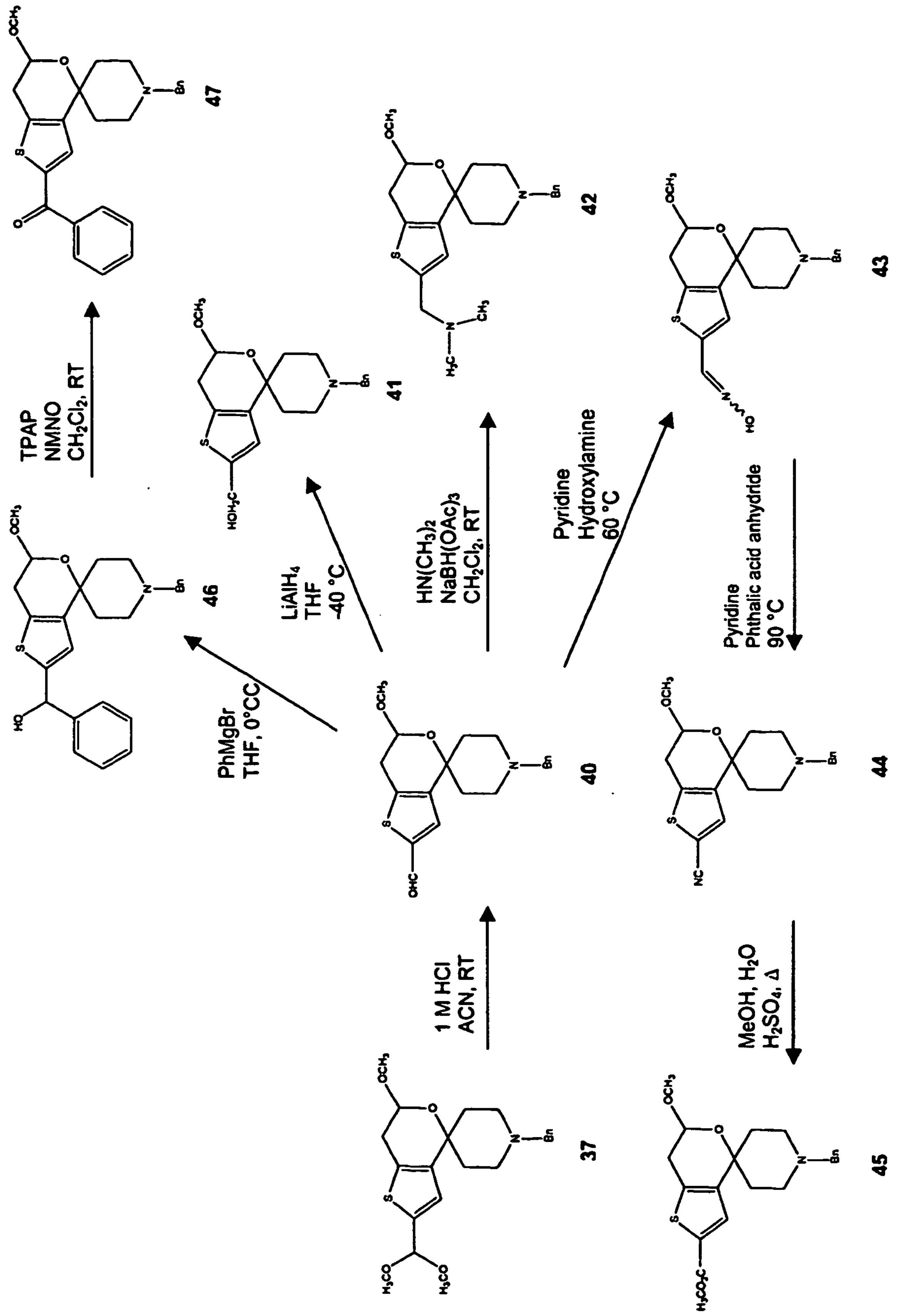


The present invention is illustrated below with the aid of examples. These illustrations are given solely by way of example and do not limit the general spirit of the present invention.

## EXAMPLES:

### General Experimental Part (Methods and Equipment of the synthesis and analysis

All solvents used for synthesis were p. a. quality.

The separation of mixtures of substances using thin-layer chromatography was done on glass plates layered by silica gel. Analysis of the plates was done after treatment with iodine gas or incubation with Dragendorff's reagent under UV light.

As a rule synthesized products were purified using flash-chromatography.

Melting points were measured using capillaries. As sometimes the products were mixtures of diastereoisomers, themelting point: had to be expressed as a range.

IR-spectra were measured using the FT-IR-480 Plus Fourier Transform Spectrometer with ATR (Fa. Jasco). All substances were either measured directly as solids or in oil.

NMR-Spectra were measured using Mercury-400BB (Fa. Varian) at a temperature of 21˚C. δ, measured in ppm, is based on the signal TMS measured in comparison to the residue signal ($CHCl_3$) of the solvent ($CDCl_3$):

$^1$H-NMR-Spectroscopy:

$$\delta\,(TMS) = \delta\,(CHCl_3) - 7.26$$

$^{13}$C-NMR-Spectroscopy:

$$\delta\,(TMS) = \delta\,(CHCl_3) - 77.0$$

Mass-spectra (MS) were measured using the GCQ Finnigan MAT (Fa. Finnigan) with Xcalibur Version 1.1. The method of ionisation is shown in brackets: EI = electronic ionisation (70 eV); CI = Chemical ionisation (Isobutane or $NH_3$, 170 eV).

Elementary analysis was done with VarioEL (Fa. Elementar).

Before using HPLC the maximum absorption of the substances was measured using the UV/Vis-Spectra done with a 50Bio Cary Spektrophotometer (Fa. Varian).

For determination of the purity and for the separation of the diastereomers a HPLC Hitachi L6200A Intelligent Pump with a UV-Detector (Merck) was used.

Synthesis was done in the synthetic microwave Discover (Fa. CEM).

Some reactions were done using protective gas.

Reactions at -78˚C were done in an acetone bath in a Dewar.

### Example A: 3-Bromthiophen-2-carbaldehyd

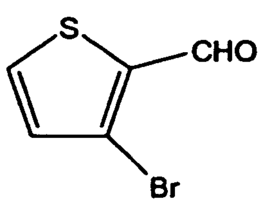

### Experimental procedure

3-Bromo-thiophene (6.523 g, 40 mmol) was dissolved under $N_2$ in THF (100 mL). After cooling to 0-2 ˚C it was mixed dropwise with a 2M LDA solution (20 mL, 40 mmol) and stirred for 0.5 h. Following that 1-Formylpiperidin (4.52 g, 40 mmol) was added. After 2 h the reaction was stopped by adding a surplus of a saturated solution of $NH_4Cl$. For purification it was extracted three times with $Et_2O$ (10 mL) and the pooled organic phases subsequently dried over $Na_2SO_4$. After filtration the solvent was removed under vacuum. The crude product (approx. 8.3 g) was purified using

flash-chromatography (8 cm, cyclohexane:ethylacetate 9:1, 30 mL, $R_f$ = 0.65) and by distillation under vacuum (Bp. 62 ˚C, 5.6 $10^{-2}$ mbar).

Colourless liquid, yield 6.05 g (79.2 %).

$C_5H_3BrOS$ (191.1)

MS (EI): m/z = 190/192 [$M^+$], 162 [$M^+$ -CHO].

IR (Film): ν ($cm^{-1}$) = 3103 (C-H), 2843 (C-H), 1657 (C=O).

$^1$H-NMR ($CDCl_3$): δ (ppm) = 7.15 (d, J = 5.5 Hz, 1 H, 4-H-Th), 7.71 (dd, J = 5.1/1.5 Hz, 1 H, 5-H-Th), 9.99 (d, J = 1.5 Hz, 1 H, Th-CHO).

**Example B: cis/trans-3-Brom-2-(2-methoxyvinyl)-thiophene**

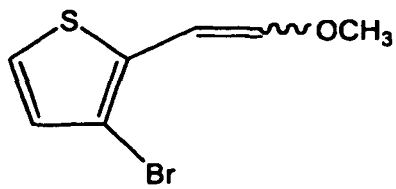

**Experimental procedure**

Methoxymethyltriphenylphosphoniumchloride (13.1 g, 38.3 mmol) was suspended in a 500 mL flask under $N_2$ in THF (200 ml). After cooling to T < -10 ˚C it was mixed with a 1 M solution of potassium tertiary butylate (38.3 mL, 38.3 mmol) (change of colour to red). Few minutes later Example A (5.64 g, 29.5 mmol) was added (change of colour to yellow). After 2 h of reaction time at -10 ˚C purification followed. Thus, $H_2O$ (80 mL) was added and three times extracted with $Et_2O$ (15 mL). The pooled organic phases were dried over $Na_2SO_4$ and subsequently filtered. Then the solvent was removed under vacuum. Triphenylphosphaneoxide was separated from the crude product by extracting with a mixture of cyclohexane/ethylacetate. The crude product was purified by distillation under vacuum (Bp. 72 ˚C 5.6 $10^{-2}$ mbar).

Colourless liquid, yield : 5.39 g (83 %).

$C_7H_7BrOS$ (219.1)

MS (EI): m/z = 218/220 [M*]. 204 [M* -$CH_3$], 175 [$M^+$ - CH($OCH_3$)].

IR (Film): ν ($cm^{-1}$) = 3008 (C=C-*H*), 2833 (C-H), 1634 (C=C), 1225 (C=C-O).

**cis-3-Brom-2-(2-methoxyvinyl)-thiophene**

$^1$H-NMR ($CDCl_3$): δ (ppm) = 3.77 (s, 3H, OC$H_3$), 5.70 (dd, J = 6.6/1.9 Hz, 1H, Th-C*H*=CHOC$H_3$), 6.18 (d, J = 6.6 Hz, 1 H, Th-CH=C*H*OC$H_3$), 6.85 (d, J = 5.4 Hz, 1 H, 4-*H*-Th), 7.09 (dd, J = 5.4/0.9 Hz, 1 H, 5-*H*-Th).

**trans3-Brom-2-(2-methoxyvinyl)-thiophene**

$^1$H-NMR ($CDCl_3$): δ (ppm) = 3.64 (s, 3 H, OC$H_3$), 5.89 (d, J = 12.9 Hz, 1 H, Th-C*H*=CH(OC$H_3$)), 6.82 (d, J = 5.4 Hz, 1 H, 4-*H*-Th), 6.89 (d, J = 5.4 Hz, 1 H, 5-*H*-Th), 6.97 (d, J = 12.9 Hz, 1 H, Th-CH=C*H*OC$H_3$).

Cis- and trans- are occurring in a ratio of 2:1.

**Example C: 2-(3-Bromthiophen-2-yl)-acetaldehyddimethylacetal**

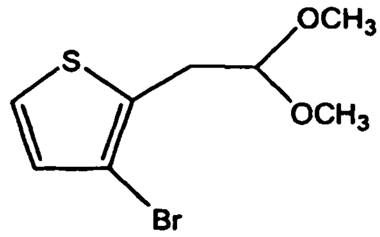

**Experimental procedure**

Example B (5.39 g, 24.6 mmol) was dissolved under $N_2$ in 80 mL MeOH. After addition of 10 mol% of p-toluene sulphonic acid (470 mg, 2.46 mmol) and trimethylorthoformiate (5 mL) it was heated for 24 h under reflux. For purification it was alkalised with 2M NaOH (15 mL) (pH = 10) and $H_2O$ (30 mL) added and then extracted three times with $CH_2Cl_2$ (10 mL). The pooled organic phases were dried over $Na_2SO_4$ and subsequently filtered. The solvent was removed under vacuum. Purification of crude product (approx. 5.8 g) was done by distillation under vacuum (Bp. 78 ˚C, 5.6 - $10^{-2}$ mbar).
Colourless liquid, yield 5.73 g (93 %).
$C_8H_{11}BrO_2S$ (251.1)

| | |
|---|---|
| MS (EI): | m/z = 251 [$M^+$], 220 [$M^+$ -$OCH_3$], 140 [$M^+$ -Br, -$OCH_3$]. |
| IR (Film): | ν ($cm^{-1}$) = 3106 (C-H), 2830 (C-H), 1057 (C-O). |
| $^1$H-NMR ($CDCl_3$): | δ (ppm) = 3.03 (d, J = 5.6 Hz, 2 H, Th-C*H*$_2$CH($OCH_3$)$_2$), 3.31 (s, 6 H, Th-$CH_2$CH(OC*H*$_3$)$_2$), 4.47 (t, J = 5.6 Hz, 1 H, Th-$CH_2$CH(OC*H*$_3$)$_2$), 6.84 (d, J = 5.4 Hz, 1 H, 4-*H*-Th), 7.15 (d, J = 5.4 Hz, 1 H, 5-*H*-Th). |

**Example D: Ethyl-4-[2-(2,2-dimethoxyethyl-thiophen-3-yl]-4-hydroxypiperidin-1-carboxylat**

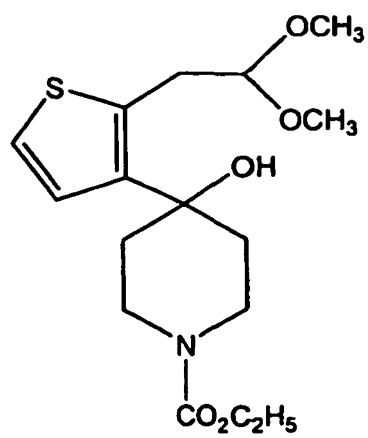

Experimental procedure

Example A (1.52 g, 6 mmol) was dissolved under $N_2$ in THF (80 mL) and cooled to - 80˚C. A solution of 1.35M n-butyllithium in n-hexane (5.2 mL, 7.8 mmol) was added dropwise within 2-3 min and stirred for 15 min. Following that 1-ethoxycarbonylypiperidin-4-one (1.02 g, 6.0 mmol), dissolved in THF (1.5 mL), was added and it was first stirred for one hour at -80˚C and subsequently for 3 h at RT. For purification $H_2O$ (30 mL) was added and extracted three times with 10 mL $CH_2Cl_2$ extracted. The pooled organic phases were dried over $Na_2SO_4$ and subsequently filtered. The solvent was removed under vacuum. The crude product (approx. 2.22 g) was purified using flash-chromatography (4 cm, Cyclohexane:ethylacetate 7:3, 30 mL, $R_f$ = 0.3).
Yellowish oil, yield 1.48 g (approx. 70 %, slightly polluted with some 1-ethoxy-carbonylpiperidin-4-one).
$C_{16}H_{25}NO_5S$ (343.4)

MS (EI): m/z = 343 [$M^+$], 264 [$M^+$ -OH, -$OCH_3$ (2x)], 210 [$M^+$ -$OCH_3$ (2x), -$COOC_2H_5$].

IR (Film): ν ($cm^{-1}$) = 3436 (O-H), 2843 (C-H), 1671 (C=O), 1051 (C-O).

$^1$H-NMR ($CDCl_3$): δ (ppm) = 1.25 (t, J = 7.2 Hz, 3H, $CO_2CH_2CH_3$), 1.65 (d, J = 14.4 Hz, 2 H, $N(CH_2CH_2)_2$). 1.87 (t, J = 14.4 Hz, 2 H, $N(CH_2CH_2)_2$). 3.09 - 3.25 (m, 4 H, (Th-$CH_2$CH) ($N(CH_2CH_2)_2$)), 3.32 (s, 6H, CH ($(OCH_3)_2$), 3.90 - 4.03 (m, 2H, $N(CH_2CH_2)_2$), 4.07 (q, J = 7.2 Hz, 2 H, $CO_2CH_2CH_3$), 4.43 (t, J = 5.2 Hz, 1 H, Th-$CH_2CH$), 6.73 (d, J = 5.6 Hz, 1 H, 4-*H*-Th), 7.11 (d, J = 5.2 Hz, 1H, 5-*H*-Th).

**Example 1: Ethyl-6'-methoxy-6',7'-dihydrospiro[piperidine-4,4'-thieno[3,2-c]pyran]-1-carboxylate**

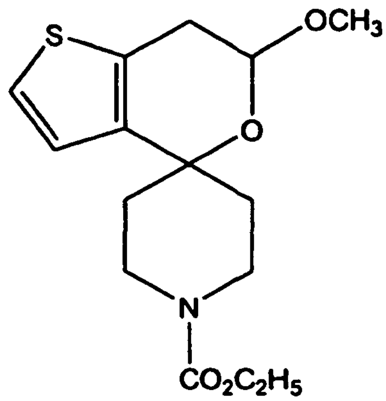

Experimental procedure

Example D (1.49 g, 4.3 mmol) was dissolved under $N_2$ in MeOH (50 mL). After addition of 1.2 equivalents of p-toluene sulphonic acid (1 g, 5.2 mmol) and trimethylorthoformiate (5 mL) it was stirred for 1 h at RT. For purification it was alkalised with 2M NaOH and $H_2O$ (30 mL) added. Following that it was extracted three times with $CH_2Cl_2$ (10 mL). The pooled organic phases were dried over Na2SO4 and subsequently filtered. The solvent was removed under vacuum. The crude product (approx. 1.56 g) was purified using flash-chromatography (3 cm, cyclohexane:ethylacetate 7:3, 30 mL, $R_f$ = 0.54).

Colourless oil, yield 990 mg (53 % over two steps).

$C_{15}H_{21}NO_4S$ (311.4)

MS (EI): m/z = 311 [$M^+$], 280 [$M^+$ -$OCH_3$].

IR (Film): ν ($cm^{-1}$) = 2924 (C-H), 1693 (C=O), 1059 (C-O), 718 (C-H).

$^1$H-NMR ($CDCl_3$): δ (ppm) = 1.22 (t, J = 7.2 Hz, 3 H, $CO_2CH_2CH_3$), 1.69 (td, J = 13.5/4.6 Hz, 1 H, $N(CH_2CH_2)_2$), 1.76 (dd, J = 13.5/4.6 Hz, 1 H $N(CH_2CH)_2$), 1.83 (td, J = 13.5/4.6 Hz, 1 H, $N(CH_2CH_2)_2$), 1.89 (dd, J = 13.5/4.6 Hz, 1 H, $N(CH_2CH_2)_2$), 2.80 (dd, J = 15.9/7.2 Hz, 1 H, Th-$CH_2$), 2.95 (dd, J = 15.9/3.3 Hz, 1 H, Th-$CH_2$), 3.10 - 3.25 (m, 2H, $N(CH_2CH_2)_2$), 3.50 (s, 3 H, $OCH_3$), 3.98 - 4.15 (m, 2 H, N ($CH_2CH_2)_2$), 4.10 (q, J = 7.2 Hz, 2 H, $CO_2CH_2CH_3$), 4.83 (dd, J = 7.2/3.3 Hz, 1 H, Th-$CH_2CH$), 6.65 (d, J = 5.1 Hz, 1 H, 4-*H*-Th), 7.05 (d, J = 5.1 Hz, 1 H. 5-*H*-Th).

**Example 2: 6'-Methoxy-6',7'-dihydrospiro[piperidin-4,4'-thieno[3;2-c]pyran**

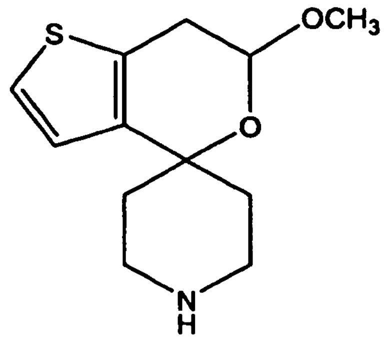

Experimental procedure

Example 1 (0.92 g, 2.96 mmol) was dissolved in a 100 mL flask in dioxane/$H_2O$ 1:1 (50 mL). After addition of 70 equivalents of 2M NaOH (100 mL) it was heated for 5 h under reflux. For purification $Et_2O$ (20 mL) was added and again extracted three times with $Et_2O$ (5 mL). The pooled organic phases were dried over $Na_2SO_4$. Following filtration the solvent was removed under vacuum. The crude product (approx. 0.72 g) was purified using flash-chromatography (3 cm, MeOH:$NH_3$ 98:2, 15 mL, $R_f$ = 0.25).

yield: Colourless Solid , Mp. 103 ˚C, yield 0.385 g (54 %).

$C_{12}H_{17}NO_2S$ (239.3)

MS (EI): m/z = 239 [$M^+$], 208 [$M^+$ -$OCH_3$], 151 ($M^+$ -$OCH_3$, -$CH_2NHCH_2CH_2$).

IR (Film): ν ($cm^{-1}$) = 3276 (N-H), 2915 (C-H), 1057 (C-O), 746 (C-H).

$^1$H-NMR ($CDCl_3$): δ (ppm) = 1.68 (td, J = 14.0/4.7 Hz, 1 H, N($CH_2CH_2$)$_2$), 1.76 (dd, J = 14.0/2.7 Hz, 1 H, N($CH_2CH_2$)$_2$), 1.84 - 1.96 (m, 2 H, N($CH_2C\mathit{H}_2$)$_2$), 2.78 (dd, J = 15.6/7.5 Hz, 1 H, Th-C$\mathit{H}_2$-$CHOCH_3$), 2.84 - 2.88 (m, 1 H, N(C$\mathit{H}_2$$CH_2$)$_2$), 2.88 - 2.93 (m, 1 H, N(C$\mathit{H}_2$$CH_2$)$_2$), 2.92 (dd, J = 15.6/3.3 Hz, 1 H, Th-C$\mathit{H}_2$ $CHOCH_3$), 3.04 (td, J = 12.3/2.7 Hz, 1 H, N(C$\mathit{H}_2$$CH_2$)$_2$), 3.15 (td, J = 12.3/3.0 Hz, 1 H, N(C$\mathit{H}_2$$CH_2$)$_2$), 3.52 (s, 3 H, Th-$CH_2$-CHOC$\mathit{H}_3$), 4.83 (dd, J = 7.2/3.3 Hz, 1 H, Th-$CH_2$-C*H*$OCH_3$), 6.72 (d, J = 5.1 Hz, 1 H, 4-*H*-Th), 7.03 (d, J = 5.1 Hz, 1 H, 5-*H*-Th). No signal for NH can be seen.

**Example E: 2-[3-(1-Benzyl-4-hydroxypiperidin-4-yl)thiophen-2-yl]acetaldehyddimethylacetal**

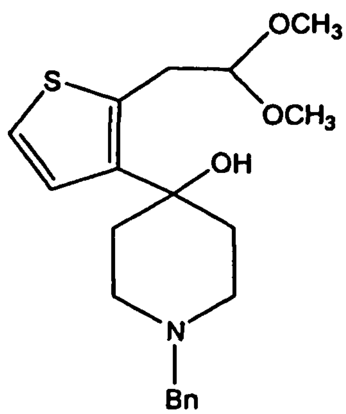

Experimental procedure

Example C (2.18 g, 8.7 mmol) was dissolved in THF (100 mL) and cooled under $N_2$ to -84 ˚C. A solution of 1,35M in n-butyllithium in hexane (8.3 mL, 11.3 mmol) was added dropwise within 2-3 min under stirring, until after 15 min 1-benzyl piperidin-4-one (1.81 g, 9.57 mmol) was added. After 1 h of stirring at -84 ˚C a further 2 h it was stirred at RT. The reaction was stopped by adding 20 mL of water. Then a solution of $NaHSO_3$ (10 mL, 10%.) was added and the

mixture extracted three times with 10 mL of $CH_2Cl_2$. The organic phases were pooled and dried over $Na_2SO_4$, followed by filtration and the solvent was removed under vacuum. The crude product (approx. 3.31 g) was purified using flash-chromatography (5 cm, cyclohexane:ethylacetate 7:3, 30 mL, $R_f$= 0.3).

Yellowish oil, yield 2.14 g (68 %).

$C_{20}H_{27}NO_3S$ (361.5)

MS (EI): m/z = 361 [$M^+$], 330 [$M^+$ -$OCH_3$], 270 [$M^+$ - $CH_2$Ph], 91 [-$CH_2$Ph].

IR (Film): ν ($cm^{-1}$) = 3450 (O-H), 2851 (C-H), 1051 (C-O), 698 (C-H).

$^1$H-NMR ($CDCl_3$): δ (ppm) = 1.67 (dd, J = 14.0/2.5 Hz, 2 H, N($CH_2$C$H_2$)$_2$), 2.04 (td, J = 13-2/4.5 Hz, 2 H, N($CH_2$C$H_2$)$_2$), 2.45 (td, J = 12.3/2.1 Hz, 2 H, N(C$H_2$$CH_2$)$_2$), 2.62 - 2.71 (m, 2 H, N(C$H_2$$CH_2$)$_2$), 3.30 (s, 6 H, Th-$CH_2$-CH(OC$H_3$)$_2$), 3.38 (d, J = 5.4 Hz, 2 H, Th-C$H_2$-CH($OCH_3$)$_2$), 3.50 (s, 2 H,N-C$H_2$-Ph), 3.78 (s, 1 H, O*H*), 4.44 (t, J = 5.4 Hz, 1 H, Th-$CH_2$-C*H*($OCH_3$)$_2$), 6.83 (d, J = 5.4 Hz, 1 H, 4-*H*-Th), 6.99 (d, J = 5.4 Hz, 1 H, 5-*H*-Th) 7.10 - 7.21 (m, 5 H, Ph-H).

**Example 3: 1-Benzyl-6'-methoxy-6',7'-dihydrospiro[piperidin-4,4'-thieno[3,2-c]pyran]**

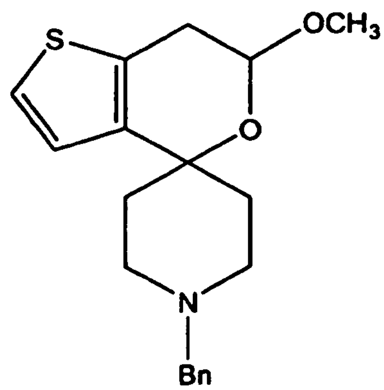

Experimental procedure

Example E (287 mg, 0.8 mmol) was dissolved in MeOH (15 mL) and mixed with trimethylorthoformiate (2 mL). After addition of p-toluene sulphonic acid (183 mg, 0.96 mmol) it was stirred for 24h under $N_2$ 24 h at RT. For purification it was alkalised with 2M NaOH and $H_2O$ (10 mL) added. Subsequently it was extracted three times with $CH_2Cl_2$ (5 mL). The organic phases were pooled and dried over $Na_2SO_4$, then it was filtered and the solvent removed under vacuum. The crude product (approx. 235 mg) was purified using flash-chromatography (2 cm, cyclohexane:ethylacetate 7:3, 15 mL, $R_f$ = 0.21).

Colourless crystalline solid, Mp. 78 °C, yield 199 mg (75%).

$C_{19}H_{23}NO_2S$ (329.5)

HPLC: Method 1: 95.0 %, $R_t$ = 18.0 min Method 2:

MS (EI): m/z = 329 [$M^+$], 298 [$M^+$ -$OCH_3$ ], 238 [$M^+$ -$CH_2$Ph], 91 [$^+CH_2$Ph].

IR (Film): ν ($cm^{-1}$) = 3099 (C-H), 2810 (C-H), 1057 (C-O), 746 (C-H).

$^1$H-NMR ($CDCl_3$): δ (ppm) = 1.76 (dd, J = 13.5/2.1 Hz, 1 H, N($CH_2$$CH_2$)$_2$), 1.80 - 1.93 (m, 2 H, N($CH_2$C$H_2$)$_2$), 2.04 (td, J = 13.4/4.5 Hz, 1 H, N($CH_2$C$H_2$)$_2$), 2.37 (td, J = 11.6/3.7 Hz, 1 H, N(C$H_2$$CH_2$)$_2$), 2.45 (td, J = 12.4/2.6 Hz, 1 H, N(C$H_2$$CH_2$)$_2$), 2.65 - 2.73 (m, 2 H, N(C$H_2$$CH_2$)$_2$), 2.77 (dd, J = 15.6/7.5 Hz, 1 H, Th-C$H_2$CHOC$H_3$), 2.92 (dd, J = 15.6/3.3 Hz, 1 H, Th-C$H_2$CHOC$H_3$), 3.48 (d, J = 13.0 Hz, 1 H, N-C$H_2$-Ph ), 3.49 (s, 3 H, Th-$CH_2$CHOC$H_3$), 3.54 (d, J = 13.0 Hz, 1 H, N-C$H_2$-Ph), 4.81 (dd, J = 7.2/3.3 Hz, 1 H, Th-$CH_2$C*H*OC$H_3$), 6.73 (d, J = 5.4 Hz, 1 H, 4-*H*-Th), 7.02 (d, J = 5.4 Hz, 1 H, 5-*H*-Th), 7.15 - 7.31 (m, 5 H, Ph-*H*).

**Example 2: 6'-Methoxy-6',7'-dihydrospiro[piperidin-4,4'-thieno[3,2-c]pyran (different approach)**

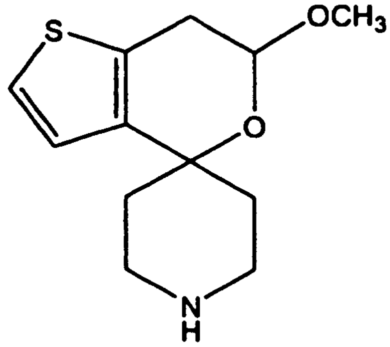

Experimental procedure

Example 3 (100 mg, 0.31 mmol) was dissolved in THF (8 mL) and cooled to -78 ˚C under $N_2$. After addition of chloro-formic-acid-chloroethylester (45 μL, 0.40 mmol) it was stirred for 20 min, then heated to RT and THF removed under vacuum. Following that the product was mixed with MeOH (8 mL) and heated for 40 min under reflux. Subsequently the methanol was removed under vacuum. The crude product (approx. 75 mg) was purified using flash-chromatography (0.7 cm, ethyl-acetate:MeOH:$NH_3$ 90:10:2, 3 mL, $R_f$ = 0.18).

Colourless solid , Mp. 103 ˚C, yield 48 mg (65 %).

**Example F: 2-{3-[4-Hydroxy-1-(2-phenylethyl)piperidin-4-yl]thiophen-2 yl}acetaldehyd-dimethylacetal**

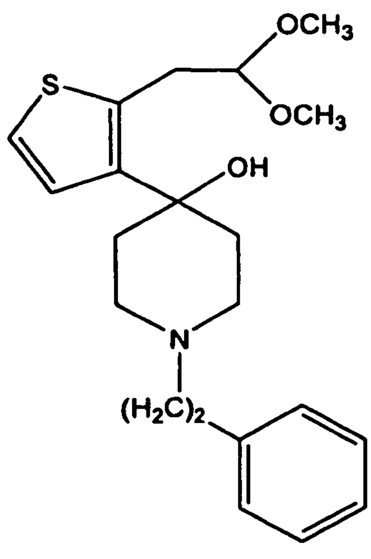

Experimental procedure

Example C (0.5 g, 2 mmol) was dissolved in THF (25 mL) and cooled to -80 ˚C under $N_2$. A solution of 1.35M n-butyllithium in hexane (1.7 mL, 2.4 mmol) was added dropwise within 2-3 min while stirring, until after 15 min 1-(2-Phenylethyl)piperidin-4-one (450 mg, 2.2 mmol) was added. After 1.5 h of stirring at -80 ˚C a further 1.5 h of stirring at RT followed. The reaction was stopped by addition of $H_2O$ (10 mL). Following that a solution of $NaHSO_3$ (10 mL, 10%) was added and extracted three times with $CH_2Cl_2$ (5 mL). The organic phases were pooled and dried over $Na_2SO_4$. Subsequently it was filtered and the solvent removed under vacuum. The crude product (approx. 1 g) was purified using flash-chromatography (3 cm, cyclohexane:ethylacetate 7:3, 30 mL, $R_f$= 0.18).

Yellowish oil, yield 62 mg (8 %).

$C_{21}H_{29}NO_3S$ (375.5)

MS (EI): m/z = 375 [$M^+$], 344 [$M^+$-$OCH_3$], 284 [$M^+$ -$CH_2Ph$], 253 [$M^+$ -$OCH_3$, -$CH_2Ph$], 91 [$^+CH_2Ph$].

IR (Film): ν ($cm^{-1}$) = 3444 (O-H); 2922, 2828 (C-H); 1070, 1055 (C-O).

$^1$H-NMR ($CDCl_3$): δ (ppm) = 1.69 - 1.81 (m, 4 H ($N(CH_2CH_2)_2$) (N-$CH_2CH_2$Ph)), 2.10 (td, J = 13.3/3.4 Hz, 2H, $N(CH_2CH_2)_2$), 2.46 - 2.67 (m, 2 H, $N(CH_2CH_2)_2$), 2.75 - 2.86 (m, 4 H (N-$CH_2CH_2$Ph) ($N(CH_2CH_2)_2$)), 3.32 (s, 6 H, Th-$CH_2CH(OCH_3)_2$), 3.39 (d, J = 5.4 Hz, 2 H, Th-$CH_2CH(OCH_3)_2$), 3.90 (s, 1 H, -OH), 4.45 (t, J = 5.4 Hz, 1 H, Th-$CH_2CH(OCH_3)_2$), 6.85 (d, J = 5.4Hz, 1 H, 4-H-Th), 7.01 (d, J = 5.4 Hz, 1 H, 5-H-Th), 7.13 - 7.25 (m, 5 H,Ph-H).

<u>**Example 4: 6'-Methoxy-1-(2-phenylethyl)-6'7'-dihydrospiro[piperidin-4,4'-thieno [3,2-c]pyran]**</u>

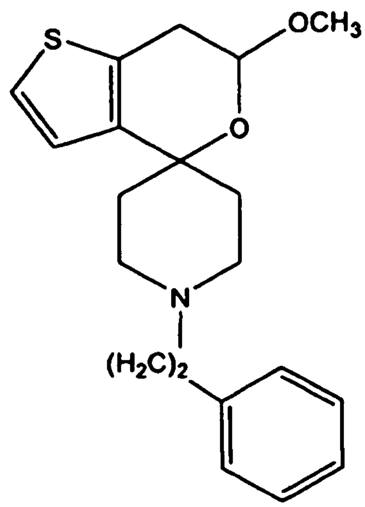

Experimental procedure

Example F (55 mg, 0.14 mmol) was dissolved in MeOH (5 mL) and after addition of trimethylorthoformiate (1 mL) mixed with p-toluene sulphonic acid (33 mg, 1.75 mmol). After stirring for 24 h at RT it was alkalised with 2M NaOH (2 mL) and extracted three times with $CH_2Cl_2$ (5 mL). The pooled organic phases were dried over $Na_2SO_4$, then filtered and the solvent removed under vacuum. The crude product (approx. 50 mg) was purified using flash-chromatography (1 cm, cyclohexane:ethylacetate 7:3, 5 mL, $R_f$= 0.10).

Beige crystalline solid, mp. 94 °C, yield 21 mg (44 %).

$C_{20}H_{25}NO_2S$ (343.5)

HPLC: Method 1: 97.9 %, $R_t$ = 18.9 min Method 2:

MS (EI): m/z = 343 [$M^+$], 312 [$M^+$-$OCH_3$], 252 [$M^+$ -$CH_2Ph$], 91 [$CH_2Ph$].

IR (Film): ν ($cm^{-1}$) = 3108 (C-H), 2954 (C-H), 1060 (C-O), 747 (C-H).

$^1$H-NMR ($CDCl_3$): δ (ppm) = 1.78 - 1.98 (m, 3 H, $N(CH_2CH_2)_2$), 2.08 (td, J = 12.6/4.4 Hz, 1 H, $N(CH_2CH_2)_2$), 2.38 - 2.56 (m, 2 H, $N(CH_2CH_2)_2$), 2.57 - 2.63 (m, 2 H, N- $CH_2CH_2$-Ph), 2.76 - 2.89 (m, 5 H (Th-$CH_2CHOCH_3$) (N-$CH_2CH_2$-Ph) ($N(CH_2CH_2)_2$)), 2.94 (dd, J = 15.6/3.3 Hz, 1 H, Th-$CH_2CHOCH_3$), 3.51 (s, 3 H, Th-$CH_2CHOCH_3$), 4.82 (dd, J = 7.2/3.3 Hz, 1 H, Th-$CH_2CHOCH_3$), 6.75 (d, J = 5.4 Hz, 1 H, 4-H-Th), 7.04 (d, J = 5.4 Hz, 1 H, 5-H-Th), 7.15 - 7.28 (m, 5 H, Ph-H).

**Example G: 2-{3-[1-(Cyclohexylmethyl)-4-hydroxypiperidin-4-yl]thiophen-2-yl} acetaldehyddimethylacetal**

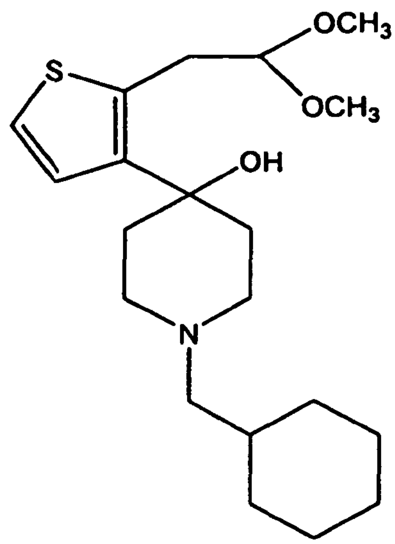

Experimental procedure

Example C (1.18 g, 4.7 mmol) was dissolved in THF (50 mL) and cooled to -78 ˚C under $N_2$. A solution of 1,5M n-butyllithium in hexane (5.2 mL, 7.0 mmol) was added dropwise within 2-3 min while stirring, until after 15 min 1-(Cyclohexylmethyl)piperidin-4-one (1.0 g, 5.2 mmol) was added. After stirring for 1.5 h at -78 ˚C it was stirred a further 2 h at RT. The reaction was stopped by addition of $H_2O$ (50 mL). Following that it was extracted three times with $CH_2Cl_2$ (10 mL). The organic phases were pooled and dried over Na2SO4, then filtered and the solvent removed under vacuum. The crude product (approx. 1.8 g) was purified using flash-chromatography (4 cm, Cyclohexane:ethylacetate 1:1, 30 mL, $R_f$= 0.21).

Colourless oil, yield 0.8 g (46 %).

$C_{20}H_{33}NO_3S$ (367.5)

| | |
|---|---|
| MS (EI): | m/z = 367 [$M^+$], 336 [$M^+$-$OCH_3$], 284 [$M^+$ -cHex]. |
| IR (Film): | v ($cm^{-1}$) = 3457 (O-H), 2918 (C-H), 1053 (C-O), 719 (C-H). |
| $^1$H-NMR ($CDCl_3$): | δ (ppm) = 0.77 - 0.87 (m, 2 H, cHex-*H*), 1.07 - 1.20 (m, 3 H, cHex-*H*), 1.40 - 1.48 (m, 1 H, cHex-*H*), 1.55 - 1.75 (m, 5 H, cHex-*H*), 1.66 (dd, J = 13.7/2.6 Hz, 2 H, N($CH_2CH_2$)$_2$), 2.03 (td, J = 12.6/3.3 Hz, 2 H, N($CH_2CH_2$)$_2$), 2.11 (d, J = 7.4 Hz, 2 H, N-$CH_2$-cHex), 2.31 (t, J = 11.7 Hz, 2 H, N($CH_2CH_2$)$_2$), 2.59 - 2.65 (m, 2 H, N($CH_2CH_2$)$_2$), 3.29 (s, 6 H, Th-$CH_2CH(OCH_3)_2$), 3.87 (d, J = 4.2 Hz, 2 H, Th-$CH_2CH(OCH_3)_2$), 3.69 (s, 1 H, O*H*), 4.44 (t, J = 4.2 Hz, 1 H, Th-$CH_2CH(OCH_3)_2$), 6.83 (d, J = 5.2 Hz, 1 H, 4-*H*-Th), 7.00 (d, J = 5.2 Hz, 1 H, 5-*H*-Th). |

**Example 5: 1-(Cyclohexylmethyl)-6'-Methoxy -6',7-dihydrospiro[piperidin-4,4'-thieno[3,2-c]pyran]**

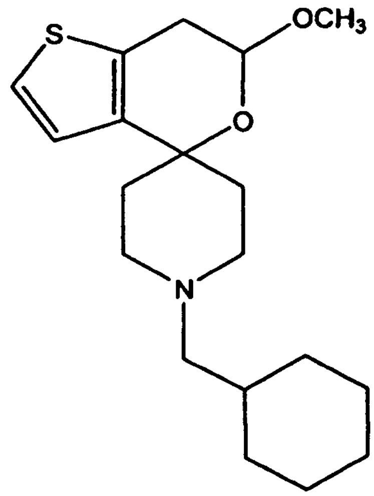

**Experimental procedure**

Example G (0.8 g, 2.2 mmol) was dissolved in MeOH (30 mL) and mixed with trimethyl orthoformiate (3 mL). Folowing addition of p-toluene sulphonic acid (0.5 g, 2.6 mmol) it was stirred for 3 h at RT under $N_2$. For purification it was alkalised with 2M NaOH and $H_2O$ (10 mL) added. Subsequently it was extracted three times with $CH_2Cl_2$ (10 mL) extracted. The organic phases were pooled and dried over $Na_2SO_4$, then filtered and the solvent removed under vacuum. The crude product was purified using flash-chromatography (2.5 cm, Cyclohexane:ethylacetate 7:3, 15 mL, $R_f$ = 0.45).
Colourless crystalline solid, Mp. 78 ˚C, yield 490 mg (68 %).

**Example H: 2-{3-[4-Hydroxy-1-(3-methylbutyl)piperidin-4-yl]thiophen-2-yl} acetaldehyddimethylacetal**

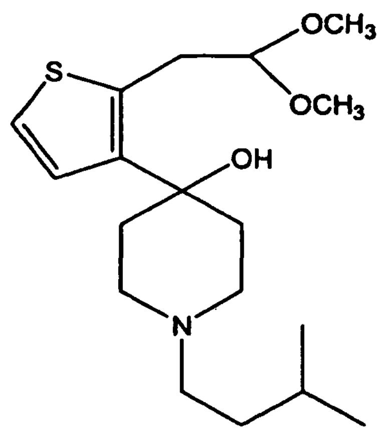

**Experimental procedure**

Example C (1.6 g, 6.4 mmol) was dissolved in THF (80 mL) and cooled to -78 ˚C under $N_2$. A solution of 1,5M n-butyllithium in hexane (5.15 mL, 7.7 mmol) was added dropwise within 2-3 min while stirring, until after 15 min 1-(3-Methylbutyl)piperidin-4-one (1.2 g, 7.0 mmol) was added. After 2 h of stirring at -78 ˚C another 2 h of stirring at RT was added. The reaction was stopped by addition of $H_2O$ (50 mL). Subsequently it was extracted three times with $CH_2Cl_2$ (10 mL) extracted. The organic phases were pooled and dried over Na2SO4, then filtered and the solvent removed under vacuum. The crude product (approx. 2.2 g) was purified using flash-chromatography (4 cm, Cyclohexane:ethylacetate 1:1, 50 mL, $R_f$= 0.22).
Colourless oil, yield 1.2 g (approx. 55 %, slight impurities of 1-(3-Methylbutyl)piperidin-4-one).
$C_{18}H_{31}NO_3S$ (341.5)

MS (EI): m/z = 341 [$M^+$], 310 [$M^+$ -$OCH_3$], 284 [$M^+$ -$CH_2CH(CH_3)_2$].

IR (Film): ν ($cm^{-1}$) = 3446 (O-H), 2951 (C-H), 1054 (C-O), 719 (C-H).

$^1$H-NMR ($CDCl_3$): δ (ppm) = 0.91 (d, J = 4.8 Hz, 6 H, $CH_2CH_2CH(CH_3)_2$), 1.41 - 1.45 (m, 1 H, $CH_2CH_2CH(CH_3)_2$), 1.52 - 1.62 (m, 2 H, $CH_2CH_2CH(CH_3)_2$), 1.76 (dd, J = 13.7/2.7 Hz, 2 H, $N(CH_2CH_2)_2$), 2.13 (td, J = 12.6/4.2 Hz, 2 H, $N(CH_2CH_2)_2$), 2.38 - 2.48 (m, 4 H ($CH_2CH_2CH(CH_3)_2$) ($N(CH_2CH_2)_2$)), 2.77 - 2.82 (m, 2 H, $N(CH_2CH_2)_2$), 3.37 (s, 6 H, Th-$CH_2CH(OCH_3)_2$), 3.45 (d, J = 4.2 Hz, 2 H, Th-$CH_2CH(OCH_3)_2$), 3.85 (s, 1 H, O*H*), 4.51 (t, J = 4.2 Hz, 1H, Th-$CH_2CH(OCH_3)_2$), 6.90 (d, J = 5.2 Hz, 1 H, 4-*H*-Th), 7.06 (d, J = 5.2 Hz, 1 H, 5-H-Th).

**Example 6: 6'-Methoxy-1-(3-methylbutyl)-6',7'-dihydrospiro[piperidin-4,4'-thieno[3,2-c]pyran]**

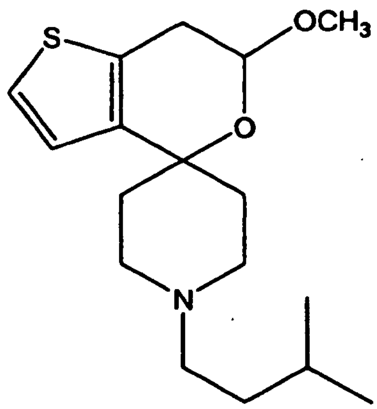

Experimental procedure

**Example H** (1.2 g, 3.5 mmol) was dissolved in MeOH (40 mL) and mixed with trimethylorthoformiate (4 mL). After addition of p-toluene sulphonic acid (0.8 g, 4.2 mmol) it was stirred under $N_2$ for 24 h at RT. For purification it was alkalised with 2M NaOH and $H_2O$ (10 mL) added. Subsequently it was extracted three times with $CH_2Cl_2$ (10 mL). The organic phases were pooled and dried over Na2SO4, then filtered and the solvent removed under vacuum. The crude product (approx. 1.1 g) was purified using flash-chromatography (4 cm, cyclohexane:ethylacetate 7:3, 30 mL, $R_f$= 0.13).

Colourless crystalline solid, Mp. 78 °C, yield 950 mg (88 %).

**Example 5: 1-(Cyclohexylmethyl)-6'-Methoxy-6',7'-dihydrospiro[piperidin-4,4'-thieno[3,2-c]pyran]] (alternative route)**

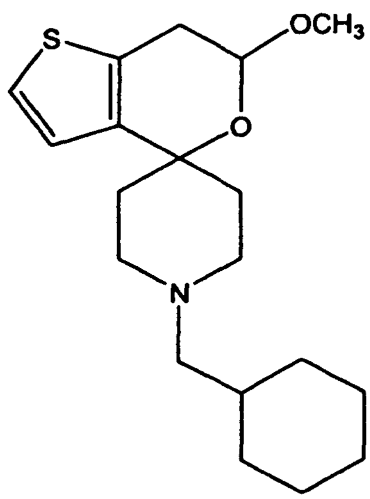

**Experimental procedure**

Example 2 (90 mg, 0.37 mmol) was dissolved in $CH_3CN$ (8 mL), mixed with cyclohexylmethylbromide (80 mg, 0.45 mmol) and $K_2CO_3$ (300 mg) and heated for 24 h under reflux. For purification $K_2CO_3$ was removed by a frit and the solution transferred into a separating funnel. After addition of saturated NaCl solution (10 mL) it was extracted three times with $Et_2O$ (5 mL). The organic phases were pooled and dried over Na2SO4, then filtered and the solvent removed under vacuum. The crude product (approx. 162 mg) was purified using flash-chromatography (1.5 cm, Cyclohexane: ethylacetate 7:3, 10 mL, $R_f$ = 0.45).

Colourless crystalline solid, Mp. 77 ˚C, yield 118 mg (95 %).

$C_{19}H_{29}NO_2S$ (335.5)

| | |
|---|---|
| HPLC: | Method 1: 98.5 %, $R_t$ = 19.1 min<br>Method 2: |
| MS (EI): | m/z = 335 [$M^+$], 304 [$M^+$-$OCH_3$ ], 252 [$M^+$ - cHex]. |
| IR (Film): | ν ($cm^{-1}$) = 3102 (C-H), 2914 (C-H), 1058 (C-O), 748 (C-H). |
| $^1$H-NMR ($CDCl_3$): | δ (ppm) = 0.82 - 0.87 (m, 2 H, cHex-*H*), 1.12 - 1.20 (m, 2 H, cHex-*H*), 1.41 - 1.51 (m, 1 H, cHex-*H*), 1.56 - 1.86 (m, 9 H (N($CH_2CH_2$)$_2$) (cHex-*H*), 2.05 (td, J = 13.2/4.5 Hz, 1 H, N($CH_2CH_2$)$_2$), 2.12 (d, J = 7.0 Hz, 2 H, N-$CH_2$-cHex), 2.27 (td, J = 12.1/3.3 Hz, 1 H, N($CH_2CH_2$)$_2$), 2.34 (td, J = 12.5/2.3 Hz, 1 H, N($CH_2CH_2$)$_2$), 2.64 - 2.70 (m, 2 H, N($CH_2CH_2$)$_2$), 2.78 (dd, J = 16.0/7.1 Hz, 1 H, Th-$CH_2CHOCH_3$), 2.92 (dd, J = 15.6/3.1 Hz, 1 H, Th-$CH_2CHOCH_3$), 3.51 (s, 3 H, Th-$CH_2CHOCH_3$), 4.81 (dd, J = 7.4/3.1 Hz, 1 H, Th-$CH_2CHOCH_3$), 6.72 (d, J = 5.1 Hz, 1 H, 4-*H*-Th), 7.02 (d, J = 5.5 Hz, 1 H, 5-*H*-Th). |

**Example 6: 6'-Methoxy-1-(3-methylbutyl)-6',7'-dihydrospiro[piperidin-4,4'-thieno[3,2-c]pyran] (alternative route)**

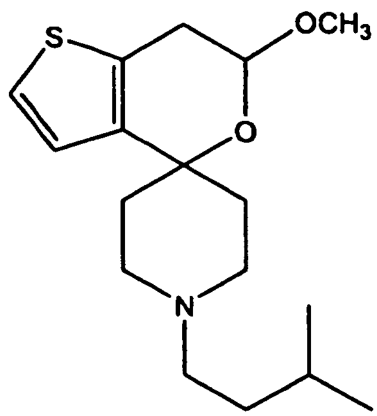

Experimental procedure

Example 2 (58 mg, 0.24 mmol) was dissolved in $CH_2Cl_2$ (5 mL), mixed with Isovaleraldehyde (22 mg, 0.25 mmol) and $NaBH(OAc)_3$ (80 mg, 0.38 mmol) and stirred for 2 h at RT. Following the end of the reaction a saturated solution of $NaHCO_3$ (5 mL) was added and extracted three times with $CH_2Cl_2$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.13).

Colourless crystalline solid, Mp. 38 ˚C, yield 61.5 mg (82.5 %).

$C_{17}H_{27}NO_2S$ (309.5)

| | |
|---|---|
| HPLC: | Method 1: 98.3 %, $R_t$ = 18.1 min<br>Method 2: |

MS (EI): m/z = 309 [$M^+$], 278 [$M^+$-$OCH_3$], 252 [$M^+$ -$C_4H_9$].

IR (Film): ν ($cm^{-1}$) = 3098 (C-H), 2949 (C-H), 1058 (C-O), 745 (C-H).

$^1$H-NMR ($CDCl_3$): δ (ppm) = 0.85 (d, J = 6.7 Hz, 6 H, N-$CH_2CH_2CH(CH_3)_2$), 1.36 - 1.41 (m, 1 H, N-$CH_2CH_2CH(CH_3)_2$), 1.51 - 1.58 (m, 2 H, N-$CH_2CH_2CH(CH_3)_2$), 1.78 (dd, J = 13.7/2.7 Hz, 1 H, N($CH_2CH_2)_2$), 1.84 (td, J = 12.6/4.3 Hz, 1 H, N($CH_2CH_2)_2$), 1.92 (dd, J = 14.2/2.8 Hz, 1 H, N($CH_2CH_2)_2$), 2.05 (td, J = 13.2/4.5 Hz, 1 H, N($CH_2CH_2)_2$), 2.27 - 2.42 (m, 4 H (N-$CH_2CH_2CH(CH_3)_2$) (N($CH_2CH_2)_2$)), 2.74 - 2.81 (m, 3 H (N($CH_2CH_2)_2$) (Th-$CH_2CHOCH_3$), 2.91 (dd, J = 15.6/3.3 Hz, 1 H, Th-$CH_2CHOCH_3$), 3.51 (s, 3 H, Th-$CH_2CH(OCH_3)$, 4.82 (dd, J = 7.1/3.3 Hz, 1 H, Th-$CH_2CHOCH_3$), 6.74 (d, J = 5.1 Hz, 1 H, 4-*H*-Th), 7.03 (d, J = 5.1 Hz, 1 H, 5-*H*-Th).

**Example 7: 6'-Methoxy-1-(3-methylbut-2-enyl)-6',7'-dihydrospiro[piperidin-4,4'-thieno[3,2-c]pyran]**

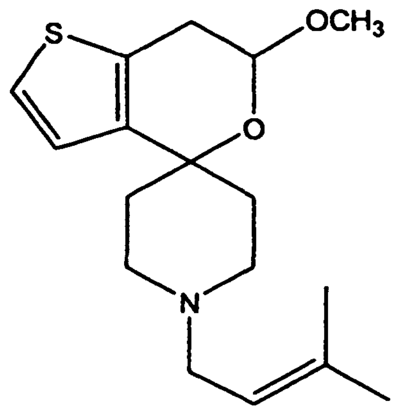

Experimental procedure

Example 2 (59 mg, 0.24 mmol) was dissolved in $CH_3CN$ (5 mL), mixed with 1-Bromo-3-methyl-2-butene (45 mg, 0.3 mmol) and $K_2CO_3$ (200 mg) and heated for 24 h under reflux. For purification $K_2CO_3$ was removed by a frit. After addition of saturated NaCl-solution (5 mL) it was extracted three times with $Et_2O$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.15).

Colourless crystalline solid, Mp. 40 °C, yield: 16 mg (21 %).

$C_{17}H_{25}NO_2S$ (307.5)

HPLC: Method 1: 97.5 %, $R_t$ = 17.8 min
Method 2:

MS (EI): m/z = 307 [$M^+$], 276 [$M^+$ -$OCH_3$ ], 239 [$M^+$ -$CH_2CH=C(CH_3)_2$].

IR (Film): ν ($cm^{-1}$) = 3096 (C-H), 2813 (C-H), 1675 (C=C), 1058 (C-O), 744 (C-H).

$^1$H-NMR ($CDCl_3$): δ (ppm) = 1.60 (s, 3 H, N-$CH_2CH=C(CH_3)_2$), 1.68 (s, 3 H, N-$CH_2CH=C(CH_3)_2$), 1.79 (dd, J = 13.7/2.7 Hz, 1 H, N($CH_2CH_2)_2$), 1.84 (td, J = 12.5/4.5 Hz, 1H, N($CH_2CH_2)_2$), 1.91 (dd, J = 13.7/2.5 Hz, 1 H, N($CH_2CH_2)_2$), 2.05 (td, J = 13.2/4.4 Hz, 1 H, N($CH_2CH_2)_2$), 2.32 (td, J = 12.0/3.3 Hz, 1 H, N($CH_2CH_2)_2$), 2.41 (td, J = 12.6/2.6 Hz, 1 H, N($CH_2CH_2)_2$), 2.76 - 2.81 (m, 3 H (N($CH_2CH_2)_2$) (Th-$CH_2CHOCH_3$), 2.90 - 2.97 (m, 3 H (Th-$CH_2CHOCH_3$) (N-$CH_2CH=C(CH_3)_2$)). 3.52 (s, 3 H, Th-$CH_2CHOCH_3$), 4.82 (dd, J = 7.1/3.3 Hz, 1 H, Th-$CH_2CHOCH_3$), 5.25 (t, J = 7.8 Hz, 1 H, N-$CH_2CH=C(CH_3)_2$), 6.73 (d, J = 5.1 Hz, 1 H, 4-*H*-Th), 7.03(d, J = 5.1 Hz, 1 H, 5-H-Th).

**Example 8: 1-Butyl-6'-methoxy-6',7'-dihydrospiro[piperidin-4,4'-thieno[3,2-c]pyran]**

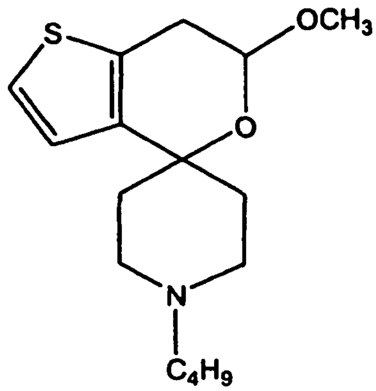

Experimental procedure

Example 2 (90 mg, 0.37mmol) was dissolved in $CH_3CN$ (8 mL), mixed with butylbromide (79 mg, 0.57 mmol) and $K_2CO_3$ (300 mg) and heated for 24 h under reflux. For purification $K_2CO_3$ was removed over a frit, and the solution transferred to separating funnel. After addition of a saturated NaCl-solution (10 mL) it was extracted three times with $Et_2O$ (5 mL). The organic phases were pooled and dried over $Na_2SO_4$, then filtered and the solvent removed under vacuum. The crude product (approx. 110 mg) was purified using flash-chromatography (1 cm, Cyclohexane:ethylacetate 7:3, 10 mL, $R_f$ = 0.14).

Colourless crystalline solid, Mp. 47 ˚C, yield 70 mg (65 %).

$C_{16}H_{25}NO_2S$ (295.4)

| | |
|---|---|
| HPLC: | Method 1: 97.4 %, $R_t$ = 16.9 min<br>Method 2: |
| MS (EI): | m/z = 295 [$M^+$], 264 [$M^+$-$OCH_3$ ], 252 [$M^+$ -$C_3H_7$]. |
| IR (Film): | v ($cm^{-1}$) = 2926 (C-H); 2807 (N-C-*H*), 1060 (C-O), 719 (C-H). |
| $^1$H-NMR ($CDCl_3$): | δ (ppm) = 0.94 (t, J = 7.2 Hz, 3H, N-$CH_2CH_2CH_2CH_3$), 1.36 (sext, J = 7.2 Hz, 2H, N-$CH_2CH_2CH_2CH_3$), 1.54 (quin, J = 7.2 Hz, 2H, N-$CH_2CH_2CH_2CH_3$), 1.85 (dd, J = 13.7/2.6 Hz, 1 H, N($CH_2CH_2$)$_2$), 1.93 (td, J = 14.2/4.4 Hz, 1 H, N($CH_2CH_2$)$_2$), 1.99 (dd, J = 13.7/2.6 Hz, 1 H, N ($CH_2CH_2$)$_2$), 2.14 (td, J = 13.2/4.5 Hz, 1 H, N($CH_2CH_2$)$_2$), 2.37 - 2.45 (m,3 H (N($CH_2CH_2$)$_2$) ($CH_2CH_2CH_2CH_3$)), 2.49 (td, J = 12.4/2.6 Hz, 1 H, N($CH_2CH_2$)$_2$), 2.80 - 2.89 (m, 3 H (N($CH_2CH_2$)$_2$) (Th-$CH_2CHOCH_3$)), 2.99 (dd, J = 15.6/3.3 Hz, 1 H, Th-$CH_2CHOCH_3$), 3.57 (s, 3 H, Th-$CH_2CHOCH_3$), 4.88 (dd, J = 7.8/3.3 Hz, 1 H, Th-$CH_2CHOCH_3$), 6.80(d, J = 5.1 Hz, 1 H, 4-*H*-Th), 7.08 (d, J = 5.4 Hz, 1 H, 5-*H*-Th). |

**Example 9: 6'-Methoxy-1-pentyl-6',7'-dihydrospiro[piperidin-4,4'-thieno[3,2-c]pyran]**

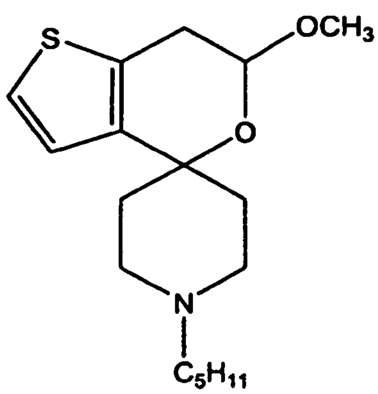

Experimental procedure

Example 2 (62 mg, 0.26 mmol) was dissolved in $CH_2Cl_2$ (5 mL), mixed with valeraldehyde (24 mg, 0.28 mmol) and $NaBH(OAc)_3$ (80 mg, 0.38 mmol) and stirred for 2 h at RT. After the finishing of the reaction saturated $NaHCO_3$-solution (5 mL) was added and three times extracted with $CH_2Cl_2$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.2).
Colourless crystalline solid, Mp. 43 ˚C, yield 69 mg (88 %).
$C_{17}H_{27}NO_2S$ (309.5)

HPLC: Method 1: 99.1 %, $R_t$ = 18.4 min
Method 2:

MS (EI): m/z = 309 [$M^+$], 278 [$M^+$ -$OCH_3$], 252 [$M^+$ -$C_4H_9$].

IR (Film): ν ($cm^{-1}$) = 3098 (C-H), 2926 (C-H), 1058 (C-O), 744 (C-H).

$^1$H-NMR ($CDCl_3$): δ (ppm) = 0.84 (t, J = 6.6 Hz, 3 H, N-$CH_2CH_2CH_2CH_2C\mathit{H}_3$), 1.23 - 1.28 (m, 4 H, N-$CH_2CH_2C\mathit{H}_2C\mathit{H}_2CH_3$), 1.42 - 1.52 (m, 2 H, N-$CH_2C\mathit{H}_2CH_2CH_2CH_3$), 1.78 (dd, J = 13.7/2.7 Hz, 1 H, N($CH_2C\mathit{H}_2)_2$), 1.84 (td, J = 12.6/4.3 Hz, 1 H, N($CH_2C\mathit{H}_2)_2$), 1.92 (dd, J = 14.2/2.8 Hz, 1 H, N ($CH_2C\mathit{H}_2)_2$), 2.05 (td, J = 13.2/4.4 Hz, 1 H, N($CH_2C\mathit{H}_2)_2$), 2.28 - 2.35 (m, 3 H (N-$C\mathit{H}_2CH_2CH_2CH_2CH_3$) (N($C\mathit{H}_2CH_2)_2$)), 2.39 (td, J = 12.6/2.6 Hz, 1 H, N($C\mathit{H}_2CH_2)_2$), 2.73 - 2.81 (m, 3 H (N($C\mathit{H}_2CH_2)_2$) (Th-$C\mathit{H}_2CHOCH_3$), 2.92 (dd, J = 15.6/3.2 Hz, 1 H, Th-$C\mathit{H}_2CHOCH_3$), 3.51 (s, 3 H, Th-$CH_2CHOC\mathit{H}_3$), 4.82 (dd, J = 7.4/3.1 Hz, 1 H, Th-$CH_2C\mathit{H}OCH_3$), 6.74 (d, J = 5.1 Hz, 1 H, 4-*H*-Th), 7.03 (d, J = 5.5 Hz, 1 H, 5-*H*-Th).

**Example 10: 6'-Methoxy-1-octyl -6',7'-dihydrospiro[piperidin-4,4'-thieno[3,2-c]pyran]**

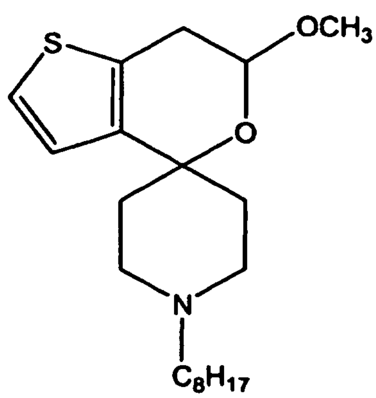

Experimental procedure

Example 2 (180 mg, 0.75 mmol) was dissolved in $CH_3CN$ (20 mL), mixed with octylbromide (174 mg, 0.9 mmol) and $K_2CO_3$ (620 mg) and heated for 24 h under reflux. For purification $K_2CO_3$ was separated over a frit and the solution transferred into a separating funnel. After addition of saturated NaCl-solution (10 mL) it was extracted three times with $Et_2O$ (5 mL). The organic phases were pooled and dried over $Na_2SO_4$, then filtered and the solvent removed under vacuum. The crude product (approx. 237 mg) was purified using flash-chromatography (2 cm, cyclohexane:ethylacetate 7:3, 10 mL, $R_f$ = 0.37).
Colourless crystalline solid, Mp. 48 ˚C, yield 229 mg (87 %).
$C_{20}H_{33}NO_2S$ (351.1)

HPLC: Method 1: 99.1 %, $R_t$ = 21.8 min
Method 2: .

MS (EI): m/z = 351 [$M^+$], 320 [$M^+$ -$OCH_3$ ], 252 [$M^+$ -$C_7H_{15}$].

IR (Film): ν ($cm^{-1}$) = 3092 (C-H), 2918 (C-H), 1058 (C-O), 742 (C-H).

$^1$H-NMR ($CDCl_3$): δ (ppm) = 0.81 (t, J = 6.0 Hz, 3 H, N-$(CH_2)_7$-C$H_3$), 1.12 (m, 10 H, N-$CH_2CH_2$(C$H_2$)$_5$$CH_3$), 1.43 (m, 2H, N-$CH_2$C$H_2$$C_6H_{13}$), 1.78 (dd, J = 13.5/2.2 Hz, 1 H, N($CH_2$C$H_2$)$_2$), 1.88 (td, J = 12.2/4.2 Hz, 1 H, N($CH_2$C$H_2$)$_2$), 1.93 (dd, J = 13.5/2.2 Hz, 1 H, N($CH_2$C$H_2$)$_2$), 2.05 (td, J = 13.2/4.5 Hz, 1 H, N ($CH_2$C$H_2$)$_2$), 2.25 - 2.45 (m, 4 H (N(C$H_2$$CH_2$)$_2$) (N-C$H_2$$(CH_2)_6CH_3$)), 2.77 (dd, J = 15.3/7.2 Hz, 1 H, Th-C$H_2$$CHOCH_3$), 2.75 - 2.80 (m, 2 H, N(C$H_2$$CH_2$)$_2$), 2.94 (dd, J = 15.3/3.3 Hz, 1 H, Th-C$H_2$$CHOCH_3$), 3.51 (s, 3 H, Th-$CH_2$CHOC$H_3$), 4.82 (dd, J = 7.2/3.3 Hz, 1 H, Th-$CH_2$-C$H$$OCH_3$), 6.73 (d, J = 5.4 Hz, 1 H, 4-*H*-Th), 7.02 (d, J = 5.4 Hz, 1 H, 5-*H*-Th).

**Example 11: 3'-Methoxy-1-(3-phenylpropyl)-6',7'-dihydrospiro[piperidin-4,4'-thieno[3,2-c]pyran]**

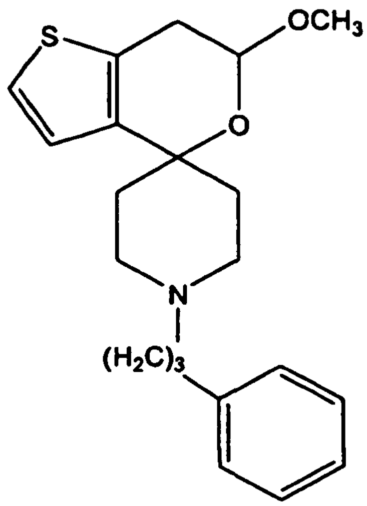

Experimental procedure

Example 2 (120 mg, 0.5 mmol) was dissolved in $CH_3CN$ (15 mL), mixed with 3-phenylpropylbromide (120 mg, 0.6 mmol) and $K_2CO_3$ (414 mg) and heated for 24 h under reflux. For purification $K_2CO_3$ was removed by a frit and the solution transferred into a separating funnel. After addition of saturated NaCl-solution (10 mL) it was extracted three times with $Et_2O$. The organic phases were pooled and dried over Na2SO4, then filtered and the solvent removed under vacuum. The crude product was purified using flash-chromatography (2 cm, cyclohexane:ethylacetate 7:3, 10 mL, $R_f$ = 0.2).

Colourless Solid, Mp. 59 ˚C, yield 68 mg (38 %).

$C_{21}H_{27}NO_2S$ (357.5)

HPLC: Method 1: %, R, = min
Method 2:

MS (EI): m/z = 357 [$M^+$], 326 [$M^+$ -$OCH_3$ ], 252 [$M^+$ -$(CH_2)_2$-Ph].

IR (Film): ν ($cm^{-1}$) = 3097 (C-H), 2913 (C-H), 1062 (C-O), 692 (C-H).

$^1$H-NMR ($CDCl_3$): δ (ppm) = 1.75 - 1.89 (m, 4 H (N($CH_2$C$H_2$)$_2$) (N-$CH_2$C$H_2$$CH_2$-Ph)), 1.92 (dd, J = 13.8/2.8 Hz, 1 H, N($CH_2$C$H_2$)$_2$), 2.04 (td, J = 13.8/4.2 Hz, 1 H, N($CH_2$C$H_2$)$_2$), 2.26 - 2.46 (m, 4 H (N(C$H_2$$CH_2$)$_2$) (N-$CH_2CH_2$C$H_2$-Ph)), 2.59 (t, J = 7.8 Hz, 2 H, N-C$H_2$$CH_2CH_2$-Ph), 2.71 - 2.79 (m, 2 H, N(C$H_2$$CH_2$)$_2$), 2.77 (dd, J = 15.6/7.2 Hz, 1 H, Th-C$H_2$$CHOCH_3$), 2.92 (dd, J = 15.6/3.3Hz, 1 H, Th-C$H_2$$CHOCH_3$), 3.50 (s, 3 H, Th-$CH_2$CHOC$H_3$), 4.81 (dd, J =7.2/3.3 Hz, 1 H, Th-$CH_2$C$H$$OCH_3$), 6.73 (d, J = 5.1 Hz, 1 H, 4-*H*-Th), 7.02 (d, J = 5.4 Hz, 1 H, 5-*H*-Th), 7.10 - 7.25 (m, 5 H, Ph-*H*).

**Example 12: 3'-Methoxy-1-(4-phenylbutyl)-6',7'-dihydrospiro[piperidin-4,4'-thieno[3,2-c]pyran]**

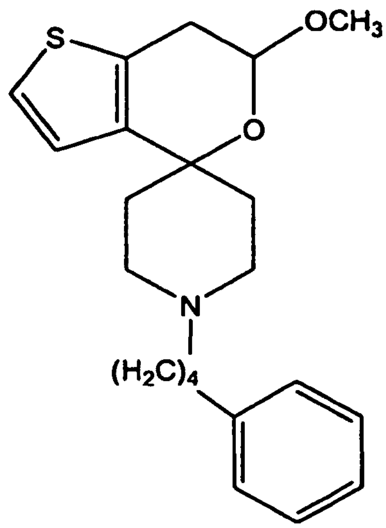

Experimental Procedure

Example 2 (89 mg, 0.36 mmol) was dissolved in $CH_3CN$ (10 mL), mixed with 4-phenylbutylchloride (76 mg, 0.45 mmol) and $K_2CO_3$ (310 mg) and heated for 24 h under reflux. For purification $K_2CO_3$ was removed by a frit and the solution transferred into a separating funnel. After addition of saturated NaCl-solution (10 mL) it was extracted three times with $Et_2O$. The organic phases were pooled and dried over $Na_2SO_4$, then filtered and the solvent removed under vacuum. The crude product (approx. 195 mg) was purified using flash-chromatography (1.5 cm, Cyclohexane:ethylacetate 7:3, 5 mL, $R_f$ = 0.13).
Colourless crystalline solid, Mp. 75 ˚C, yield 46 mg (34 %).
$C_{22}H_{29}NO_2S$ (371.5)

HPLC: Method 1: %, $R_t$ = min
Method 2:

MS (EI): m/z = 371 [$M^+$], 340 [$M^+$ -$OCH_3$ ], 252 [$M^+$ -$(CH_2)_3$Ph].

IR (Film): ν ($cm^{-1}$) = 3096 (C-H), 2922 (C-H), 1059 (C-O), 745 (C-H).

$^1$H-NMR ($CDCl_3$): δ (ppm) = 1.49 - 1.63 (m, 4 H, N-$CH_2CH_2CH_2CH_2$.Ph), 1.78 (dd, J = 13.7/2.7 Hz, 1 H, N($CH_2CH_2$)$_2$), 1.85 (td, J = 12.4/4.5 Hz, 1 H, N($CH_2CH_2$)$_2$), 1.91 (dd, J = 13.7/2.7 Hz, 1 H, N($CH_2CH_2$)$_2$), 2.04 (td, J = 13.3/4.3 Hz, 1 H, N($CH_2CH_2$)$_2$), 2.26 - 2.45 (m, 4 H (N($CH_2CH_2$)$_2$) (N-CH2CH2CH2C$H_2$-Ph)), 2.58 (t, J = 7.2 Hz, 2 H, N-$CH_2(CH_2)_3$-Ph), 2.69 - 2.81 (m, 3 H (N($CH_2CH_2$)$_2$) (Th-$CH_2CHOCH_3$), 2.92 (dd, J = 15.6/3.3 Hz, 1 H, Th-$CH_2CHOCH_3$), 3.51 (s, 3 H, Th-$CH_2CHOCH_3$), 4.81 (dd, J =7.2/3.3 Hz, 1 H, Th-$CH_2CHOCH_3$), 6.72 (d, J = 5.4 Hz, 1 H, 4-*H*-Th), 7.02 (d, J = 5.4 Hz, 1 H, 5-*H*-Th), 7.10 - 7.23 (m, 5 H, Ph-*H*).

**Example 13: 1-(4-Flourbenzyl)-6'-Methoxy -6',7-dihydrospiro[piperidin-4,4'-thieno]3,2-c]pyran]**

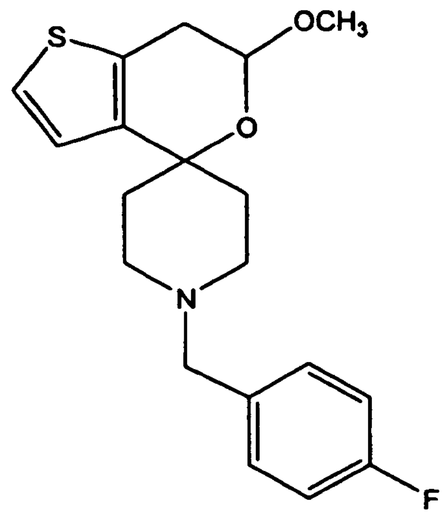

Experimental procedure

Example 2 (129 mg, 0.5 mmol) was dissolved in $CH_3CN$ (20 mL), mixed with 4-fluorobenzylchloride (87 mg, 0.6 mmol) and $K_2CO_3$ (414 mg) and heated for 24 h under reflux. For purification $K_2CO_3$ was removed by a frit and the solution transferred into a separating funnel. After addition of saturated NaCl-solution (10 mL) it was extracted three times with Et2O. The organic phases were pooled and dried over Na2SO4, then filtered and the solvent removed under vacuum. The crude product was purified using flash-chromatography (2 cm, cyclohexane:ethylacetate 7:3, 10 mL, $R_f$ = 0.32).

Colourless crystalline solid, Mp. 83 ˚C, yield 83 mg (48 %).

$C_{19}H_{22}FNO_2S$ (347.4)

| | |
|---|---|
| HPLC: | Method 1: 98.8 %, $R_t$ = 18.6 min<br>Method 2: |
| MS (EI): | m/z = 347 [$M^+$], 316 [$M^+$ -$OCH_3$], 238 [$M^+$ -$CH_2Ph$-$_p$F]. |
| IR (Film): | v ($cm^{-1}$) = 3099 (C-H), 2812 (C-H), 1057 (C-O), 746 (C-H). |
| $^1$H-NMR ($CDCl_3$): | δ (ppm) = 1.76 (dd, J = 13.6/2.7 Hz, 1 H, N($CH_2CH_2$)$_2$), 1.83 (td, J = 12.2/4.0 Hz, 1 H, N($CH_2C$*H*$_2$)$_2$), 1.89 (dd, J = 13.6/2.5 Hz, 1 H, N($CH_2C$*H*$_2$)$_2$), 2.05 (td, J = 13.2/4.5 Hz, 1 H, N($CH_2C$*H*$_2$)$_2$), 2.35 (td, J = 12.6/3.3 Hz, 1 H, N(C*H*$_2CH_2$)$_2$), 2.43 (td, J = 12.6/2.6 Hz, 1 H, N(C*H*$_2CH_2$)$_2$). 2.62 - 2.72 (m, 2 H, N(C*H*$_2CH_2$)$_2$), 2.79 (dd, J = 15.6/7.2 Hz, 1 H, Th-C*H*$_2$CHOCH$_3$), 2.92 (dd, J = 15.6/3.3 Hz, 1 H, Th-C*H*$_2$CHOCH$_3$). 3.44 (d, J = 13.3 Hz, 1 H, N-C*H*$_2$Ph$_{(4-F)}$)), 3.47 (d, J = 13.3 Hz, 1 H, N-C*H*$_2$Ph$_{(4-F)}$), 3.49 (s, 3 H, OC*H*$_3$), 4.80 (dd, J = 7.2/3.3 Hz, 1 H, Th-CH$_2$C*H*OCH$_3$), 6.72 (d, J = 5.1 Hz, 1 H, 4-*H*-Th), 6.91 - 6-98 (m, 2 H, $_{(4-F)}$Ph-*H*), 7.02 (d, J = 5.1 Hz, 1 H, 5-*H*-Th), 7.23 - 7.29 (m. 2 H, $_{(4-F)}$Ph-*H*). |

**Example 14: 6'-Methoxy-1-(4-methoxybenzyl)-6',7'-dihydrospiro[piperidin-4,4'-thieno[3.2-c]pyran]**

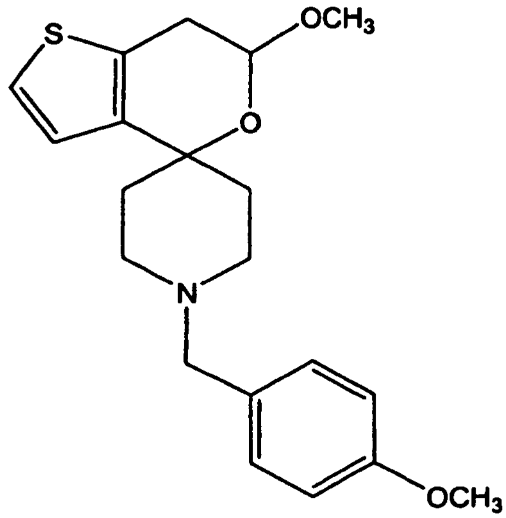

## Experimental procedure

Example 2 (70 mg, 0.29 mmol) was dissolved in $CH_2Cl_2$ (5 mL), mixed with anisaldehyde (45 mg, 0.33 mmol) and $NaBH(OAc)_3$ (75 mg, 0.35 mmol) and stirred for 4 h at RT. After the end of the reaction a solution of $NaHCO_3$ (5 mL) was added and extracted three times with $CH_2Cl_2$ (5 mL). The organic phases were pooled and dried over Na2SO4, then filtered and the solvent removed under vacuum. The crude product (approx. 136 mg) was purified using flash-chromatography (1 cm, cyclohexane:ethylacetate 7:3, 5 mL, $R_f$ = 0.16).

Colourless crystalline solid, Mp. 91 ˚C, yield 94 mg (91 %).

$C_{2O}H_{25}NO_3S$ (359.5)

| | |
|---|---|
| HPLC: | Method 1: 96.8 %, Rt = 18.6 min<br>Method 2: |
| MS (EI): | m/z = 359 [$M^+$], 297 [$M^+$ -$OCH_3$ (2x)], 238 [$M^+$ -$CH_2Ph$-$_pOCH_3$]. |
| IR (Film): | ν ($cm^{-1}$) = 3096 (C-H), 2926 (C-H), 1060 (C-O), 719 (C-H). |
| $^1$H-NMR ($CDCl_3$): | δ (ppm) = 1.75 (dd, J = 13.7/2.5 Hz, 1 H, $N(CH_2CH_2)_2$), 1.83 (td, J = 12.5/4.5 Hz, 1 H, $N(CH_2CH_2)_2$), 1.88 (dd, J = 13.7/2.5 Hz, 1 H, $N(CH_2CH_2)_2$), 2.03 (td, J = 13.2/4.2 Hz, 1 H, $N(CH_2CH_2)_2$), 2.34 (td, J = 12.0/3.1 Hz, 1 H, $N(CH_2CH_2)_2$), 2.43 (td, J = 12.5/2.6 Hz, 1 H, $N(CH_2CH_2)_2$), 2.65 - 2.75 (m, 2 H, $N(CH_2CH_2)_2$), 2.77 (dd, J = 15.6/7.1 Hz, 1 H, Th-$CH_2CHOCH_3$), 2.92 (dd, J = 15.6/3.3 Hz, 1 H, Th-$CH_2CHOCH_3$), 3.43 (d, J = 14.0 Hz, 1 H, N-$CH_2Ph_{(4-OMe)}$), 3.47 (d, J = 14.0 Hz, 1 H, N-$CH_2Ph_{(4-OMe)}$), 3.49 (s, 3 H, Th-$CH_2CHOCH_3$), 3.74 (s, 3 H, Ph-$OCH_3$), 4.80 (dd, J = 7.1/3.3 Hz, 1 H, Th-$CH_2CHOCH_3$), 6.72 (d, J = 5.5 Hz, 1 H, 4-*H* Th), 6.80 (d, J = 8.4 Hz, 2 H, $_{(4-OMe)}$Ph-*H*), 7.02 (d, J = 5.1 Hz, 1H, 5-*H*-Th), 7.20 (d, J = 8.4 Hz, 2 H, $_{(4-OMe)}$Ph-*H*). |

## Example 15: 6'-Methoxy-1-(thien-2-ylmethyl)-6',7'-dihydrospiro[piperidin-4,4'-thieno[3.2-c]pyran]

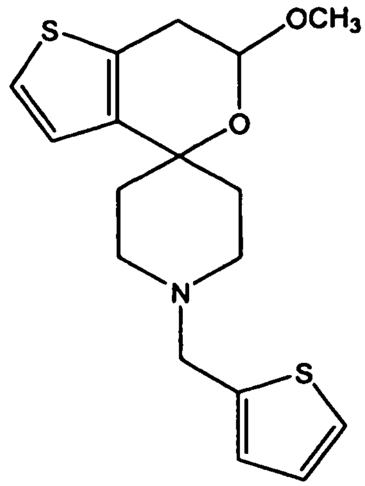

Experimental procedure

Example 2 (58 mg, 0.24 mmol) was dissolved in $CH_2Cl_2$ (5 mL), mixed with thiophene-2-carbaldehyde (31 mg, 0.28 mmol) and $NaBH(OAc)_3$ (80 mg, 0.38 mmol) and stirred for 2 h at RT. After the end of the reaction a saturated solution of $NaHCO_3$ (5 mL) was added and it was extracted three times with $CH_2Cl_2$ (5 mL). The organic phases were pooled and dried over Na2SO4, then filtered and the solvent removed under vacuum. The crude product (approx. 100 mg) was purified using flash-chromatography (1 cm, cyclohexane:ethylacetate 7:3, 5 mL, $R_f$ = 0.34).

Colourless crystalline solid, Mp. 73 ˚C, yield 69 mg (82 %).

$C_{17}H_{21}NO_2S_2$ (335.5)

| | |
|---|---|
| HPLC: | Method 1: 98.3 %, $R_t$ = 17.5 min<br>Method 2: |
| MS (EI): | m/z = 335 [$M^+$], 304 [$M^+$ -$OCH_3$ ], 238 [$M^+$ -$CH_2$Th], 97 [$^+CH_2$Th]. |
| IR (Film): | v ($cm^{-1}$) = 3100 (C-H), 2809 (C-H), 1058 (C-O), 749 (C-H). |
| $^1$H-NMR ($CDCl_3$): | δ (ppm) = 1.77 (dd, J = 13.6/2.7 Hz, 1 H, N($CH_2C\mathit{H}_2$)$_2$), 1.84 (td, J = 12.2/4.3 Hz, 1 H, N($CH_2C\mathit{H}_2$)$_2$), 1.91 (dd, J = 14.0/2.8 Hz, 1 H, N($CH_2C\mathit{H}_2$)$_2$), 2.05 (td, J = 13.2/4.5 Hz, 1 H, N($CH_2C\mathit{H}_2$)$_2$), 2.42 (td, J = 11.8/3.4 Hz, 1 H, N($C\mathit{H}_2CH_2$)$_2$), 2.51 (td, J = 12.6/2.6 Hz, 1 H, N($C\mathit{H}_2CH_2$)$_2$). 2.73 - 2.81 (m, 3 H (N($C\mathit{H}_2CH_2$)$_2$) ($Th_a$-$C\mathit{H}_2CHOCH_3$), 2.91 (dd, J = 15.6/3.1 Hz, 1 H, $Th_a$-$C\mathit{H}_2CHOCH_3$), 3.48 (s, 3 H, $Th_a$ $CH_2CHOC\mathit{H}_3$), 3.72 (d, J = 13.7 Hz, 1 H, N-$C\mathit{H}_2Th_b$) 3.76 (d, J = 13.7 Hz, 1 H, N-$C\mathit{H}_2$-$Th_b$), 4.81 (dd, J = 7.8/3.1 Hz, 1 H, $Th_a$-$CH_2C\mathit{H}OCH_3$), 6.73 (d, J = 5.1 Hz, 1 H, 4-*H*-$Th_a$), 6.88 (m, 2 H (3-*H*-$Th_b$) (4-*H*-$Th_b$)), 7.02 (d, J = 5.5 Hz, 1 H, 5-*H*-$Th_a$), 7.17 (dd, J = 4.7/1.6 Hz, 1 H, 5-*H*-$Th_b$). |

**Example I: 1-(Cyclohexylmethyl)piperidin-4-one**

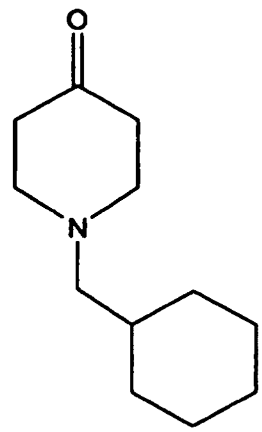

Experimental procedure

Piperidin-4-one hydrochloride monohydrate (0.7 g, 4.55 mmol) was dissolved in $CH_3CN:H_2O$ 1:1 (15 mL), mixed with cyclohexylmethylbromide (0.96 mg, 5.47 mmol) and $K_2CO_3$ (3.8 g) and heated for 34 h under reflux. For purification it was extracted three times with $CH_2Cl_2$ (10 mL) and the organic phase dried over $Na_2SO_4$. Following filtration the solvent was removed under vacuum and the crude product purified by chromatography (3 cm, cyclohexane: ethylacetate 7:3, 15 mL, $R_f$ = 0.40).
Colourless oil, yield 656 mg (74 %).
$C_{12}H_{21}NO$ (195.1)

| | |
|---|---|
| MS (EI): | m/z = 195 [$M^+$, 112 [$M^+$-cHex]. |
| IR (Film): | ν ($cm^{-1}$) = 2919 (C-H), 1718 (C=O), 758 (C-H). |
| $^1H$-NMR ($CDCl_3$): | δ (ppm) = 0.78 - 0.88 (m, 2 H, cHex-*H*), 1.05 - 1.25 (m, 3 H, cHex-*H*), 1.38 - 1.43 (m, 1 H, cHex-H), 1.58 - 1.69 (m. 3 H, cHex-*H*), 1.61 - 1.68 (m, 2 H, cHex-*H*). 2.17 (d, J = 5.4 Hz, 2 H, N-C$H_2$-cHex), 2.38 (t, J = 4.5 Hz, 4 H, N($CH_2CH_2$)$_2$), 2.63 (t, J = 4.5 Hz, 4 H, N($CH_2CH_2$)$_2$). |

**Example J: 1-(3-Methylbutyl)piperidin-4-on**

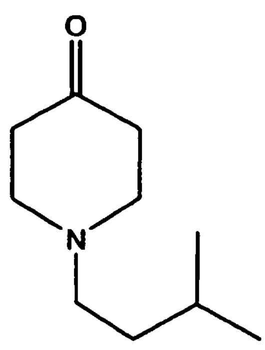

Experimental procedure

Piperidin-4-on hydrochlorid monohydrate (1.5 g, 9.76 mmol) was dissolved in $CH_3CN:H_2O$ 1:1 (50 mL), mixed with 1-bromo-3-methylbutane (1.77 g, 11.7 mmol) and $K_2CO_3$ (8.1 g) and heated for 18 h under reflux. For purification it was extracted three times with $CH_2Cl_2$ (10 mL) and the organic phases dried over $Na_2SO_4$. Following filtration the solvent was removed under vacuum and the crude product purified by flash-chromatography (3 cm, cyclohexane: ethylacetate 7:3, 30 mL, $R_f$ = 0.34).
Colourless oil, yield 1.21 g (73 %).

$C_{10}H_{19}NO$ (169.3)

MS (EI): m/z = 169 [$M^+$], 112 [$M^+$ -$CH_2CH(CH_3)_2$].

IR (Film): ν ($cm^{-1}$) = 2953 (C-H), 1718 (C=O), 756 (C-H).

$^1$H-NMR ($CDCl_3$): δ (ppm) = 0.92 (d, J = 5.1 Hz, 6 H, N-$CH_2CH_2CH(C\mathit{H}_3)_2$), 1.38 - 1.44 (m, 1 H, $CH_2CH_2C\mathit{H}(CH_3)_2$), 1.59 - 1.68 (m, 2 H, N-$CH_2C\mathit{H}_2CH(CH_3)_2$), 2.45 - 2.52 (m, 6 H (N($CH_2C\mathit{H}_2)_2$) (N-$C\mathit{H}_2CH_2CH(CH_3)_2$)), 2.86 (t, J = 4.5 Hz, 4 H, N($C\mathit{H}_2CH_2)_2$).

**Example 16: 1-Benzylspiro[piperidin-4,4'-thieno[3,2-c]pyran]**

**Example 17: 1-Benzyl-6',7'-dihydrospiro[piperidin-4,4'-thieno[3,2-c]pyran]-6'-carbonitrile**

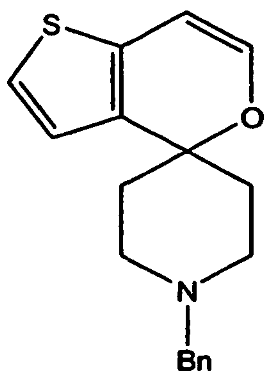

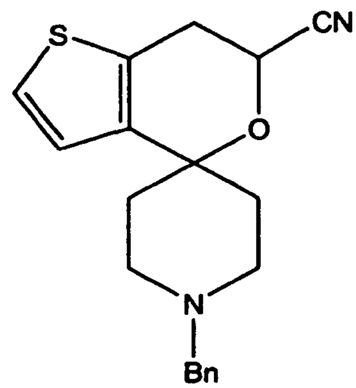

**Experimental procedure**

Example 3 (329 mg, 1 mmol) was dissolved in $CH_2Cl_2$ (20 mL) and cooled to -20 °C under $N_2$. Then TMS-CN (1 mL, 8 mmol) and $BF_3 \cdot Et_2O$ (0.2 mL, 1.6 mmol) were added consecutively through a septum and stirred for 30 min at -20 °C, and subsequently for 1 h at 0 °C in an ice bath. For purification MeOH (3 mL) and 2M NaOH (3 mL) were added (pH > 9) and then extracted three times with $CH_2Cl_2$ (5 mL). The organic phases were pooled and dried over Na2SO4, then filtered and the solvent removed under vacuum. The crude product was purified using flash-chromatography (2 cm, cyclohexane:ethylacetate 7:3, 5 mL, $R_f$ (**11**) = 0.42, $R_f$ (**10**) = 0.34).

**Example 16:** Colourless oil, yield 116 mg (38 %).

$C_{18}H_{19}NOS$ (297.4)

HPLC: Method 1: 97.0 %, $R_t$ = 18.8 min
Method 2:

MS (EI): m/z = 297 [$M^+$], 206 [$M^+$ -$CH_2$Ph], 91 [$^+CH_2$Ph].

IR (Film): ν ($cm^{-1}$) = 3024 (C-H), 2922 (C-H). 1598 (C=C), 1046 (C-O), 696 (C-H).

$^1$H-NMR ($CDCl_3$): δ (ppm) = 1.84 (td, J = 13.4/4.6 Hz, 2 H, N($CH_2C\mathit{H}_2)_2$). 2.16 (dd, J = 14.3/2.1 Hz, 2 H, N($CH_2C\mathit{H}_2)_2$), 2.37 (td, J = 12.5/2.4 Hz, 2 H, N($C\mathit{H}_2CH_2)_2$), 2.68 (m, 2 H, N($C\mathit{H}_2CH_2)_2$), 3.50 (s, 2 H, N-$C\mathit{H}_2$-Ph), 5.71 (d, J = 5.9 Hz, 1 H, Th-C*H*=CH), 6.38 (d, J = 5.9 Hz, 1 H, Th-CH=C*H*), 6.71(d, J = 5.1 Hz, 1 H, 4-*H*-Th), 6.97 (d, J = 5.1 Hz, 1 H, 5-*H*-Th), 7.19 - 7.31 (m, 5 H, Ph-*H*).

**Example 17:** Colourless crystalline solid, Mp. 123 °C, yield 178 mg (55 %).

$C_{19}H_{20}N_2OS$ (324.4)

HPLC: Method 1: 98.7 %, $R_t$ = 18.4 min
Method 2:

MS (EI): *mlz* = 324 [$M^+$], 298 [$M^+$ -CN], 233 [$M^+$ -$CH_2PH$], 91 [$^+CH_2Ph$].

IR (Film): ν ($cm^{-1}$) = 3090 (C-H), 2824 (C-H), 2243 (C≡N), 1067 (C-O), 696 (C-H).

$^1$H-NMR ($CDCl_3$): δ (ppm) = 1.77 (dd, J = 13.8/2.7 Hz, 1 H, $N(CH_2CH_2)_2$),1.80 - 1.89 (m, 2 H, $N(CH_2CH_2)_2$), 2.03 (td, J = 13-2/4.5 Hz, 1 H, $N(CH_2CH_2)_2$), 2.33 (td, J = 11.8/3.1 Hz, 1 H, $N(CH_2CH_2)_2$), 2.43 (td, J = 12.6/2.7 Hz, 1 H, $N(CH_2CH_2)_2$), 2.64 - 2.75 (m, 2 H, $N(CH_2CH_2)_2$), 3.04 (dd, J = 15.6/3.9 Hz, 1 H, Th-$CH_2$CH), 3.16 (dd, J = 15.6/9.0 Hz, 1 H, Th-$CH_2$CH), 3.48 (d, J = 13.0 Hz, 1 H, N-$CH_2$-Ph), 3.53 (d, J = 13.0 Hz, 1 H, N-$CH_2$-Ph), 4.65 (dd, J = 9.0/3.3 Hz, 1 H, Th-$CH_2$C*H*), 6.75 (d, J = 5.1 Hz, 1 H, 4-*H*-Th), 7.09 (d, J = 5.1 Hz, 1 H, 5-*H*-Th), 7.19 - 7.31 (m, 5 H, Ph-*H*).

$^{13}$C-NMR ($CDCl_3$): δ (ppm) = 30.2 (1 C, $N(CH_2CH_2)_2$), 35.6 (1 C, $N(CH_2CH_2)_2$), 37.8 (1 C, Th-$CH_2$CH), 49.0 (2 C, N $(CH_2CH_2)_2$), 58.6 (1 C, Th-$CH_2$CH), 63.6 (1 C, N-$CH_2$-Ph), 76.4 (1 C, Th-CO), 118.6 (1 C, CN), 124.2 (1 C, Th-CH), 124.4 (1 C, Th-CH), 127.3 (1 C, Ph-CH), 128.5 (2 C, Ph-CH), 128.8 (1 C, Ph-C), 129.5 (2 C, Ph-CH), 138.7 (1 C, Th-C), 140.5 (1 C, Th-C).

**Example 20: 1-Benzyl-6',7'-dihydrospiro[piperidin-4,4'-thieno[3,2-c]pyran]-6'-ol**

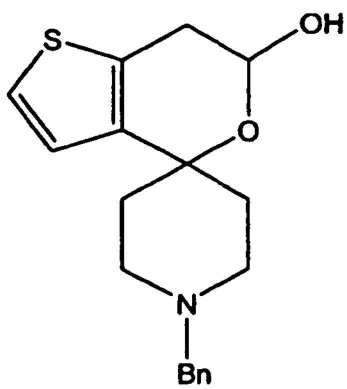

Experimental procedure

Example 3 (330 mg, 1.0 mmol) was dissolved in $CH_3CN$ (7 mL), mixed with 2M HCl (3 mL) and heated for 1 h under reflux. Following that it was alkalised with 2M NaOH (pH > 9) and extracted with $Et_2O$. For a better separation of the phases a few ml of a saturated solution of NaCl was added. The organic phases were pooled and dried over $Na_2SO_4$. Then it was filtered and the solvent removed under vacuum. The purification of the crude product was achieved by re-crystallization from $Et_2O$.
Colourless Solid, Mp. 184 ˚C, yield: 163g (68 %).
$C_{18}H_{21}NO_2S$ (315.4)

MS (EI): m/z =

IR (Film): ν ($cm^{-1}$) = 3100 (C-H), 1038 (C-O), 693 (C-H).

$^1$H-NMR ($CDCl_3$): δ (ppm) = 1.76 (dd, J = 13.6/2.3 Hz, 1 H, $N(CH_2CH_2)_2$), 1.80 - 1.89 (m, 2 H, $N(CH_2CH_2)_2$), 2.04 (td, J = 13.2/4.4 Hz, 1 H, $N(CH_2CH_2)_2$), 2.37 (td, J = 11.7/4.5 Hz, 1 H, $N(CH_2CH_2)_2$), 2.43 (td, J = 11.7/2.5 Hz, 1 H, $N(CH_2CH_2)_2$), 2.62 - 2.72 (m, 2 H, $N(CH_2CH_2)_2$), 2.74 (dd, J = 15.5/7.3 Hz, Th-$CH_2$CH), 2.91 (s, 1 H, O*H*), 3.00 (dd, J = 15.5/3.1 Hz, 1 H, Th-$CH_2$CH), 3.48 (d, J = 14.2 Hz, 1 H, N-$CH_2$Ph), 3.51 (d, J = 14.2 Hz, 1 H, N-$CH_2$Ph), 5.25 (dd, J = 7.3/3.1 Hz, 1 H, Th-$CH_2$C*H*). 6.74 (d, J = 5.2 Hz, 1 H, 4-*H* Th), 7.04 (d, J = 5.2 Hz, 1 H, 5-*H*-Th), 7.16 - 7.29 (m, 5 H, Ph-*H*).

**Example 18: {1-Benzyl-6',7'-dihyrospiro[piperidin-4,4'-thieno[3,2-c]pyran]-6'-yl}acetonitril**

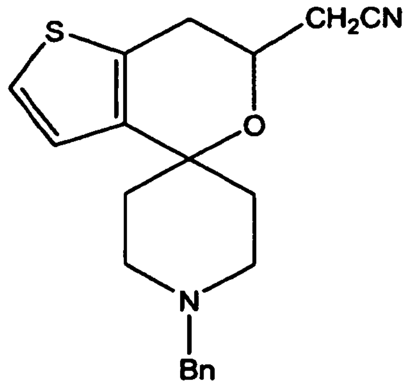

Experimental procedure

Example 20 (91 mg, 0.29 mmol) was dissolved in THF (5 mL), mixed with cyanomethylentriphenyl-phosphorane (126 mg, 0.42 mmol) und $Cs_2CO_3$ (100 mg, 0.3 mmol) and heated for 3.5 h under reflux. Subsequently $H_2O$ (10 mL) was added and a few times extracted with $CH_2Cl_2$. The pooled organic phases were dried over $Na_2SO_4$ and then filtered. After removal of the solvent under vacuum the crude product was purified using flash-chromatography (1.5 cm, cyclohexane:ethylacetate 7:3, 10 mL, $R_f$ = 0.23).
Colourless crystalline solid, Mp. 133 ˚C, yield 92 mg (94 %).
$C_{20}H_{22}N_2OS$ (338.5)

| | |
|---|---|
| HPLC: | Method 1: 98.4 %, $R_t$ = 18.3 min<br>Method 2: |
| MS **(EI):** | m/z = 338 [$M^+$], 298 [$M^+$ -$CH_2CN$], 247 [$M^+$ -$CH_2Ph$]. |
| IR (Film): | ν ($cm^{-1}$) = 3029 (C-H), 2923 (C-H). 2251 (C≡N), 1061 (C-O), 697 (C-H). |
| $^1$H-NMR ($CDCl_3$): | δ (ppm) = 1.62 (dd, J = 13.5/2.6 Hz, 1 H, $N(CH_2CH_2)_2$), 1.79 (td, J = 12.6/4.4 Hz, 1 H, $N(CH_2CH_2)_2$), 1.91 (dd, J = 14.3/2.7 Hz, 1 H, $N(CH_2CH_2)_2$), 2.06 (td, J = 13.1/4.6 Hz, 1 H, $N(CH_2CH_2)_2$), 2.38 - 2.46 (m, 2 H, $N(CH_2CH_2)_2$). 2.62 - 2.70 (m, 5H (Th-$CH_2CHCH_2CN$) ($N(CH_2CH_2)_2$)), 2.80 (dd, J = 15.6/3.1 Hz, 1 H, Th-$CH_2CHCH_2CN$), 3.50 (s, 2 H, N-$CH_2Ph$), 3.98 - 4.06 (m, 1 H, Th-$CH_2CHCH_2CN$), 6.74 (d, J = 5.1 Hz, 1 H, 4-*H*-Th), 7.04 (d, J = 5.1 Hz, 1 H, 5-*H*-Th), 7.18 - 7.32 (m, 5 H, Ph-*H*). |

**Example 19: 1-Benzyl-6',7'-dihydrospiro[piperidin-4,4'-thieno[3,2-c]pyran]**

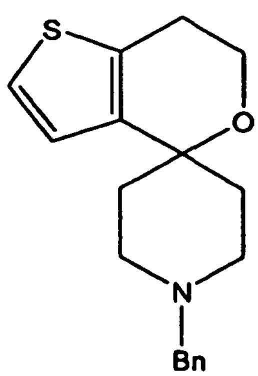

Experimental procedure

Unsaturated example 16 (60 mg, 0.2 mmol) was dissolved in a special pressure resistant flask in MeOH (8 mL) and mixed with Pd/C (15 mg, 20 % (m/m)). The solution was shaken for 24 h under a pressure of 4.5 bar $H_2$ in an hydr Hydrogenation apparatus. After the end of the reaction Pd/C was filtered off and the solvent removed under vacuum. The crude product was purified using flash-chromatography (0.7 cm, cyclohexane:ethylacetate 9:1,5 mL, $R_f$ = 0.20).

Colourless oil, yield 53.8 mg (90 %).

$C_{18}H_{21}NOS$ (299.4)

| | |
|---|---|
| HPLC: | Method 1: 97.2 %, $R_t$ = 18.3 min<br>Method 2: |
| MS (EI): | m/z = 299 [$M^+$], 208 [$M^+$ -$CH_2$PH], 91 [$^+CH_2$Ph]. |
| IR (Film): | ν ($cm^{-1}$) = 3026 (C-H), 2922 (C-H), 1073 (C-O), 697 (C-H). |
| $^1$H-NMR ($CDCl_3$): | δ (ppm) = 1.77 (dd, J = 14.4/2.3 Hz, 2 H, N($CH_2CH_2$)$_2$), 1.91 (td, J = 13.4/4.5 Hz, 2 H, N($CH_2CH_2$)$_2$), 2.33 (td, J = 12.6/2.5 Hz, 2 H, N($CH_2CH_2$)$_2$), 2.64 - 2.67 (m, 2 H, N($CH_2CH_2$)$_2$), 2.75 (t, J = 5.4 Hz, 2 H, Th-$CH_2CH_2$), 3.49 (s, 2 H, N-$CH_2$Ph), 3.85 (t, J = 5.4 Hz, 2 H, Th-$CH_2CH_2$), 6.74 (d, J = 5.5 Hz, 1 H, 4-*H*-Th), 6.98 (d, J = 5.5 Hz, 1 H, 5-*H*-Th), 7.19 - 7.30 (m, 5 H, Ph-H). |

**Example 21: 1-(Cyclohexylmethyl)spiro[piperidine-4,4'-thieno[3,2-c]pyranol** and

**Example 22: 1-(Cyclohexylmethyl)-6',7'-dihydrospiro[piperidine-4,4'-thieno[3,2-c]pyranol-6'-carbonitrile**

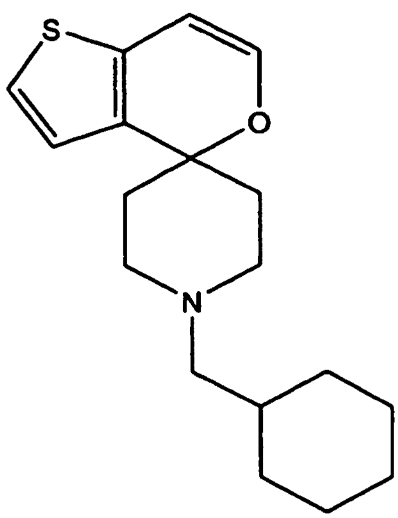

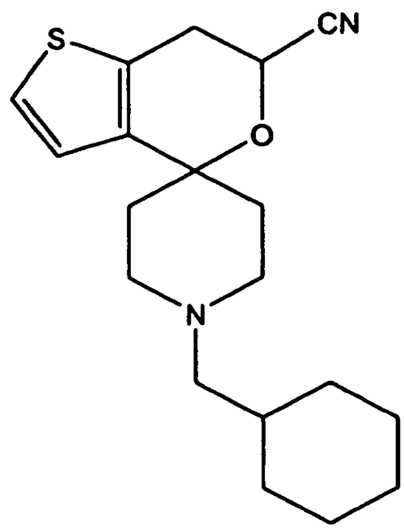

**Example 5** (330 mg, 0.98 mmol) was dissolved in $CH_2Cl_2$ (10 mL) and cooled under $N_2$ to -20 °C. Following that TMS-CN (1 mL, 7.8 mmol) and $BF_3$ •$Et_2O$ (175 μL, 1.4 mmol) were added one after the other through a septum and the mixture stirred for 30 min at -20 °C. For purification 2 M NaOH (3 mL) were added (pH > 9) and it was extracted with $CH_2Cl_2$ (3 x 5 mL). The organic phases were pooled and dried over $Na_2SO_4$. Then it was filtered and the solvent removed under vacuum. Both products were separated from one another using flash-chromatography (3 cm, cHex:EA 8:2, 10 mL, $R_f$ (**EX 21**) = 0.56, $R_f$ (**EX 22**) = 0.30).

**EX 21**: Colourless Solid, MP 75 °C, Yield 91 mg (30 %).
**HPLC:** 96.7 %, $t_R$ = 19.8 min.

$C_{18}H_{25}NOS$ (303.5)

**MS (EI):** m/z = 303 [$M^+$, 220 [$M^+$ -cHex].

| | |
|---|---|
| **IR (Film):** | ν ($cm^{-1}$) = 3043 (C-H), 2926 (C-H), 1592 (C=C), 1047 (C-O), 714 (C-H). |
| **$^1$H-NMR ($CDCl_3$):** | δ (ppm) = 0.84 - 0.95 (m, 2 H, cHex-*H*), 1.13 - 1.30 (m, 3 H, cHex-*H*), 1.45 - 1.55 (m, 1 H, $NCH_2$-C*H* $(CH_2)_5$), 1.63 - 1.74 (m, 3 H, cHex-*H*), 1.75 - 1.84 (m, 2 H, cHex-*H*), 1.89 (td, J = 13.6/4.5 Hz, 2 H, $N(CH_2CH_2)_2$), 2.17 (d, J =7.2 Hz, 2 H, $NCH_2$-cHex), 2.19 (dd, J = 14.4/2.8 Hz, 2 H, $N(CH_2CH_2)_2$), 2.31 (td, J = 12.8/2.8 Hz, 2 H, $N(CH_2CH_2)_2$), 2.66 - 2.72 (m, 2 H, $N(CH_2CH_2)_2$), 5.76 (d, J = 6.4 Hz, 1 H, ThC*H*=CH), 6.44 (d, J = 6.4 Hz, 1 H, ThCH=C*H*). 6.77 (d, J = 5.2 Hz, 1 H, 3'-*H* -Th), 7.03 (d, J = 5.2 Hz, 1 H, 2'-*H*-Th). |
| **EX 22:** | Colourless Solid, MP 111 ˚C, Yield 139 mg (43 %). |
| **HPLC:** | 95.9 %, ambient temperature =19.8 min |

$C_{19}H_{26}N_2OS$ (330.5)

| | |
|---|---|
| **MS (EI):** | m/z = 330 [$M^+$], 247 [$M^+$ -cHex]. |
| **IR (Film):** | ν ($cm^{-1}$) = 2920 (C-H), 1072 (C-O), 647 (C-H). No band for CN is detectable. |
| **$^1$H-NMR ($CDCl_3$):** | δ (ppm) = 0.84 - 0.95 (m, 2 H, cHex-*H*), 1.13 - 1.29 (m, 3 H, cHex-*H*), 1.45 - 1.55 (m, 1 H, $NCH_2$-C*H* $(CH_2)_5$),1.64 - 1.74 (m, 3 H, cHex-*H*), 1.75 - 1.82 (m, 2 H, cHex-*H*), 1.83 - 1.94 (m, 3 H, N $(CH_2CH_2)_2$), 2.08 (td, J = 13.6/4.8 Hz,1 H, $N(CH_2CH_2)_2$), 2.18 (d, J = 7.2 Hz, 2 H, $NCH_2$-cHex), 2.28 (td, J = 11.6/3.2 Hz,1 H, $N(CH_2CH_2)_2$), 2.33 (td, J = 11.6/3.2 Hz, 1 H, $N(CH_2CH_2)_2$), 2.65 - 2.74 (m, 2 H, $N(CH_2CH_2)_2$), 3.09 (dd, J = 15.6/4.0 Hz, 1 H, $ThCH_2CH$), 3.21 (dd, J = 15.6/8.8 Hz. 1 H, $ThCH_2CH$), 4.70 (dd, J = 8.8/4.0 Hz. 1 H, $ThCH_2$C*H*), 6.81 (d, J = 5.2 Hz, 1 H, 3'-*H*-Th), 7.15 (d, J = 5.2 Hz, 1 H, 2'-*H*-Th). |
| **$^{13}$C-NMR ($CDCl_3$):** | δ (ppm) = 26.0 (2 C, cHex-C), 26.6 (1 C, cHex-C), 26.7 (1 C, cHex-C), 29.8 (1 C, $ThCH_2CH$), 31.9 (2 C, cHex-C), 35.1 (1 C, $N(CH_2CH_2)_2$), 37.3 (1 C, $N(CH_2CH_2)_2$), 49.0 (1 C, $N(CH_2CH_2)_2$), 49.1 (1 C, $N(CH_2CH_2)_2$), 58.0 (1 C, $ThCH_2CH$), 65.7 (1 C, $NCH_2$cHex), 76.1 (1 C, ThCO), 118.2 (1 C, CN), 123.7 (1 C, 2'-C-Th), 124.0 (1 C, 3'-GTh), 128.3 (1 C, 7a'-C-Th), 140.3 (1 C, 3a'-C-Th). |

## Example 23: 1-(1-Benzyl-6',7-dihydrospiro[piperidine-4,4'-thieno[3,2-c]pyranol-6'-yl)acetone

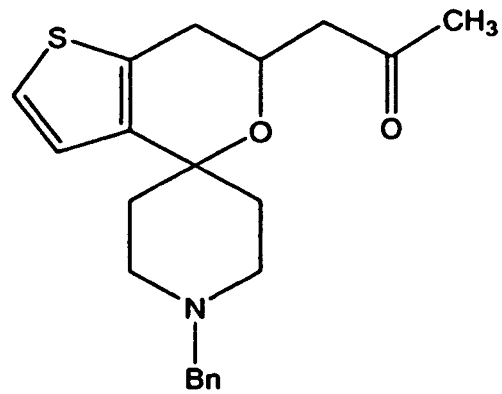

**Example 20** (60 mg, 0.19 mmol) was dissolved in THF (4 mL), Acetylmethylentriphenyl-phosphorane (91 mg, 0.28 mmol) and $Cs_2CO_3$ (67 mg, 0.20 mmol) added and heated for 12 h under reflux. Following that $H_2O$ (10 mL) was added and repeatedly extracted with $CH_2Cl_2$. The organic phases were pooled and dried over $Na_2SO_4$. After removing the solvent under vacuum the crude product was purified using flash-chromatography (1 cm, cHex:EA 7:3, 10 mL, $R_f$ = 0.25). Colourless Oil, Yield 45 mg (67 %).

| | |
|---|---|
| **HPLC:** | 95.6 %, $t_R$ = 18.7 min |
| $C_{21}H_{25}NO_2S$ | (355.5) |
| **MS (EI):** | m/z = 355 [$M^+$], 312 [$M^+$ -$COCH_3$], 264 [$M^+$ -$CH_2Ph$]. |
| **IR (Film):** | ν ($cm^{-1}$) = 3029 (C-H), 2811 (C-H), 1714 (C=O), 698 (C-H). |
| **$^1$H-NMR ($CDCl_3$):** | δ (ppm) = 1.58 (dd, J = 14.2/5.2 Hz, 1 H, $N(CH_2CH_2)_2$), 1.73 (td, J = 12.9/4.4 Hz, 1 H, $N(CH_2CH_2)_2$), |

1.94 (dd, J = 14.2/5.2 Hz, 1 H, N(C*H*$_2$CH$_2$)$_2$), 2.04 (td, J = 13.2/4.2 Hz, 1 H, N(C*H*$_2$CH$_2$)$_2$), 2.17 (td, J = 11.8/2.4 Hz, 1 H, N(C*H*$_2$CH$_2$)$_2$), 2.18 (s, 3 H, C*H*$_3$), 2.30 (td, J = 11.8/2.4 Hz, 1 H, N (C*H*$_2$CH$_2$)$_2$), 2.49 (dd, J = 15.0/4.0 Hz, 1 H, ThCH$_2$CHC*H*$_2$), 2.55 (dd, J = 15.6/10.2 Hz, 1 H, ThC*H*$_2$CHCH$_2$), 2.58 - 2.67 (m, 2 H, N(C*H*$_2$CH$_2$)$_2$), 2.70 (dd, J = 15.6 /3.1 Hz, 1 H, ThC*H*$_2$CHCH$_2$) 2.82 (dd, J = 15.0/8.5 Hz, 1 H, ThCH$_2$CHC*H*$_2$), 3.45 (d, J = 13.0 Hz, 1 H, NC*H*$_2$Ph), 3.48 (d, J = 13.0 Hz, 1 H, NC*H*$_2$Ph), 4.18 (m, 1 H, ThCH$_2$C*H*CH$_2$), 6.72 (d, J = 5.2 Hz, 1 H, 3'-*H*-Th), 6.99 (d, J = 5.2 Hz, 1 H, 2'-*H*-Th), 7.17 - 7.24 (m, 5 H, Ph-*H*).

**Example 24: Methyl-2-(1-benzyl-6',7'-dihyrospiro[piperidin-4,4'-thieno[3,2-c]pyrano]-6'-yl)-acetate**

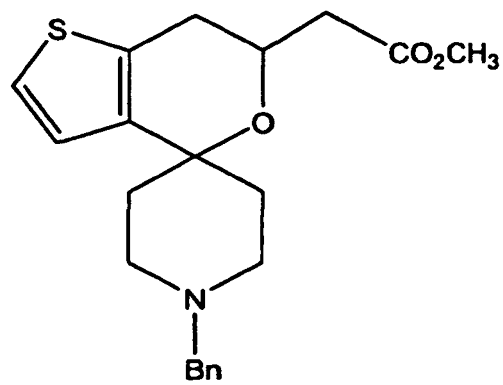

**Example 20** (75 mg, 0.23 mmol) was dissolved in THF (6 mL), methoxycarbonylmethylentriphenylphosphorane (130 mg, 0.38 mmol) and $Cs_2CO_3$ (83 mg, 0.25 mmol) added and heated for 6 h under reflux. Following that a solution of NaCl (10 mL) was added and it was repeatedly extracted with $CH_2Cl_2$. The organic phases were pooled and dried over $Na_2SO_4$. After removing the solvent under vacuum the crude product was purified using flash-chromatography (3 cm, cHex:EA 7:3, 30 mL, $R_f$ = 0.18). Colourless Solid, MP 65 ˚C, Yield 62 mg (70 %).

**HPLC:** 98.8 %, $t_R$ = 19.4 min

$C_{21}H_{25}NO_3S$ (371.5)

**MS (EI):** m/z = 371 [M$^+$, 298 [M$^+$ -CH$_2$CO$_2$CH$_3$], 280 [M$^+$ -CH$_2$Ph], 91 [M$^+$ - CH$_2$Ph].

**IR (Film):** ν (cm$^{-1}$) = 3028 (C-H), 2922 (C-H), 1738 (C=O), 698 (C-H).

**$^1$H-NMR (CDCl$_3$):** δ (ppm) = 1.66 (dd, J = 13.5/2.8 Hz, 1 H, N(CH$_2$C*H*$_2$)$_2$), 1.80 (td, J = 13.0/4.5 Hz, 1 H, N(CH$_2$C*H*$_2$)$_2$), 2.02 (dd, J = 13.5/2.8 Hz, 1 H, N(CH$_2$C*H*$_2$)$_2$), 2.10 (td, J = 13.0/4.5 Hz, 1 H, N(CH$_2$C*H*$_2$)$_2$), 2.25 (td, J = 11.2/2.8 Hz, 1 H, N(C*H*$_2$CH$_2$)$_2$), 2.38 (td, J = 12.8/2.8 Hz, 1 H, N(C*H*$_2$CH$_2$)$_2$), 2.61 (dd, J = 14.8/4.8 Hz, 1 H, ThC*H*$_2$CHCH$_2$), 2.65 (dd, J = 15.6/10.4 Hz, 1 H, ThCH$_2$CHC*H*$_2$), 2.68 - 2.73 (m, 2 H, N(C*H*$_2$CH$_2$)$_2$), 2.70 (dd, J = 14.8/8.8 Hz, 1 H, ThC*H*$_2$CHCH$_2$), 2.79 (dd, J = 15.6/3.2 Hz, 1 H, ThCH$_2$CHC*H*$_2$), 3.51 (d, J = 13.2 Hz, 1 H, NC*H*$_2$Ph), 3.54 (d, J = 13.2 Hz, 1 H, NC*H*$_2$Ph), 3.72 (s, 3 H, CO$_2$C*H*$_3$), 4.21 - 4.29 (m, 1 H, ThCH$_2$C*H*CH$_2$), 6.80 (d, J = 5.2 Hz, 1 H, 3'-*H*-Th), 7.06 (d, J = 5.2 Hz, 1 H, 2'-H-Th), 7.22 - 7.32 (m, 5 H, Ph-*H*).

**Example 25: Ethyl-2-(1-benzyl-6',7'-dihyrospiro[piperidine-4,4'-thieno[3,2-c]pyranol-6'-yl)acetate**

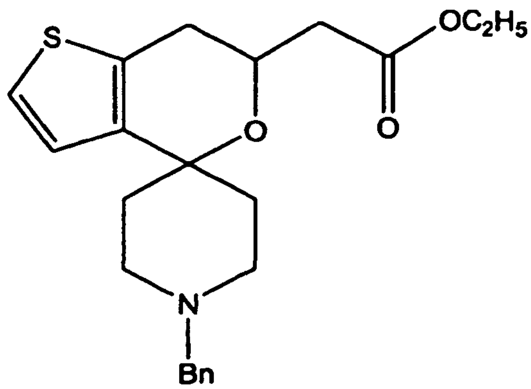

**Example 20** (60 mg, 0.19 mmol) was dissolved in THF (3 mL), Ethoxycarbonylmethylentriphenylphosphorane (129 mg, 0.42 mmol) and KO$^t$Bu (22 mg, 0.19 mmol) added and heated for 10.5 h under reflux. Following that $H_2O$(10 mL) was added and it was repeatedly extracted with $CH_2Cl_2$. The organic phases were pooled and dried over $Na_2SO_4$. After removing the solvent under vacuum the crude product was purified using flash-chromatography (1 cm, cHex:EA 7:3, 10 mL, $R_f$ = 0.18). Colourless Oil, Yield 34 mg (47 %).

**HPLC:** 97.7 %, $t_R$ = 20.4 min

$C_{22}H_{27}NO_3S$ (385.5)

**MS (EI):** m/z = 385 [$M^+$], 298 [$M^+$ -$CH_2CO_2C_2H_5$], 294 [$M^+$ -$CH_2$Ph].

**IR (Film):** ν ($cm^{-1}$) = 3024 (C-H), 2813 (C-H), 1732 (C=O), 698 (C-H).

**$^1$H-NMR ($CDCl_3$):** δ (ppm) = 1.31 (t, J = 7.2 Hz, 3 H, $CO_2CH_2CH_3$), 1.66 (dd, J = 13.5/2.7 Hz, 1H, $N(CH_2CH_2)_2$), 1.81 (td, J = 13.8/4.2 Hz, 1 H, $N(CH_2CH_2)_2$), 2.03 (dd, J = 14.0/2.7 Hz, 1 H, $N(CH_2CH_2)_2$), 2.11 (td, J = 14.2/2.7 Hz, 1 H, $N(CH_2CH_2)_2$), 2.28 (td, J = 13.2/2.2 Hz, 1 H, $N(CH_2CH_2)_2$), 2.39 (td, J = 13.0/2.3 Hz, 1 H, $N(CH_2CH_2)_2$), 2.59 (dd, J = 15.2/4.8 Hz, 1 H, $ThCH_2CHCH_2$), 2.65 - 2.77 (m, 4 H ($N(CH_2CH_2)_2$) ($ThCH_2CHCH_2$)), 2.79 (dd, J = 15.6/3.2 Hz, 1 H, $ThCH_2CHCH_2$), 3.52 (s, 2 H, $NCH_2$Ph), 4.12 - 4.24 (m, 1 H, $ThCH_2CHCH_2$), 4.18 (q, J = 7.2 Hz, 2 H, $CO_2CH_2CH_3$). 6.80 (d, J = 5.2 Hz, 1 H, 3'-*H*-Th), 7.07 (d, J = 5.2 Hz, 1 H, 2'-*H*-Th), 7.18 - 7.25 (m, 5 H, Ph-*H*).

**Example 26: 1-(Cyclohexylmethyl)-6'-7'-dihydrospiro[piperidine-4,4'-thieno[3,2-c]pyrano]**

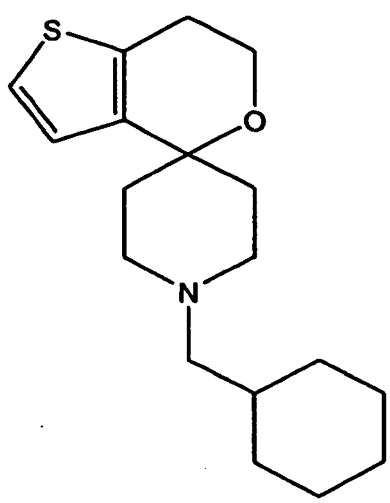

The unsaturated **compound 21** (60 mg, 0.2 mmol) was dissolved in MeOH (8 mL) in a pressure resistant

vessel and Pd/C (15 mg, 10 % (m/m)) added. Under $H_2$-pressure of 4.5 bar the mixture was stirred for 6 h in a hydration apparatus. After the end of the reaction Pd/C was filtered off and the solvent removed in vacuum. The crude product was purified using flash-chromatography (0.7 cm, cHex:EA 9:1, 5 mL, $R_f$ = 0.2). Colourless Solid, MP 77 °C, 31 mg (64 %).

**Analysis of elements:** calc..: C 70.77 H 8.91 N 4.59; found.: C 70.85 H 8.94 N 4.59
**HPLC:** 96.6 %, $t_R$ = 19.1 min

$C_{18}H_{27}NOS$ (305.5)

**MS (EI):** m/z = 305 [$M^+$], 222 [$M^+$ -cHex].

**IR (Film):** v ($cm^{-1}$) = 3092 (C-H), 2925 (C-H), 1074 (C-O), 744 (C-H).

**$^1$H-NMR ($CDCl_3$):** δ (ppm) = 0.83 - 0.95 (m, 2 H, cHex-*H*), 1.13 - 1.30 (m, 3 H, cHex-*H*), 1.45 - 1.55 (m, 1 H, $NCH_2$-C*H* $(CH_2)_5$), 1.63 - 1.74 (m, 3 H, cHex-*H*), 1.75 - 1.83 (m, 2 H, cHex-*H*). 1.83 (dd, J = 14.4/2.8 Hz, 2 H, $N(CH_2CH_2)_2$), 1.96 (td, J = 13.6/4.5 Hz, 2 H, $N(CH_2CH_2)_2$), 2.17 (d, J = 7.2 Hz, 2 H, $NCH_2$cHex), 2.27 (td, J = 12.8/2.8 Hz, 2 H, $N(CH_2CH_2)_2$), 2.65 - 2.71 (m, 2 H, $N(CH_2CH_2)_2$), 2.82 (t, J = 5.6 Hz, 2 H, $ThCH_2CH_2$), 3.92 (t, J = 5.6 Hz, 2 H, $ThCH_2CH_2$), 6.81 (d, J = 5.2 Hz, 1 H, 3'-*H* -Th), 7.06 (d, J = 5.2 Hz, 1 H, 2'-*H*-TH).

**Example 27: 2-(1-Benzyl-6',7'-dihyrospiro[piperidine-4,4'-thieno[3,2-c]pyrano]-6'-yl)ethanol**

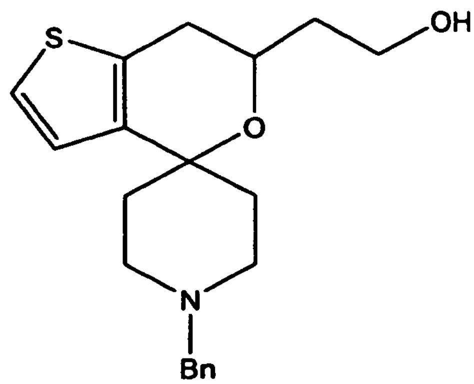

**Example 30** (see below) (25 mg, 0.065 mmol) was dissolved in THF (2 mL) under $N_2$ and cooled to -20 °C. Following careful addition of a solution of $LiAlH_4$ (1 M in THF, 140 μL, 0.13 mmol) it was stirred for 30 min at -20 °Ct. Thereafter, the superfluous $LiAlH_4$ was destroyed by adding a saturated solution of NaCl. After repeated extraction with $CH_2Cl_2$ the organic phases were pooled, dried over $Na_2SO_4$ and filtered. After removing the solvent under vacuum the crude product was purified using flash-chromatography (0.8 cm, cHex:EA 2:8, 5 mL, $R_f$= 0.18). Slightly yellowish resin, Yield 18.3 mg (80 %).

**HPLC:** 98.0 %, $t_R$ = 16.8 min

$C_{20}H_{25}NO_2s$ (343.5)

**MS (EI):** m/z = 343 [$M^+$], 298 [$M^+$ -$(CH_2)_2OH$], 252 [$M^+$ -$CH_2Ph$].

**IR (Film):** v ($cm^{-1}$) = 3364 (O-H), 3028 (C-H), 2922 (C-H), 1056 (C-O), 697 (C-H).

**$^1$H-NMR ($CDCl_3$):** δ (ppm) = 1.70 (dd, J = 13.6/2.8 Hz, 1 H, $N(CH_2CH_2)_2$), 1.87 (td, J = 14.4/4.4 Hz, 1 H, $N(CH_2CH_2)_2$), 1.90 - 1.97 (m, 2 H, $CH_2CH_2OH$), 2.03 (dd, J = 14.4/2.8 Hz, 1 H, $N(CH_2CH_2)_2$), 2.18 (td, J = 13.2/4.8 Hz, 1 H, $N(CH_2CH_2)_2$), 2.32 - 2.42 (m, 2 H, $N(CH_2CH_2)_2$), 2.47 (s, 1 H, O*H*), 2.71 - 2.74 (m, 2 H, $ThCH_2CH$), 2.75 - 2.83 (m, 2 H, $N(CH_2CH_2)_2$), 3.59 (s, 2 H, $NCH_2Ph$), 3.88 (t, J = 5.2 Hz, 2 H, $CH_2CH_2OH$), 3.96 - 4.05 (m, 1 H, $ThCH_2CH$), 6.81 (d, J = 5.2 Hz, 1 H, 3'-*H*-Th), 7.07 (d, J =

5.2 Hz, 1 H, 2'-*H*-Th), 7.24 - 7.37 (m, 5 H, Ph-*H*).

**Reference Example 28:**

**1-Benzylspiro[piperidine-4,4'-thieno[3,2-c]pyrano]-6'(7'H)-one**

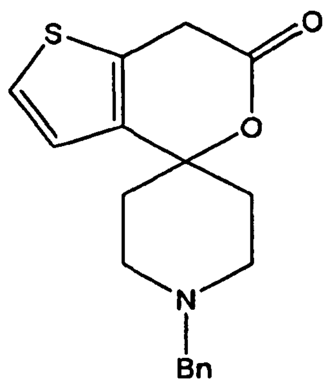

**Example 20** (50 mg, 0.16 mmol) was dissolved in $CH_2Cl_2$ (4 mL) and N-Methylmorpholin-N-oxid (NMO) and $N(iPr)_4RuO_4$ (TPAP) added under $N_2$. After stirring for 2 h at ambient temperature a saturated solution of NaCl (5 mL) was added and extracted with $CH_2Cl_2$ (3 x 5 mL). The organic phase was dried over NaSO4 and filtered thereafter. After removing the solvent under vacuum the crude product was purified using flash-chromatography (0.7 cm, cHex:EA 1:1, 3 mL, $R_f$ = 0.35). Colourless crystalline Solid, MP 153 °C, Yield 33 mg (66 %).

**HPLC:** 99.6 %, $t_R$ = 15.9 min

$C_{18}H_{19}NO_2S$ (313.4)

**MS (EI):** m/z = 313 [$M^+$], 222 [$M^+$ -$CH_2$Ph], 91 [$^+CH_2$Ph].
**IR (Film):** ν ($cm^{-1}$) = 3097 (C-H), 2827 (C-H), 1722 (C=O), 696 (C-H).
**$^1$H-NMR ($CDCl_3$):** δ (ppm) = 1.88 - 1.94 (m, 2 H, N($CH_2CH_2$)$_2$), 2.21 (td, J = 13.3/4.3 Hz, 2 H, N($CH_2CH_2$)$_2$), 2.61 (td, J = 12.5/2.3 Hz, 2 H, N($CH_2CH_2$)$_2$), 2.78 - 2.82 (m, 2 H, N($CH_2CH_2$)$_2$), 3.60 (s, 2 H, N$CH_2$Ph), 3.83 (s, 2 H, Th$CH_2$), 6.88 (d, J = 5.1 Hz, 1 H, 3'-*H*-Th), 7.19 (d, J = 5.1 Hz, 1 H, 2'-*H*-Th), 7.22 - 7.33 (m, 5 H, Ph-*H*).

**Example 29: 1-Benzyl-6',7'-dihyrospiro[piperidine-4,4'-thieno[3,2-c]pyrano]-6'-carboxamide**

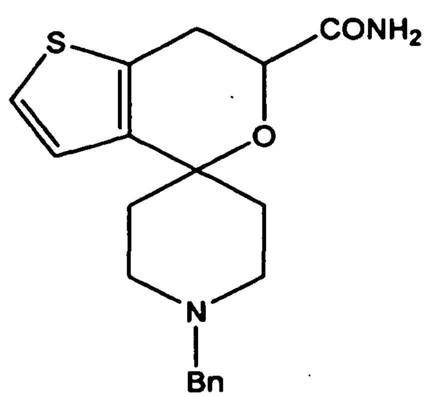

**Example 17** (98 mg, 0.31 mmol) was dissolved in AcOH (3.5 mL) and cooled in ice to 2 °C. Following that

$TiCl_4$ (170 μL, 1.55 mmol) was slowly added under stirring. After 15 min $H_2O$ (66 μL, 3.6 mmol) was added and further stirred 2 h at ambient temperature. For purification 2 M NaOH (20 mL) was added and it was repeatedly extracted with $CH_2Cl_2$. The organic phases were pooled and dried over $Na_2SO_4$ After removing the solvent under vacuum the crude product was purified using flash-chromatography (2 cm, cHex:EA 1.5:8.5, 10 mL, $R_f$ = 0.16). Colourless Solid, MP 63-65 °C, Yield 60 mg (59 %).

**HPLC:** 95.9 %, $t_R$ = 15.7 min

$C_{19}H_{22}N_2O_2S$ (342.5)

**MS (EI):** m/z = 342 [$M^+$], 298 [$M^+$ -$CONH_2$], 251 [$M^+$ -$CH_2$Ph].

**IR (Film):** v ($cm^{-1}$) = 3470 (N-H), 2807 (C-H), 1684 (C=O), 1072 (C-O), 698 (C-H).

**$^1$H-NMR ($CDCl_3$):** δ (ppm) = 1.75 (dd, J = 13.6/2.7 Hz, 1 H, N($CH_2CH_2$)$_2$), 1.87 (td, J = 12.4/4.4 Hz, 1 H, N($CH_2CH_2$)$_2$), 1.97 (dd, J = 14.5/2.8 Hz, 1 H, N($CH_2CH_2$)$_2$), 2.18 (td, J = 13.2/4.6 Hz, 1 H, N($CH_2CH_2$)$_2$), 2.28 (td, J = 12.2/2.9 Hz, 1 H, N($CH_2CH_2$)$_2$), 2.39 (td, J = 11.8/2.6 Hz, 1 H, N($CH_2CH_2$)$_2$), 2.73 - 2.78 (m, 1 H, N($CH_2CH_2$)$_2$), 2.80 - 2.86 (m, 1 H, N($CH_2CH_2$)$_2$), 2.82 (dd, J = 16.0/11.1 Hz, 1 H, Th$CH_2$CH), 3.24 (dd, J = 16.0/3.6 Hz, 1 H, Th$CH_2$CH), 3.57 (s, 2 H, N$CH_2$Ph), 4.27 (dd, J = 11.1/3.6 Hz, 1 H, Th$CH_2$*CH*), 5.55 (s, 1 H, N*H*$_2$), 6.68 (s, 1 H, N*H*$_2$), 6.80 (d, 5.2 Hz, 1 H, 3'-*H*-Th), 7.11 (d, J = 5.2 Hz, 1 H, 2'-*H*-Th), 7.22 - 7.34 (m, 5 H, Ph-*H*).

**Example 30: 1-Benzyl-6',7'-dihyrospiro[piperidine-4,4'-thieno[3,2-c]pyrano]-6'-carbaldehyde**

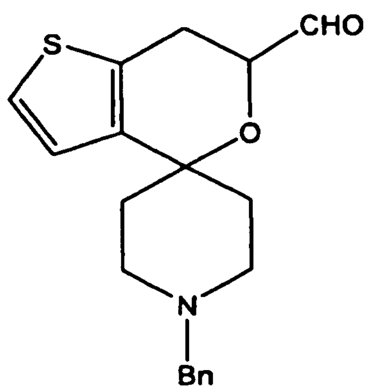

Example 22 (100 mg, 0.3 mmol) was dissolved in toluol (4 mL) and under $N_2$ cooled to -78 °C. After slowly adding of a solution of diisobutylaluminiumhydride (1 M, 0.60 mL, 0.60 mmol) the mixture was stirred for 45 min at -78 °C and a saturated solution of $NH_4Cl$ (5 mL) added. After heating to ambient temperature 2 M NaOH (3 mL) were added under strong stirring and precipitated $Al(OH)_3$ seperated by a frit. Following that it was extracted with $CH_2Cl_2$ (3 × 5 mL) and the pooled organic phases dried over $Na_2SO_4$. After filtration the solvent was removed under vacuum and the crude product purified by flash-chromatography (1 cm, cHex:EA 1:1, 10 mL, $R_f$ = 0.21). Colourless, viscous oil, yield 36 mg (37 %).

$C_{19}H_{21}NO_2S$ (327.4)

**MS (EI):** m/z = 327 [$M^+$], 298 [$M^+$ -CHO], 236 [$M^+$ -$CH_2$Ph].

**IR (Film):** v ($cm^{-1}$) = 2921 (C-H), 1737 (C=O), 1071 (C-O), 697 (C-H).

**$^1$H-NMR ($CDCl_3$):** δ (ppm) = 1.70 (dd, J = 13.7/2.5 Hz, 1 H, N($CH_2CH_2$)$_2$), 1.82 (td, J = 12.2/4.4 Hz, 1 H, N($CH_2CH_2$)$_2$), 1.90 (dd, J = 13.4/2.7 Hz, 1 H, N($CH_2CH_2$)$_2$), 2.12 (td, J = 13.2/4.6 Hz, 1 H, N($CH_2CH_2$)$_2$), 2.33 (td, J = 11.8/3.1 Hz, 1 H, N($CH_2CH_2$)$_2$), 2.48 (td, J = 11.4/2.5 Hz, 1 H, N($CH_2CH_2$)$_2$), 2.60 - 2.80 (m, 3 H (Th$CH_2$CH) (N($CH_2CH_2$)$_2$)), 2.91 (dd, J = 15.9/3.8 Hz, 1 H, Th$CH_2$CH), 3.51 (s, 2 H, N$CH_2$Ph)), 4.13 (dd, J = 10.8/3.8 Hz, 1 H, Th$CH_2$*CH*), 6.75 (d, J = 5.2 Hz, 1 H, 3'-*H* -Th), 7.04 (d, J = 5.2 Hz, 1 H, 2'-*H*-Th), 7.18 - 7.28 (m, 5 H, Ph-*H*), 9.80 (s, 1 H, C*H*O).

**Example 31: (1-Benzyl-6',7'-dihydrospiro[piperidine-4,4'-thieno[3,2-c]pyrano]-6'-yl)methanamine**

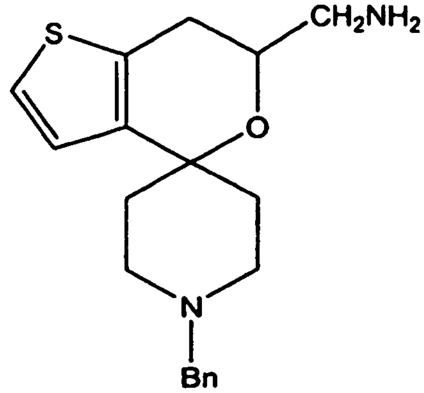

**Example 22** (100 mg, 0.31 mmol) was dissolved in THF (4 mL) under $N_2$ and cooled to -10 ˚C. After careful addition of $LiAlH_4$ (24 mg, 0.62 mmol) the mixture was stirred for 1 h at -10 ˚C. Following that it was alkalised with 2 M NaOH and precipitated $Al(OH)_3$ removed through a frit. After repeated extraction of the filtrate with $CH_2Cl_2$ the organic phases were pooled, dried over $Na_2SO_4$ and filtered. After removing the solvent under vacuum the crude product was purified using flash-chromatography (1 cm, cHex:EA:MeOH 1:7:2, 10 mL, $R_f$ = 0.28). Slightly yellowish resin, Yield 57 mg (57 %).

**HPLC:** 95.1 %, $t_R$ = 13.5 min

$C_{19}H_{24}N_2OS$ (328.5)

**MS (EI):** m/z = 328 [$M^+$], 298 [$M^+$ -$CH_2NH_2$], 237 [$M^+$ -$CH_2$Ph].

**IR (Film):** ν ($cm^{-1}$) = 3328 (N-H), 3025 (C-H), 2919 (C-H), 1070 (C-O), 697(C-H).

**$^1$H-NMR ($CDCl_3$):** δ (ppm) = 1.62 (dd, J = 13.5/2.7 Hz, 1 H, N($CH_2CH_2$)$_2$), 1.77 (td, J = 13.2/4.4 Hz, 1 H, N($CH_2CH_2$)$_2$), 1.95 (dd, J = 14.4/2.7 Hz, 1 H, N($CH_2CH_2$)$_2$), 2.09 (td, J = 13.1/4.5 Hz, 1 H, N($CH_2CH_2$)$_2$), 2.29 (td, J = 12.6/2.6 Hz, 1 H, N($CH_2CH_2$)$_2$), 2.42 (td, J = 12.6/2.6 Hz, 1 H, N($CH_2CH_2$)$_2$), 2.54 (dd, J = 15.0/10.0 Hz, 1 H, Th$CH_2$CH$CH_2$), 2.61 (dd, J = 15.6/3.7 Hz, 1 H, Th$CH_2$CH$CH_2$), 2.64 - 2.72 (m, 2 H, N($CH_2CH_2$)$_2$), 2.76 - 2.83 (m, 2 H, Th$CH_2$CH$CH_2$), 3.50 (s, 2 H, N$CH_2$Ph), 3.66 - 3.72 (m, 1 H, Th$CH_2$CH$CH_2$), 6.74 (d, J = 5.2 Hz, 1 H, 3'-*H*-Th), 7.00 (d, J = 5.2 Hz, 1 H, 2'-*H*-Th), 7.18 - 7.27 (m, 5 H, Ph-*H*). The signals of the 2 protons of the $NH_2$-group are not visible.

**Example 32: Methyl-1-benzyl-6',7'-dihyrospiro[piperidine-4,4'-thieno[3,2-c]pyrano]-6'-carboxylate**

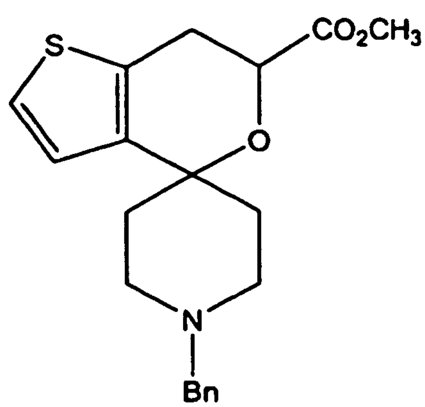

**Example 22** (58 mg, 0.18 mmol) was dissolved in MeOH (3 mL), $H_2SO_4$ conc. (1 g) and 3 drops of $H_2O$ were

added and heated for 40 h under reflux. After finishing the reaction the mixture was neutralized with 2 M NaOH under cooling with ice (pH 7-8) and following addition of a saturated solution of NaCl repeatedly extracted with $Et_2O$. The organic phases were pooled and dried over $Na_2SO_4$. After removing the solvent under vacuum the crude product was purified using flash-chromatography (1 cm, cHex:EA 8:2, 5 mL, $R_f$ = 0.13). Colourless Oil, Yield 37 mg (56 %).

**HPLC:** 96.9 %, $t_R$ = 18.5 min

$C_{20}H_{23}NO_3S$ (357.5)

**MS (EI):** m/z = 357 [$M^+$], 298 [$M^+$ -$CO_2CH_3$], 266 [$M^+$ -$CH_2$Ph].
**IR (Film):** ν ($cm^{-1}$) = 3024 (C-H), 2923 (C-H), 1760 (C=O), 697 (C-H).
**$^1$H-NMR ($CDCl_3$):** δ (ppm) = 1.73 (dd, J = 13.5/2.8 Hz, 1 H, N($CH_2CH_2$)$_2$), 1.78 (td, J = 12.6/4.4 Hz, 1 H, N($CH_2C\mathit{H}_2$)$_2$), 1.90 (dd, J = 14.5/2.8 Hz, 1 H, N($CH_2C\mathit{H}_2$)$_2$), 2.07 (td, J = 13.1/4.6 Hz, 1 H, N($CH_2C\mathit{H}_2$)$_2$), 2.33 (td, J = 12.5/ 2.8 Hz, 1 H, N($C\mathit{H}_2CH_2$)$_2$), 2.48 (td, J = 12.7/2.6 Hz, 1 H, N($C\mathit{H}_2CH_2$)$_2$), 2.60 - 2.65 (m, 1 H, N($C\mathit{H}_2CH_2$)$_2$), 2.67 - 2.72 (m, 1 H, N($C\mathit{H}_2CH_2$)$_2$), 2.94 (dd, J = 15.8/9.6 Hz, 1 H, ThC$\mathit{H}_2$CH), 2.98 (dd, J = 15.8/4.8 Hz, 1 H, ThC$\mathit{H}_2$CH), 3.48 (d, J = 13.0 Hz, 1 H, NC$\mathit{H}_2$Ph), 3.52 (d, J = 13.0 Hz, 1 H, NC$\mathit{H}_2$Ph), 3.76 (s, 3 H, $CO_2C\mathit{H}_3$), 4.32 (dd, J = 9.6/4.8 Hz, 1 H, Th$CH_2$C*H*), 6.74 (d, J = 5.2 Hz, 1 H, 3'-*H*-Th), 7.03 (dd, J = 5.2 Hz, 1 H, 2'-*H*-Th), 7.15 - 7.28 (m, 5 H, Ph-*H*).

**Example 33: Etyhl-1-benzyl-6',7'-dihyrospiro[piperidine-4,4'-thieno[3,2-c]pyrano]-6'-carboxylate**

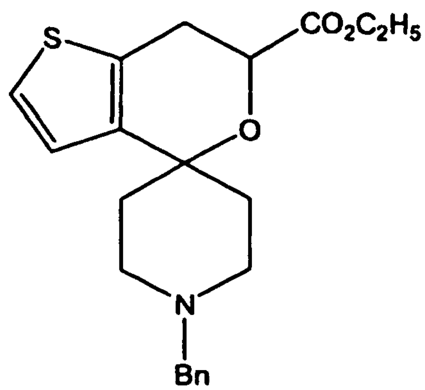

**Example 22** (59 mg, 0.18 mmol) was dissolved in EtOH (3 mL), $H_2SO_4$ conc. (1 g) and 3 drops of $H_2O$ added and heated for 18 h under reflux. After finishing the reaction the mixture was neutralized with 2 M NaOH under cooling with ice (pH 7-8) and following addition of saturated solution of NaCl repeatedly extracted with $Et_2O$. The organic phases were pooled and dried over $Na_2SO_4$. After removing the solvent under vacuum the crude product was purified using flash-chromatography (1 cm, cHex:EA 7:3, 5 mL, $R_f$ = 0.18). Colourless oil, yield 49 mg (71 %).

**HPLC:** 98.6 %, $t_R$ = 19.4 min

$C_{21}H_{25}NO_3S$ (371.5)

**MS (EI):** m/z = 371 [$M^+$], 298 [$M^+$ -$CO_2C_2H_5$], 280 [$M^+$ -$CH_2$Ph].
**IR (Film):** ν ($cm^{-1}$) = 3029 (C-H), 2924 (C-H), 1734 (C=O), 697 (C-H).
**$^1$H-NMR ($CDCl_3$):** δ (ppm) = 1.26 (t, J = 7.2 Hz, 3 H, $CO_2CH_2C\mathit{H}_3$), 1.72 (dd, J = 13.5/2.7 Hz, 1 H, N($CH_2C\mathit{H}_2$)$_2$), 1.77 (td, J = 12.6/4.4 Hz, 1 H, N($CH_2C\mathit{H}_2$)$_2$), 1.90 (dd, J = 14.4/2.8 Hz, 1 H, N($CH_2C\mathit{H}_2$)$_2$), 2.07 (td, J = 13.5/4.6 Hz, 1 H, N($CH_2C\mathit{H}_2$)$_2$), 2.34 (td, J = 12.6/ 2.6 Hz, 1 H, N($C\mathit{H}_2CH_2$)$_2$), 2.49 (td, J = 12.5/2.6 Hz, 1 H N($C\mathit{H}_2CH_2$)$_2$), 2.58 - 2.64 (m, 1 H, N($C\mathit{H}_2CH_2$)$_2$), 2.66 - 2.71 (m, 1 H, N ($C\mathit{H}_2CH_2$)$_2$), 2.93 (dd, J = 15.8/9.6 Hz, 1 H, ThC$\mathit{H}_2$CH), 2.98 (dd, J = 15.8/4.8 Hz, 1 H, ThC$\mathit{H}_2$CH), 3.48 (d, J = 13.0 Hz, 1 H, NC$\mathit{H}_2$Ph), 3.52 (d, J = 13.0 Hz, 1 H, NC$\mathit{H}_2$Ph), 4.08 - 4.18 (m, 2 H, $CO_2C\mathit{H}_2CH_3$), 4.29 (dd, J = 9.6/4.8 Hz, 1 H, Th$CH_2$C*H*), 6.74 (d, J = 5.2 Hz, 1 H, 3'-*H*-Th), 7.03 (dd, J = 5.2 Hz, 1 H, 2'-*H*-Th), 7.15 - 7.28 (m, 5 H, Ph-*H*).

**Example 34: (1-Benzyl-6',7'-dihyrospiro[piperidine-4,4'-thieno[3,2-c]pyrano]-6'-yl)methanol**

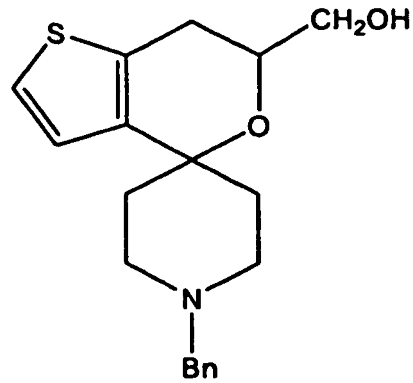

**Example 30** (85 mg, 0.26 mmol) was dissolved in MeOH (3 mL) and under cooling with ice $NaBH_4$ (20 mg, 0.52 mmol) was added. After 15 min of stirring at 0 °C the mixture was stirred a further 45 min at ambient temperature. Following that a saturated solution of NaCl was added and the mixture was extracted 3 times with 5 mL $CH_2Cl_2$. The organic phases were pooled and dried over $Na_2SO_4$. After removing the solvent under vacuum the crude product was purified using flash-chromatography (1 cm, cHex:EA 7:3, 10 mL, $R_f$ = 0.08). Colourless solid, MP 108 °C, Yield 60 mg (70 %).

**HPLC:** 98.9 %, $t_R$ = 16.5 min
$C_{19}H_{23}NO_2S$ (329.5)
**MS (EI):** m/z = 329 [$M^+$], 298 [$M^+$ -$CH_2OH$], 238 [$M^+$ -$CH_2Ph$].
**IR (Film):** ν ($cm^{-1}$) = 3428 (O-H), 3028 (C-H), 2849 (C-H), 1054 (C-O), 697 (C-H).
**$^1$H-NMR ($CDCl_3$):** δ (ppm) = 1.69 (dd, J = 13.6/2.8 Hz, 1 H, N($CH_2CH_2$)$_2$), 1.83 (td, J = 12.8/4.4 Hz, 1 H, N($CH_2CH_2$)$_2$), 2.02 (dd, J = 13.6/2.8 Hz, 1 H, N($CH_2CH_2$)$_2$), 2.15 (td, J = 13.2/4.8 Hz, 1 H, N($CH_2CH_2$)$_2$), 2.30 (td, J = 12.8/2.4 Hz, 1 H, N($CH_2CH_2$)$_2$), 2.44 (td, J = 12.0/2.4 Hz, 1 H, N($CH_2CH_2$)$_2$), 2.64 (dd, J = 16.0/4.0 Hz, 1 H, Th$CH_2$), 2.67 - 2.78 (m, 3 H (N($CH_2CH_2$)$_2$) (Th$CH_2$)), 3.55 (s, 2 H, N$CH_2$Ph), 3.70 (dd, J = 11.0/7.3 Hz, 1 H, Th$CH_2$CH$CH_2$), 3.78 - 3.84 (m, 1 H, Th$CH_2$CH$CH_2$), 3.90 - 3.97 (m, 1 H, Th$CH_2$CHCH$_2$), 6.81 (d, J = 5.2 Hz, 1 H, 3'-*H*-Th), 7.08 (d, J = 5.2 Hz, 1 H, 2'-*H*-Th), 7.23 - 7.34 (m, 5 H, Ph-*H*). A signal for the proton of the OH-group was not detectable.

**Example 35: (1-Benzyl-6',7'-dihydrospiro[piperidine-4,4'-thieno[3,2-c]pyrano]-6'-yl)-N,N-dimethylmethanamine**

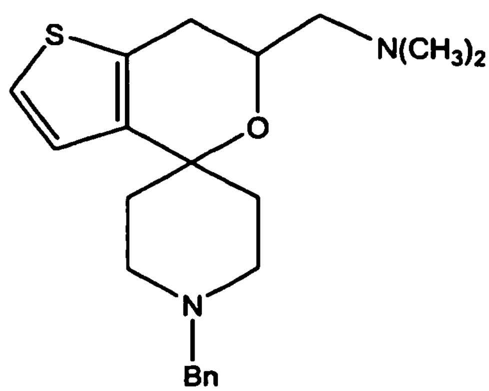

**Example 30** (70 mg, 0.22 mmol) was dissolved in $CH_2Cl_2$ (2 mL), $NaBH(OAc)_3$ (70 mg, 0.34 mmol) and a solution of dimethylamine (2 M in THF, 120 μl, 0.24 mmol) added. After stirring for 2 h at ambient temperature a saturated solution of $NaHCO_3$ (3 mL) was added and the mixture was extracted repeatedly with $CH_2Cl_2$. The organic phases were pooled and dried over $Na_2SO_4$. After removing the solvent under vacuum the crude product was purified using flash-chromatography (1 cm, cHex:EA:MeOH 1:7:2, 10 mL, $R_f$ = 0.24). Colourless Oil, Yield 36 mg (46 %).

**HPLC:** 97.8 %, $t_R$ = 13.9 min

$C_{21}H_{28}N_2OS$ (356.5)

**MS (EI):** m/z = 356 [$M^+$], 298 [$M^+$ -$CH_2N(CH_3)_2$], 265 [$M^+$ -$CH_2Ph$].
**IR (Film):** v ($cm^{-1}$) = 3029 (C-H), 2815 (C-H), 1068 (C-O), 697 (C-H).
**$^1$H-NMR ($CDCl_3$):** δ (ppm) = 1.62 (dd, J = 13.6/2.8 Hz, 1 H, $N(CH_2CH_2)_2$), 1.79 (td, J = 12.8/4.8 Hz, 1 H, $N(CH_2CH_2)_2$), 1.93 (dd, J = 13.6/2.8 Hz, 1 H, $N(CH_2CH_2)_2$), 2.09 (td, J = 13.2/4.4 Hz, 1 H, $N(CH_2CH_2)_2$), 2.29 (s, 6 H, $N(CH_3)_2$), 2.34 - 2.47 (m, 2 H, $N(CH_2CH_2)_2$), 2.52 - 2.58 (m, 3 H, $ThCH_2CHCH_2$), 2.66 - 2.73 (m, 3 H ($N(CH_2CH_2)_2$), ($ThCH_2CHCH_2$)), 3.55 (s, 2 H, $NCH_2Ph$), 3.86 - 3.94 (m, 1 H, $ThCH_2CHCH_2$), 6.75 (d, J = 5.2 Hz, 1 H, 3'-*H*-Th), 7.00 (d, J = 5.2 Hz, 1 H, 2'-*H*-Th), 7.18 - 7.25 (m, 5 H, Ph-*H*).

**Example 36: (1-Benzyl-6',7'-dihydrospiro[piperidine-4,4'-thieno[3,2-c]pyrano]-6'-yl)-N-benzyl-methanamine**

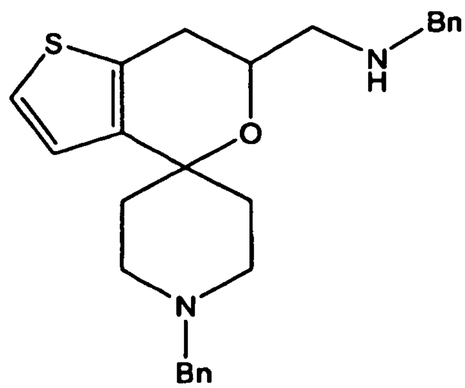

**Example 34** (55 mg, 0.17 mmol) was dissolved in $CH_2Cl_2$ (3 mL), benzaldehyde (17.7 mg, 0.17 mmol) and $NaBH(OAc)_3$ (53 mg, 0.25 mmol) were added and the mixture stirred for 1 h at ambient temperature under $N_2$. After the reaction finished a saturated solution of $NaHCO_3$ (5 mL) was added and the mixture was extracted 3-times with $CH_2Cl_2$ (5 mL). The organic phases were pooled, dried over $Na_2SO_4$, filtered and the solvent removed under vacuum. The crude product was purified using flash-chromatography (1 cm, cHex:EA:MeOH 1.5:8:0.5, 3 mL, $R_f$ = 0.16). Colourless Oil, Yield 17.9 mg (25 %).

**HPLC:** 96.0 %, $t_R$ = 16.8 min

$C_{26}H_{30}N_2OS$ (418.6)

**MS (EI):** m/z = 418 [$M^+$], 327 [$M^+$ -$CH_2Ph$], 298 [$M^+$ -$CH_2NHCH_2Ph$].
**IR (Film):** v ($cm^{-1}$) = 3331 (N-H), 3025 (C-H), 2920 (C-H), 696 (C-H).
**$^1$H-NMR ($CDCl_3$):** δ (ppm) = 1.75 (dd, J = 14.4/2.8 Hz, 1 H, $N(CH_2CH_2)_2$), 1.81 (td, J = 14.4/4.4 Hz, 1 H, $N(CH_2CH_2)_2$), 2.03 (dd, J = 14.4/2.8 Hz, 1 H, $N(CH_2CH_2)_2$), 2.14 (td, J = 14.4/4.4 Hz, 1 H, $N(CH_2CH_2)_2$), 2.31 (td, J = 13.6/2.8 Hz, 1 H, $N(CH_2CH_2)_2$), 2.44 (td, J = 13.6/2.8 Hz, 1 H, $N(CH_2CH_2)_2$), 2.63 - 2.67 (m, 2 H, $ThCH_2CHCH_2$), 2.68 - 2.78 (m, 2 H, $N(CH_2CH_2)_2$), 2.81 (dd, J = 12.0/3.6 Hz, 1 H, $ThCH_2CH$), 2.84 (dd, J = 12.0/8.0 Hz, 1 H, $ThCH_2CH$), 3.50 (d, J = 12.8 Hz, 1 H, $NCH_2Ph$), 3.55 (d, J = 12.8 Hz, 1 H, $NCH_2Ph$), 3.85 (d, J = 13.6 Hz, 1 H, $NHCH_2Ph$), 3.89 (d, J = 13.6 Hz, 1 H, $NHCH_2Ph$), 3.91 - 4.02 (m, 1 H, $ThCH_2CH$), 6.79 (d, J = 5.2 Hz, 1 H, 3'-*H*-Th), 7.06 (dd, J = 5.2 Hz, 1 H, 2'-*H*-Th), 7.21 - 7.38 (m, 10 H, Ph-*H*). A signal for the proton of the NH-group is not detected.

**Example K: 4-Bromo-5-(2,2-dimethoxyethyl)thiophen-2-carbaldehyde**

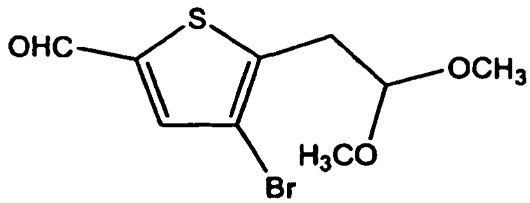

**Example C** (250 mg, 1 mmol) was dissolved under $N_2$ in THF (5 mL) and cooled to - 40 °C. A solution of LDA (2 M in THF, 890 μL, 1.79 mmol) was slowly added while stirring and the mixture stirred for a further 10 min. Thereafter, 1-Formylpiperidine (200 mg, 1.79 mmol), dissolved in THF (0.5 mL) was added. After 30 min the mixture was heated to ambient temperature, a saturated solution of NaCl (5 mL) added and extracted with $CH_2Cl_2$ (3 x 5 mL).The organic phase was dried over Na2SO4, filtered and the solvent removed under vacuum. The crude product was purified using flash-chromatography (3 cm, cHex:EA 9:1, 15 mL, $R_f$ = 0.18). Colourless Liquid, Yield 259 mg (93 %).

**HPLC:** 99.7 %, $t_R$ = 19.9 min

$C_9H_{11}BrO_3S$ (279.2)

**MS (EI):** m/z = 280/278 [$M^+$], 249/247 [$M^+$ -$OCH_3$].
**IR (Film):** ν ($cm^{-1}$) = 3083 (C-H), 2932 (C-H), 1668 (C=O), 1066 (C-O), 661 (C-H).
**$^1$H-NMR ($CDCl_3$):** δ (ppm) = 3.16 (d, J = 5.6 Hz, 2 H, ThC*$H_2$*), 3.40 (s, 6 H, CH(OC*$H_3$*$)_2$), 4.58 (t, J = 5.6 Hz, 1 H, $ThCH_2$C*H*), 7.60 (s, 1 H, 3-*H*-Th), 9.80 (s, 1 H, ThC*H*O).

**Example L: 4-Bromo-5-(2,2-dimethoxyethyl)thiophen-2-carbaldehydedimethylacetal**

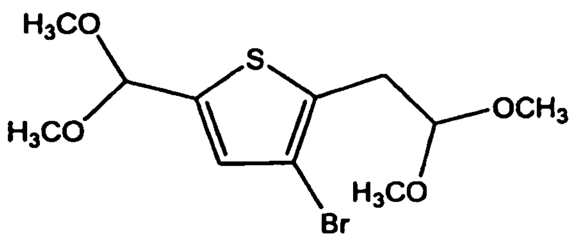

**Example K** (700 mg, 2.5 mmol) was dissolved in methanol (15 mL), Trimethylorthoformiate (3 mL) and p-Toluolsulfonic acid (24 mg, 125 μmol) added and heated for 3 h under reflux. For purification the mixture was made alkaline with 2 M NaOH (5 mL) and extracted with $CH_2Cl_2$ (3 x 10 mL). After drying the organic phase over $Na_2SO_4$ the mixture was filtered and the solvent removed under vacuum. The crude product was purified using flash-chromatography (3 cm, cHex:EA 9:1, 30 mL, $R_f$ = 0.41). Colourless Liquid, Yield 752 mg (92 %).

**HPLC:** 99.6 %, $t_R$ = 19.6 min

$C_{11}H_{17}BrO_4S$ (325.2)

**MS (EI):** m/z = 326/324 [$M^+$], 295/293 [$M^+$ -$OCH_3$].
**IR (Film):** ν ($cm^{-1}$) = 2932 (C-H), 2830 (C-H), 1049 (C-O).
**$^1$H-NMR ($CDCl_3$):** δ (ppm) = 3.07 (d, J = 5.6 Hz, $ThCH_2CH(OCH_3)_2$), 3.35 (s, 6 H, $ThCH_2$CH(OC*$H_3$*$)_2$), 3.38 (s, 6 H, ThCH(OC*$H_3$*$)_2$), 4.54 (t, J = 5.6Hz, 1 H, $ThCH_2$C*H*), 5.53 (s, 1 H, ThC*H*($OCH_3)_2$), 6.90 (s, 1 H, 3-*H*-Th).

**Example M: 2-(3-Bromo-5-chlorothiophene-2-yl)-acetaldehydedimethylacetal**

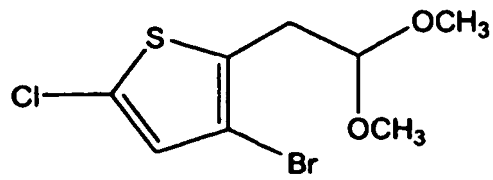

**Example C** (300 mg, 1.2 mmol) was dissolved in a 1:1 mixture of $CH_2Cl_2$ and $CH_3OH$ (8 mL) and N-Chlorsuccinimid (240 mg, 1.8 mmol) added. Then it was stirred under $N_2$ for 3 h at ambient temperature. After the end of the reaction a saturated solution of NaCl (10 mL) was added and repeatedly extracted with $CH_2Cl_2$. The pooled organic phases were dried over $Na_2SO_4$, filtered and the solvent removed under vacuum. The crude product was purified using flash-chromatography (3 cm, cHex:EA 9:1, 10 mL, $R_f$ = 0.61). Colourless Liquid, Yield 192 mg (60 %).

**HPLC:** 97.2 %, $t_R$ = 23.2 min

$C_8H_{10}BrClO_2S$ (285.6)

**MS (EI):** m/z = 257/255/253 [$M^+$ -$OCH_3$], 176/174 [$M^+$ -$OCH_3$, -Br].
**IR (Film):** ν ($cm^{-1}$) = 3096 (C-H), 2930 (C-H), 1069 (C-O).
**$^1$H-NMR ($CDCl_3$):** δ (ppm) = 3.03 (d, J = 5.2 Hz, 2 H, ThC*H*$_2$CH), 3.40 (s, 6 H, CH(OC*H*$_3$)$_2$), 4.49 (t, J = 5.2 Hz, 1 H, ThCH$_2$C*H*), 6.76 (s, 1 H, 4-*H*-Th).

**Example N: 2-[3-Bromo-5-(α-hydroxybenzyl)thiophene-2-yl]acetaldehyde-dimethylacetal**

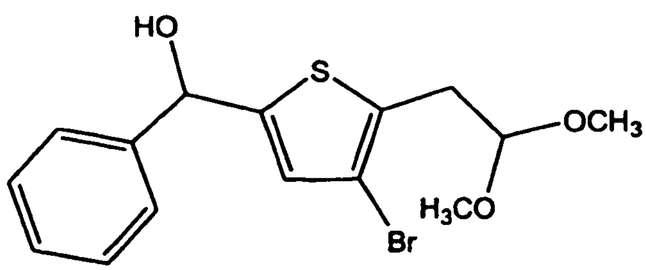

**Example C** (146 mg, 0.58 mmol) was dissolved under $N_2$ in THF (5 mL) and cooled to -40 °C. A solution of LDA (2 M in THF, 520 μL, 1.04 mmol) was slowly added while stirring and the mixture was stirred for a further 10 min. Following that benzaldehyde (110 mg, 1.04 mmol), dissolved in THF (0.5 mL) was added. After 30 min the mixture was heated to ambient temperature, a saturated solution of NaCl (10 mL) added and extracted with $CH_2Cl_2$ (3 x 5 mL). The organic phase was dried over Na2SO4, filtered and the solvent removed under vacuum. The crude product was purified using flash-chromatography (2 cm, cHex:EA 8:2, 10 mL, $R_f$ = 0.32). Colourless Liquid, Yield 259 mg (93 %).

**HPLC:** 99.3 %, $t_R$ = 21.5 min

$C_{15}H_{17}BrO_3S$ (357.3)

**MS (EI):** m/z = 358/356 [$M^+$], 327/325 [$M^+$ -$OCH_3$], 77 [$Ph^+$].
**IR (Film):** ν ($cm^{-1}$) = 3413 (O-H), 2926 (C-H), 1040 (C-O), 698 (C-H).
**$^1$H-NMR ($CDCl_3$):** δ (ppm) = 2.40 (d, J = 4.0 Hz, 1 H, OH), 3.03 (dd, J = 15.0/5.5 Hz, 1 H, ThC*H*$_2$CH), 3.07 (dd, J = 15.0/5.5 Hz, 1 H, ThC*H*$_2$CH), 3.37 (s, 6 H, CH(OC*H*$_3$)$_2$), 4.53 (t, J = 5.5 Hz, 1 H, ThCH$_2$C*H*), 5.93 (d, J = 4.0 Hz, 1 H, C*H*OH), 6.67 (s, 1 H, 4-*H*-Th), 7.28 - 7.43 (m, 5 H, Ph-*H*).

**Example O: 2-(5-Benzyl-3-bromothiophene-2-yl)acetaldehyde-dimethylacetal**

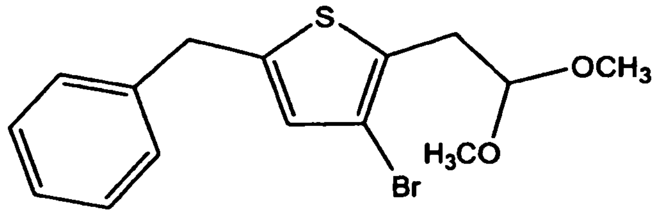

**Example N** (60 mg, 0.17 mmol) was dissolved in $CH_2Cl_2$ (2 mL) and n-butylsilane (17.8 mg, 0.2 mmol) followed by tris(pentafluorphenyl)borane (5.1 mg, 10 μmol) were aded. After 8 h of stirring at ambient temperature 2 M NaOH (5 mL) were added and extracted with $CH_2Cl_2$ (3 x 5 mL).The organic phase was dried over $Na_2SO_4$, filtered and the solvent removed under vacuum. The crude product was purified using flash-chromatography (2 cm, cHex:EA 9:1, 5 mL, $R_f$ = 0.65). Colourless Oil, Yield 33.3 mg (58 %).

**HPLC:** 96.3 %, $t_R$ = 24.6 min

**$C_{15}H_{17}BrO_2S$** (341.3)

**MS (EI):** m/z = 342/340 [$M^+$], 311/309 [$M^+$ -$OCH_3$], 267/265 [$M^+$ -$CH(OCH_3)_2$].
**IR (Film):** ν ($cm^{-1}$) = 3027 (C-H), 2930 (C-H), 1058 (C-O), 697 (C-H).
**$^1$H-NMR ($CDCl_3$):** δ (ppm) = 3.03 (d, J = 5.6 Hz, 2 H, ThC*H*$_2$CH), 3.37 (s, 6 H, CH(OC*H*$_3$)$_2$), 4.04 (s, 2 H, ThC*H*$_2$Ph), 4.52 (t, J = 5.6 Hz, 1 H, ThCH$_2$C*H*), 6.59 (s, 1 H, 4-*H*-Th), 7.21 - 7.27 (m, 3 H, Ph-*H*), 7.29 - 7.34 (m, 2 H, Ph-*H*).

**<u>Example P: 4-(1-Benzyl-4-hydroxypiperidine-4-yl)-5-(2,2-dimethoxyethyl)thiophenecarb-aldehyde-dimethylacetal</u>**

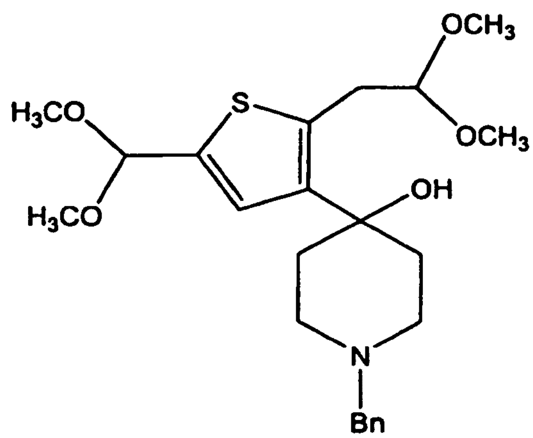

**Erxample L** (740 mg, 2.27 mmol) was dissolved in THF (20 mL) and under $N_2$ cooled to -84 °C. A solution of n-butyllithium (1.5 M in hexane, 2.2 mL, 3.18 mmol) was added within 2-3 min while stirring, until - 15 min later - 1-Benzylpiperidine-4-one (430 mg, 2.27 mmol) was added. After 1 h of stirring at -90 °C the mixture was stirred a further 1 h at ambient temperature. The reaction was stopped by adding a saturated solution of NaCl (20 mL). Following that it was extracted with $CH_2Cl_2$ (3 x 5 mL). The pooled organic phases were dried over $Na_2SO_4$, filtered and the solvent removed under vacuum. The crude product was purified using flash-chromatography (4 cm, cHex:EA 1:1, 30 mL, $R_f$ = 0.14). Colourless oil, the yield was determined after cyclisation from EX P to **Example 37.**

**MS (EI):** m/z = 435 [$M^+$], 404 [$M^+$ -$OCH_3$], 344 [$M^+$ -$CH_2Ph$].

$C_{23}H_{33}NO_5S$ (435.6)

**IR (Film):** ν ($cm^{-1}$) = 3455 (O-H), 2935 (C-H). 1050 (C-O), 698 (C-H).

**$^1$H-NMR ($CDCl_3$):** δ (ppm) = 1.68 - 1.74 (m, 2 H, N($CH_2CH_2$)$_2$), 2.08 (td, J = 13-2/4.0 Hz, 2 H, N($CH_2CH_2$)$_2$), 2.46 (td, J = 12.4/2.4 Hz, 2 H, N($CH_2CH_2$)$_2$), 2.68 - 2.74 (m, 2 H, N($CH_2CH_2$)$_2$), 3.35 (s, 12 H, 2 x CH ($OCH_3$)$_2$), 3.40 (d, J = 5.6 Hz, 2 H, Th$CH_2$CH), 3.55 (s, 2 H, N$CH_2$Ph), 3.80 (s, 1 H, O*H*), 4.49 (t, J = 5.6 Hz, 1 H, Th$CH_2$C*H*), 5.48 (s, 1 H, ThC*H*), 6.88 (s, 1 H, 3-*H*-Th), 7.20 - 7.37 (m, 5 H, Ph-*H*).

**Example Q: 2-[3-(1-Benzyl-4-hydroxypiperidine-4-yl)-5-chlorothiophene-2-yl]acetaldehyde-dimethylacetal**

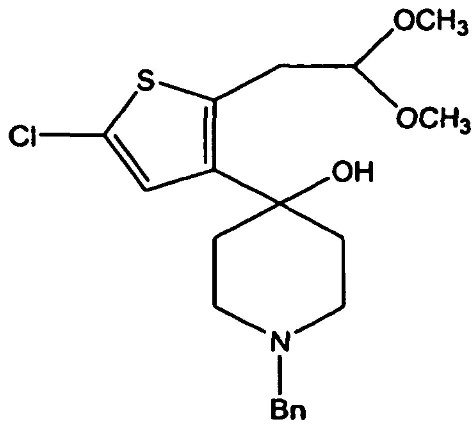

**Example M** (180 mg, 0.63 mmol) was dissolved in THF (15 mL) and cooled under $N_2$ to -78 ˚C. A solution of n-butyllithium (1.5 M in hexane, 630 µL, 0.94 mmol) was added within 2-3 min while stirring, until - 15 min later - 1-Benzylpiperidine-4-one (120 mg, 0.63 mmol) was added. After 1 h of stirring at -78 ˚C the mixture was stirred a further 1 h at ambient temperature. The reaction was stopped by adding $H_2O$ (20 mL). After extraction with $CH_2Cl_2$ (3 x 5 mL) the pooled organic phases were dried over $Na_2SO_4$, filtered and the solvent removed under vacuum. The crude product was purified using flash-chromatography (3 cm, cHex:EA 7:3, 10 mL, $R_f$ = 0.28). Colourless Oil, the yield was determined after cyclisation from **EX Q** to **Example 38.**

$C_{20}H_{26}ClO_3S$ (395.9)

**MS (EI):** m/z = 397/395 [$M^+$], 366/364 [$M^+$ -$OCH_3$], 306/304 [$M^+$ -$CH_2$Ph].

**IR (Film):** ν ($cm^{-1}$) = 3444 (O-H), 3061 (C-H), 2921 (C-H), 1042 (C-O), 697 (C-H).

**$^1$H-NMR ($CDCl_3$):** δ (ppm) = 1.70 (dd, J = 14.4/2.0 Hz, 2 H, N($CH_2CH_2$)$_2$), 2.02 (td, J = 12.8/4.4 Hz, 2 H, N($CH_2CH_2$)$_2$), 2.43 (td, J = 11.6/2.4 Hz, 2 H, N($CH_2CH_2$)$_2$), 2.67 - 2.74 (m, 2 H, N($CH_2CH_2$)$_2$), 3.35 (d, J = 5.2 Hz, 2 H, Th$CH_2$CH), 3.37 (s, 6 H, CH($OCH_3$)$_2$), 3.55 (s, 2 H, N$CH_2$Ph), 3.69 (s, 1 H, O*H*), 4.46 (t, J = 5.2 Hz, 1 H, Th$CH_2$C*H*), 6.72 (s, 1 H, 4-*H*-Th), 7.19 - 7.36 (m, 5 H, Ph-*H*).

**Example R: 2-[5-Benzyl-3-(1-benzyl-4-hydroxypiperidine-4-yl)-5-benzylthiophene-2-yl]acetaldehyde-dimethyl-acetal**

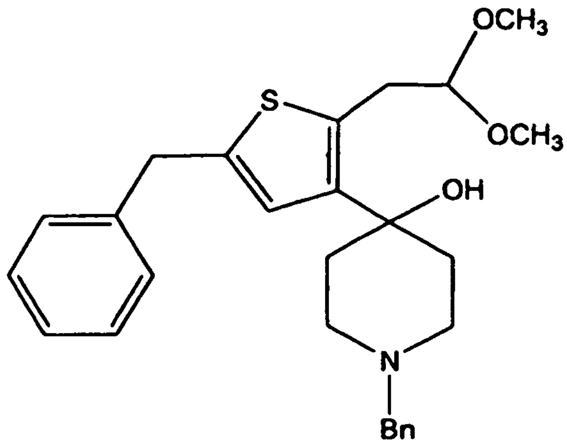

**Example O** (250 mg, 0.74 mmol) was dissolved in THF (15 mL) and cooled under $N_2$ to -78 °C. A solution of n-butyllithium (1.5 M in hexane, 0.7 mL, 1.04 mmol) was added within 2-3 min while stirring, until - 15 min later - 1-Benzylpiperidine-4-one (140 mg, 0.74 mmol) was added. After 2 h of stirring at -78 °C the mixture was stirred a further 1 h at ambient temperature. The reaction was stopped by adding a saturated solution of NaCl (20 mL). After extraction with $CH_2Cl_2$ (3 x 5 mL) the pooled organic phases were dried over $Na_2SO_4$, filtered and the solvent removed under vacuum. The crude product (approx. 3.31 g) was purified using flash-chromatography (3 cm, cHex:EA 7:3, 30 mL, $R_f$ = 0.2). Colourless oil, the yield was determined after cyclisation from **EX R** to **Example 39.**

**MS (EI):** m/z = 451 [$M^+$], 420 [$M^+$ -$OCH_3$], 360 [$M^+$ -$CH_2Ph$].

$C_{27}H_{33}NO_3S$ (451.6)

**IR (Film):** ν ($cm^{-1}$) = 3451 (O-H), 3026 (C-H), 2923 (C-H), 1039 (C-O), 697 (C-H).

**$^1$H-NMR ($CDCl_3$):** δ (ppm) = 1.48 - 1.55 (m, 2 H, N($CH_2CH_2$)$_2$), 2.03 (td, J = 13.2/4.4 Hz, 2 H, N($CH_2CH_2$)$_2$), 2.41 - 2.50 (m, 2 H, N($CH_2CH_2$)$_2$), 2.68 - 2.76 (m, 2 H, N($CH_2CH_2$)$_2$), 3.35 (d, J = 5.2 Hz, 2 H, Th$CH_2$CH), 3.36 (s, 6 H, CH($OCH_3$)$_2$), 3.55 (s, 2 H, N$CH_2$Ph), 3.82 (s, 1 H, O*H*), 4.01 (s, 2H, Th$CH_2$Ph), 4.47 (t, J = 5.2 Hz, 1 H, Th$CH_2$C*H*), 6.58 (s, 1 H, 4-*H*-Th), 7.18 - 7.38 (m, 10 H, Ph-*H*).

**Example 37: (1-Benzyl-6'-methoxy-6',7'-dihydrospiro[piperidine-4,4'-thieno[3,2-c]pyrano]-2-carbaldehyde-dimethylacetal**

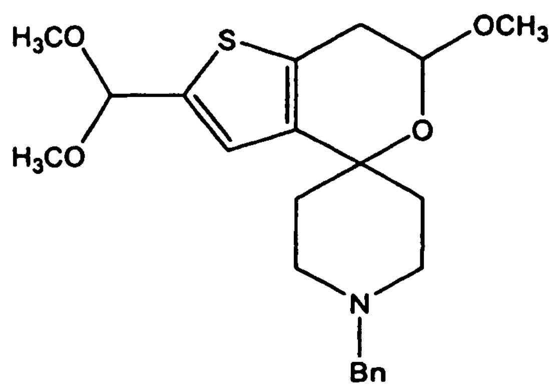

**Example P** (ca. 460 mg, non-purified) was dissolved in MeOH (15 mL) and trimethylorthoformiate (2 mL) added. After adding p-toluene-sulfonic acid (260 mg, 1.37 mmol) the mixture was stirred under $N_2$ for 45 min at ambient temperature. For purification the mixture was made alkaline with 2 M NaOH and extracted with $CH_2Cl_2$ (3 x 5 mL). The organic phases were pooled and dried over $Na_2SO_4$, filtered and the solvent removed under vacuum. The crude product was purified using flash-chromatography (3 cm, cHex:EA 7:3, 15 mL, $R_f$ = 0.23 (cHex:EA 1:1)). Colourless solid, MP

71 °C, yield 287 mg (31.5 % with 2 steps).

**HPLC:** 96.4 %, $t_R$ = 16.9 min

$C_{22}H_{29}NO_4S$ (403.5)

**MS (EI):** m/z = 403 [$M^+$], 372 [$M^+$ -$OCH_3$], 328 [$M^+$ -$CH(OCH_3)_2$], 312 [$M^+$ -$CH_2Ph$].
**IR (Film):** ν ($cm^{-1}$) = 2924 (C-H), 2828 (C-H), 1049 (C-O), 698 (C-H).
**$^1$H-NMR ($CDCl_3$):** δ (ppm) = 1.81 (dd, J = 13.6/2.8 Hz, 1 H, $N(CH_2CH_2)_2$), 1.85 (td, J = 12.0/4.0 Hz, 1 H, $N(CH_2CH_2)_2$), 1.94 (dd, J = 13.6/2.8 Hz, 1 H, $N(CH_2CH_2)_2$), 2.08 (td, J = 13.6/4.8 Hz, 1 H, $N(CH_2CH_2)_2$), 2.42 (td, J = 12.0/3.2 Hz, 1 H, $N(CH_2CH_2)_2$), 2.51 (td, J = 12.0/2.8 Hz, 1 H, $N(CH_2CH_2)_2$), 2.72 - 2.79 (m, 2 H, $N(CH_2CH_2)_2$), 2.74 (dd, J = 15.6/7.2 Hz, 1 H, $ThCH_2CH$), 2.94 (dd, J = 15.6/3.6 Hz, 1 H, $ThCH_2CH$), 3.35 (s, 6 H, $CH(OCH_3)_2$), 3.55 (s, 3 H, $ThCH_2CHOCH_3$), 3.57 (d, J = 16.0 Hz, 1 H, $NCH_2Ph$), 3.59 (d, J = 16.0 Hz, 1 H, $NCH_2Ph$), 4.86 (dd, J = 7.2/3.6 Hz, 1 H, $ThCH_2CH$), 5.51 (s, 1 H, ThC*H*), 6.79 (s, 1 H, 3'-*H*-Th), 7.19 - 7.32 (m, 5 H, Ph-*H*).

**Example 38: 1-Benzyl-2'-chloro-6'-methoxy-6',7'-dihydrospiro[piperidine-4,4'-thieno[3.2-c]-pyranol]**

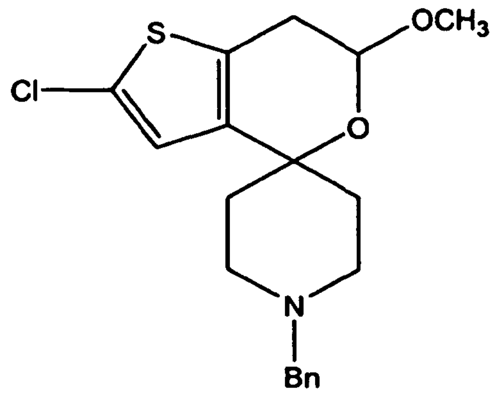

**Example Q** (ca. 120 mg, non-purified) was dissolved in MeOH (6 mL) and trimethylorthoformiate (0.5 mL) added. After adding p-toluene-sulfonic acid (120 mg, 0.63 mmol) the mixture was stirred under $N_2$ for 3 h at ambient temperature. For purification the mixture was made alkaline with 2 M NaOH and $H_2O$ (10 mL) added. Following that it was extracted with $CH_2Cl_2$ (3 x 5 mL). The organic phases were pooled and dried over $Na_2SO_4$, filtered and the solvent removed under vacuum. The crude product was purified using flash-chromatography (2 cm, cHex:EA 7:3, 10 mL.

$R_f$ = 0.36). Colourless Solid, MP 117 °C, Yield 77 mg (33 % in 2 steps).

**HPLC:** 98.0 %, $t_R$ = 19.8 min

$C_{19}H_{22}ClNO_2S$ (363.6)

**MS (EI):** m/z = 365/363 [$M^+$]. 334/332 [$M^+$ -$OCH_3$], 272/274 [$M^+$ -$CH_2Ph$].
**IR (Film):** ν ($cm^{-1}$) = 3069 (C-H), 2917 (C-H), 1059 (C-O), 700 (C-H).
**$^1$H-NMR ($CDCl_3$):** δ (ppm) = 1.81 (dd, J = 13.2/2.8 Hz, 1 H, $N(CH_2CH_2)_2$), 1.83 (td, J = 13.6/4.4 Hz, 1 H, $N(CH_2CH_2)_2$), 1.93 (dd, J = 14.0/2.8 Hz, 1 H, $N(CH_2CH_2)_2$), 2.01 (td, J = 13.6/4.4 Hz, 1 H, $N(CH_2CH_2)_2$), 2.41 (td, J = 11.6/2.8 Hz, 1 H, $N(CH_2CH_2)_2$), 2.50 (td, J = 11.6/2.8 Hz, 1 H, $N(CH_2CH_2)_2$), 2.72 - 2.79 (m, 2 H, $N(CH_2CH_2)_2$), 2.74 (dd, J = 15.6/7.2 Hz, 1 H, $ThCH_2CH$), 2.86 (dd, J = 15.6/3.2 Hz, 1 H, $ThCH_2CH$), 3.54 (s, 3 H, $OCH_3$), 3.54 (d, J = 12.8 Hz, 1 H, $NCH_2Ph$), 3.58 (d, J = 12.8 Hz, 1 H, $NCH_2Ph$), 4.86 (dd, J = 7.2/3.2 Hz, 1 H, $ThCH_2CH$), 6.62 (s, 1 H, 3'-*H*-Th), 7.20 - 7.35 (m, 5 H, Ph-*H*).

**Example 39: 1,2'-Dibenzyl-6'methoxy-6',7'dihydrospiro[piperidine-4,4'-thieno[3.2-c]pyrano]**

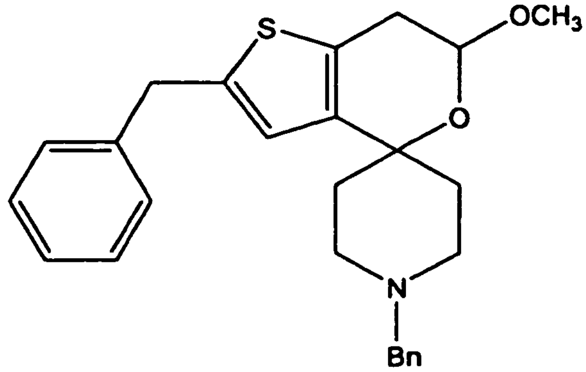

**Example R** (ca. 185 mg, non-purified) was dissolved in MeOH (6 mL) and trimethylorthoformiate (3 mL) added. After adding p-toluene-sulfonic acid (120 mg, 0.61 mmol) the mixture was stirred under $N_2$ for 2.5 h at ambient temperature. For purification the mixture was made alkaline with 2 M NaOH and $H_2O$ (10 mL) was added. Following that it was extracted with $CH_2Cl_2$ (3 x 5 mL). The organic phases were dried over $Na_2SO_4$, filtered and the solvent removed under vacuum. The crude product was purified using flash-chromatography (2 cm, cHex:EA 7:3, 15 mL, $R_f$ = 0.22). Colourless, oily substance, yield 82 mg (26 % in 2 steps).

**HPLC:** 98.3 %, $t_R$ = 21.8 min

$C_{26}H_{29}NO_2S$ (419.6)

**MS (EI):** m/z = 419 [$M^+$], 388 [$M^+$ -$OCH_3$], 328 [$M^+$ -$CH_2Ph$].

**IR (Film):** v ($cm^{-1}$) = 3027 (C-H), 2922 (C-H), 1060 (C-O), 696 (C-H).

**$^1$H-NMR ($CDCl_3$):** δ (ppm) = 1.79 (dd, J = 13.6/2.8 Hz, 1 H, $N(CH_2CH_2)_2$), 1.82 (td, J = 14.0/4.4 Hz, 1 H, $N(CH_2CH_2)_2$), 1.93 (dd, J = 14.0/2.8 Hz, 1 H, $N(CH_2CH_2)_2$), 2.03 (td, J = 13.6/4.4 Hz, 1 H, $N(CH_2CH_2)_2$), 2.40 (td, J = 11.6/2.8 Hz, 1 H, $N(CH_2CH_2)_2$), 2.49 (td, J = 11.6/2.4 Hz, 1 H, $N(CH_2CH_2)_2$), 2.70 - 2.78 (m, 2 H, $N(CH_2CH_2)_2$), 2.75 (dd, J = 15.6/7.2 Hz, 1 H, $ThCH_2CH$), 2.86 (dd, J = 15.6/3.2 Hz, 1 H, $ThCH_2CH$), 3.54 (d, J = 16.4 Hz, 1 H, $NCH_2Ph$), 3.54 (s, 3 H, $OCH_3$), 3.56 (d, J = 16.4Hz, 1 H, $NCH_2Ph$), 4.04 (s, 2 H, $ThCH_2Ph$), 4.83 (dd, J = 7.2/3.2 Hz, 1 H, $ThCH_2CH$), 6.45 (s, 1 H, 3'-*H*-Th), 7.19 - 7.35 (m, 10 H, Ph-H).

**Example 40: (1-Benzyl-6'-methoxy-6',7'-dihydrospiro[piperidine-4,4'-thieno[3.2-c]pyrano]-2-carbaldehyde**

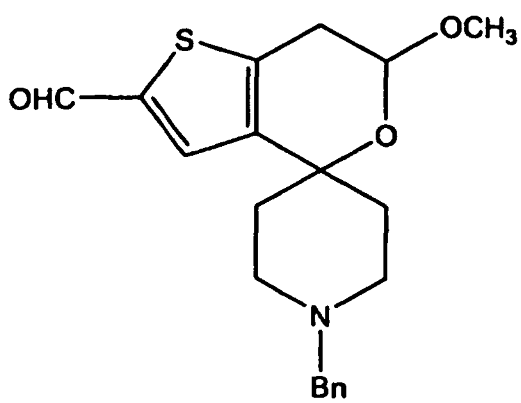

**Example 37** (287 mg, 0.71 mmol) was dissolved in $CH_3CN$ (15 mL) and HCl (1 M, 1 mL) added. After 15 min the mixture was made alkaline with 2 M NaOH and the aqueous phase repeatedly extracted with $CH_2Cl_2$. Following that the pooled organic phases were dried over Na2SO4, filtered and the solvent removed under vacuum. The purification of the crude product was done using flash-chromatography (2 cm, cHex:EA 1:1, 15 mL, $R_f$ = 0.14). Colourless viscous oil, 244 mg (96 %).

**HPLC:** 98.0 %, $t_R$ = 17.1 min

$C_{20}H_{23}NO_3S$ (357.5)

**MS (EI):** m/z = 357 [$M^+$], 326 [$M^+$ -$OCH_3$], 265 [$M^+$ -$CH_2Ph$].
**IR (Film):** ν ($cm^{-1}$) = 3027 (C-H), 2910 (C-H), 1665 (C=O), 1059 (C-O), 698 (C-H).
**$^1$H-NMR ($CDCl_3$):** δ (ppm) = 1.84 - 2.14 (m, 3H, N($CH_2CH_2$)$_2$), 2.01 (td, J = 13.6/4.8 Hz, 1 H, N($CH_2CH_2$)$_2$), 2.43 (td, J = 11.6/3.6 Hz, 1 H, N($CH_2CH_2$)$_2$), 2.53 (td, J = 12.0/2.8 Hz, 1 H, N($CH_2CH_2$)$_2$), 2.65 - 2.73 (m, 2 H, N($CH_2CH_2$)$_2$), 2.91 (dd, J = 16.4/6.4 Hz, 1 H, Th$CH_2$CH, 3.06 (dd, J = 16.4/3.2 Hz, 1 H, Th$CH_2$CH), 3.55 (s, 3 H, $OCH_3$), 3.58 (s, 2 H, N$CH_2$Ph), 4.92 (dd, J = 6.4/3.2 Hz, 1 H, Th$CH_2$C*H*), 7.18 - 7.37 (m, 5 H, Ph-*H*), 7.49 (s, 1 H, 3'-*H*-Th), 9.80 (s, 1 H, ThC*H*O).

**Example 41: 1-Benzyl-6'-methoxy-6',7'dihydrospiro[piperidine-4,4'-thieno[3.2-c]pyrano]-2'yl)-methanol**

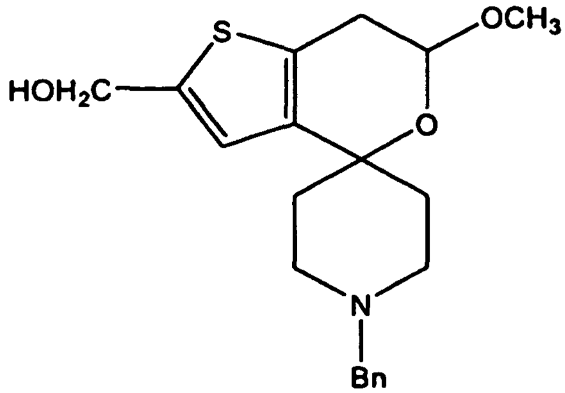

The aldehyde **40** (60 mg, 0.16 mmol) was dissolved under $N_2$ in THF (3 mL) and cooled to -40 ˚C. A solution of $LiAlH_4$ (1 M in THF, 84 μL, 0.084 mmol) was slowly added and the mixture stirred for 30 min. After adding a saturated solution of NaCl (10 mL) it was extracted repeatedly with $CH_2Cl_2$. The pooled organic phases were dried over Na2SO4, filtered and the solvent removed under vacuum. The crude product was purified using flash-chromatography (1 cm, cHex:EA 1:9, 10 mL, $R_f$ = 0.18). Colourless viscous oil, yield 30 mg (57 %).

**HPLC:** 98.0 %, $t_R$ = 16.0 min

$C_{20}H_{25}NO_3S$ (359.5)

**MS (EI):** m/z = 359 [$M^+$], 328 [$M^+$ -$OCH_3$] 268 [$M^+$ -$CH_2Ph$].
**IR (Film):** ν ($cm^{-1}$) = 3411 (O-H), 3024 (C-H), 2923 (C-H), 1027 (C-O), 698 (C-H).
**$^1$H-NMR ($CDCl_3$):** δ (ppm) = 1.80 (dd, J = 13.6/2.4 Hz, 1 H, N($CH_2CH_2$)$_2$), 1.84 - 1.95 (m, 2 H, N($CH_2CH_2$)$_2$), 1.99 (s, 1 H, OH), 2.07 (td, J = 13.4/4.4 Hz, 1 H, N($CH_2CH_2$)$_2$), 2.43 (td, J = 11.6/3.6 Hz, 1 H, N ($CH_2CH_2$)$_2$), 2.52 (td, J = 11.6/2.4 Hz,1 H, N($CH_2CH_2$)$_2$), 2.74 - 2.79 (m, 2 H, N($CH_2CH_2$)$_2$), 2.81 (dd, J = 16.0/7.2 Hz, 1 H, Th$CH_2$CH), 2.94 (dd, J = 16.0/3.6 Hz, 1 H, Th$CH_2$CH), 3.54 (s, 3 H, $OCH_3$), 3.55 (d,J = 13.2 Hz, 1 H, N$CH_2$Ph), 3.59 (d, J = 13.2 Hz, 1 H, N$CH_2$Ph), 4.72 (s, 2 H, Th$CH_2$OH), 4.86 (dd, J = 7.2/3.6 Hz, 1 H, Th$CH_2$C*H*), 6.71 (s, 1 H, 3'-*H*-Th), 7.18 - 7.36 (m, 5 H, Ph-*H*).

**Example 42; (1-Benzyl-6'-methoxy-6',7'-dihydrospiro[piperidine-4,4'-thieno[3.2-c]pyrano]-2'-yl)-N,N-dimethyl-methanamine**

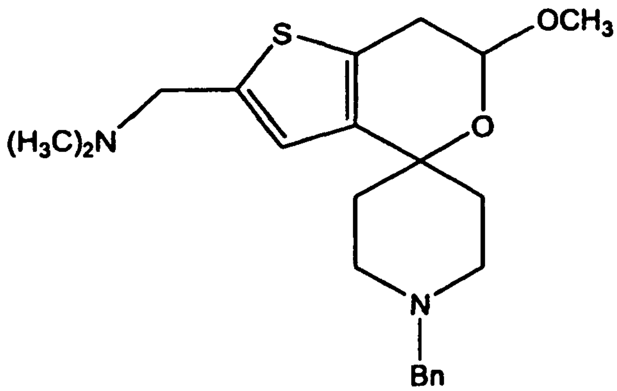

Example 40 (51 mg, 0.14 mmol) was dissolved in $CH_2Cl_2$ (4 mL), a solution of dimethylamine (2 M in THF, 280 μL, 0.56 mmol) and $NaBH(OAc)_3$ (55 mg, 0.27 mmol) added and stirred for 1.5 h at ambient temperature. After the reaction finished a saturated solution of NaCl (10 mL) was added and extracted with $CH_2Cl_2$ (3 x 5 mL). The organic phases were dried over Na2SO4, filtered and the solvent removed under vacuum. The crude product was purified using flash-chromatography (1 cm, cHex:EA:MeOH 2:6.5:1.5. 5 mL, $R_f$ = 0.10). Colourless oil, yield 41 mg (77 %).

**HPLC:** 98.3 %, $t_R$ = 13.9 min

$C_{22}H_{30}N_2O_2S$ (386.6)

**MS (EI):** m/z = 386 [$M^+$], 355 [$M^+$ -$OCH_3$], 342 [$M^+$ -$N(CH_3)_2$].

**IR (Film):** ν ($cm^{-1}$) = 2921 (C-H), 2813 (C-H), 1061 (C-O), 697 (C-H).

**$^1$H-NMR ($CDCl_3$):** δ (ppm) = 1.80 (dd, J = 13.6/2.8 Hz, 1 H, $N(CH_2CH_2)_2$), 1.89 (td, J = 12.4/4.4 Hz, 1 H, $N(CH_2CH_2)_2$), 1.94 (dd, J = 14.0/2.8 Hz, 1 H, $N(CH_2CH_2)_2$), 2.08 (td, J = 14.0/4.4 Hz, 1 H, $N(CH_2CH_2)_2$), 2.26 (s, 6 H, $N(CH_3)_2$). 2.44 (td, J = 11.6/3.2 Hz, 1 H, $N(CH_2CH_2)_2$), 2.54 (td, J = 11.6/2.4 Hz, 1 H, N $(CH_2CH_2)_2$), 3.74 - 3.81 (m,2 H, $N(CH_2CH_2)_2$), 3.79 (dd, J = 15.6/7.6 Hz, 1 H, $ThCH_2CH$), 2.92 (dd, J = 15.6/3.6 Hz, 1 H, $ThCH_2CH$), 3.52 (d, J = 13.6 Hz, 1 H, $ThCH_2N(CH_3)_2$), 3.54 (s, 3 H, $OCH_3$), 3.55 (d, J = 13.6 Hz, 1 H, $ThCH_2N(CH_3)_2$), 3.57 (d, J = 13.2 Hz, 1 H, $NCH_2Ph$), 3.60 (d, J = 13.2 Hz, 1 H, $NCH_2Ph$), 4.86 (dd, J = 7.6/3.6 Hz, 1 H, $ThCH_2CH$), 6.59 (s, 1 H, 3'-*H*-Th), 7.20 - 7.37 (m, 5 H, Ph-*H*).

**Example 43: (E/Z)-1-Benzyl-6'-methoxy-6',7'-dihydrospiro[piperidine-4,4'-thieno[3,2-c]pyranol]-2-carbaldehydoxim**

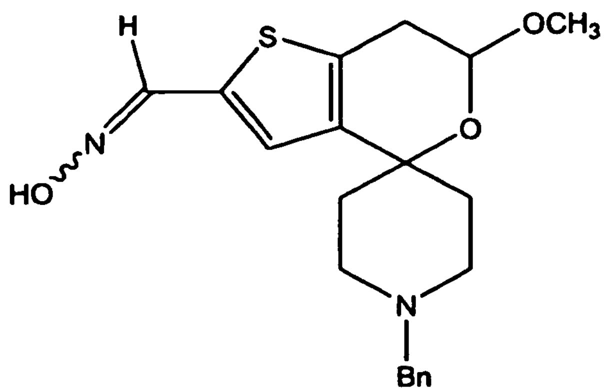

**Example 40** (43 mg, 0.12 mmol) was dissolved in pyridine (2 mL) and Hydroxylamine-hydrochloride (20 mg, 0.3 mmol) added. After stirring for 30 min at 60 °C the pyridine was removed under vacuum and the oxime precipitated as **colourless** solid. Superfluous hydroxylamine hydrochloride and impurities were removed by repeated washing with $CH_3OH$. Colourless solid, MP 233 °C, yield 31 mg (70 %).

**HPLC:** 99.7 %, $t_R$ = 17.0 min

$C_{20}H_{24}N_2O_2S$ (372.5)

**MS (EI):** m/z = 372 [$M^+$], 355 [$M^+$ -OH], 281 [$M^+$-$CH_2$Ph].

**IR (Film):** ν ($cm^{-1}$) = 3028 (C-H), 2961 (C-H), 1622 (C=N), 1053 (C-O).

**$^1$H-NMR ($DMSO_6$):** δ (ppm) = 1.60 - 1.68 (m, 1 H, N($CH_2CH_2)_2$), 1.67 - 1.80 (m, 1 H, N($CH_2CH_2)_2$), 1.88 - 2.07 (m, 2 H, N($CH_2CH_2)_2$), 2.34 - 2.45 (m, 2 H, N($CH_2CH_2)_2$). 2.66 (td, J = 16.4/6.8 Hz, 1 H, N($CH_2CH_2)_2$), 2.62 - 2.73 (m, 2 H, Th$CH_2$CH), 2.95 (ddd, J = 19.2/16.4/3.2 Hz, 1 H, N($CH_2CH_2)_2$), 3.42 (s, 3 H, $OCH_3$). 3.52 (s (breit),2 H, N$CH_2$Ph), 4.89 - 4.95 (m, 1 H, Th$CH_2$CH), 7.14 (s, 0.46 H, (E) 3'-*H*-Th), 7.30 (s, 0.54 H, (Z) 3'-*H*-Th), 7.32 - 7.40 (m, 5 *H*, Ph-H), 7.70 (s, 0.54 H, (Z) C*H*=NOH), 8.20 (s, 0.46 H, (E) C*H*=NOH), 11.12 (s, 0.46 H, (E) CH=NO*H*), 11.76 (s, 0.54 H, (Z) CH=NO*H*). The exact identification of the 3'-thiophene-protons and the protons of CH- and OH of the oxim was done with gHSQC- and gHMBC-spectra and analogously to similar oxims. The ratio (E):(Z) of 46:54 was calculated by integrating the signals of the (E)/(Z)-OH-protons.

**Example 44: 1-Benzyl-6'-methoxy-6',7'dihydrosniro[piperidine-4,4'thieno[3,2-c]pyranol-2- carbonitrile**

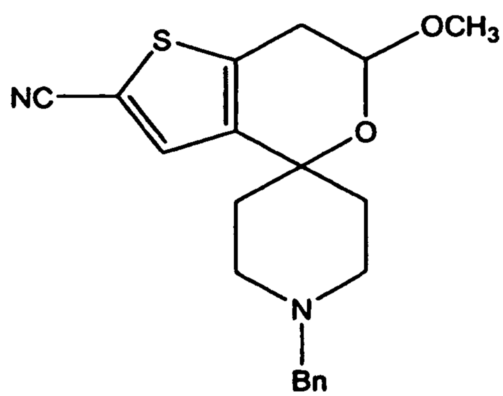

**Example 40** (150 mg, 0.42 mmol) was dissolved in pyridine (4 mL) and hydroxylamine hydrochloride (50 mg, 0.72 mmol) added. After heating for 30 min at 60 ˚C phthalic acid anhydride (240 mg, 1.6 mmol) was added and the mixture was heated for a further 60 min at 90 ˚C. For purification a saturated solution of NaCl was added and extracted with $CH_2Cl_2$ (3 x 5 mL). The pooled organic phases were dried over Na2SO4, filtered and the solvent removed under vacuum. The crude product was purified using flash-chromatography (3 cm, cHex:EA 4:6, 10 mL, $R_1$ = 0.37). Colourless solid, MP 154 ˚C, yield 68 mg (67 %).

**Analysis** of **elements:** calc.: C 67.77 H 6.26 N 7.73 found.: C 67.38 H 6.34 N 7.73

**HPLC:** 99.7 %, $t_R$ = 18.0 min

$C_{20}H_{22}N_2O_2S$ (354.4)

**MS (EI):** m/z = 354 [$M^+$]. 323 [$M^+$ -$OCH_3$], 232 [$M^+$ -$OCH_3$, -$CH_2$Ph].

**IR (Film):** ν ($cm^{-1}$) = 3061 (C-H), 2936 (C-H), 2211 (C≡N), 1058 (C-O), 697 (C-H).

**$^1$H-NMR ($CDCl_3$):** δ (ppm) = 1.80 - 1.88 (m, 2 H, N($CH_2CH_2)_2$). 1.92 (dd, J = 14.0/2.8 Hz, 1 H, N($CH_2CH_2)_2$), 2.03 (td, J = 12.8/4.8 Hz, 1 H, N($CH_2CH_2)_2$), 2.42 (td, J = 11.6/2.8 Hz, 1 H, N($CH_2CH_2)_2$), 2.51 (td, J = 11.8/2.8 Hz, 1 H, N($CH_2CH_2)_2$), 2.74 - 2.83 (m, 2 H, N($CH_2CH_2)_2$), 2.87 (dd, J = 16.4/6.4 Hz, 1 H, Th$CH_2$CH), 3.10 (dd, J = 16.4/3.6 Hz, 1 H, Th$CH_2$CH), 3.56 (s, 3 H, $OCH_3$), 3.57 (d, J = 13.2 Hz, 1 H, N$CH_2$Ph), 3.59 (d, J = 13.2 Hz, 1 H, N$CH_2$Ph), 4.92 (dd, J = 6.4/3.6 Hz, 1 H, Th$CH_2$C*H*), 7.33 (s, 1 H, 3'-H-Th), 7.20 - 7.38 (m, 5 H, Ph-*H*).

**$^{13}$C-NMR ($CDCl_3$):** δ (ppm) = 31.8 (1 C, Th$CH_2$CH), 36.3 (1 C, N($CH_2CH_2)_2$), 38.7 (1 C, N($CH_2CH_2)_2$), 49.1 (1 C, N

$(CH_2CH_2)_2$), 49.2 (1 C, N($CH_2CH_2)_2$), 56.8 (1 C, O$CH_3$), 63.5 (1 C, N$CH_2$Ph), 74.3 (1 C, Th*C*O), 96.4 (1 C, Th$CH_2$*C*H), 107.7 (1 C,*C*-2'-Th), 114.6 (1 C, *C*N), 127.3 (1 C, para-*C*-Ph), 128.5 (2 C, meta-*C*-Ph), 129.4(2 C, ortho-C-Ph-CH), 134.4 (1 C, *C*-3'-Th), 138.5 (1 C, Ph-*C*), 139.0 (1 C, *C*-7a'-Th), 141.7 (1 C, *C*-3a'-Th). The exact identification of the signals in the $^{13}$C-NMR-spectra was done with the help of gHSQC- and gHMBC-spectra.

**Example 45: Methyl-1-benzyl-6'-methoxy-6' 7'-dihydrospirolpioeridine-4,4'-thieno[3,2-c]pyranol -2-carboxylate**

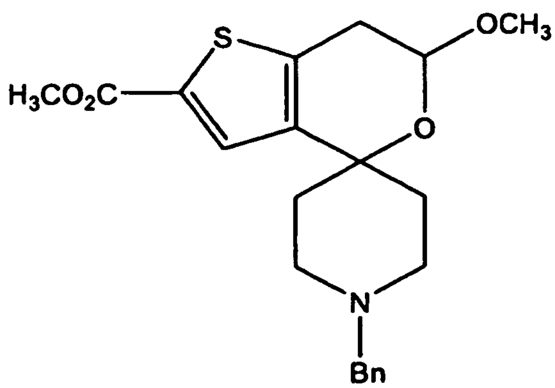

**Example 44** (26 mg, 0.073 mmol) was dissolved in $CH_3OH$, $H_2SO_4$ conc. (0.5 mL) and 3 drops of $H_2O$ added and heated for 22 h under reflux. After alkalinisation with NaOH (2 M, 7 mL) under cooling with ice the mixture was extracted with $CH_2Cl_2$ (3 x 5 mL).The organic phases were pooled dried over Na2SO4, filtered and the solvent removed under vacuum. The crude product was purified using flash-chromatography (1 cm, cHex:EA 7:3, 5 mL, R, = 0.17). Colourless solid, MP 136-138 ˚C, yield 17.1 mg (60 %).

**HPLC:** 98.6 %, $t_R$ = 18.1 min

$C_{21}H_{25}NO_4S$ (387.5)

**MS (EI):** m/z = 387 [$M^+$], 356 [$M^+$ -$OCH_3$], 296 [$M^+$ -$CH_2$Ph].

**IR (Film):** v ($cm^{-1}$) = 3023 (C-H), 2944 (C-H), 1701 (C=O), 1070 (C-O), 697 (C-H).

**$^1$H-NMR ($CDCl_3$):** δ (ppm) = 1.82 (dd, J = 13.2/2.8 Hz, 1 H, N($CH_2CH_2)_2$),1.86 - 1.95 (m, 2 H, N($CH_2CH_2)_2$), 2.08 (td, J = 13.2/4.4 Hz, 1 H, N($CH_2CH_2)_2$), 2.42 (td, J = 11.6/3.6 Hz, 1 H, N($CH_2CH_2)_2$), 2.51 (td, J = 13.2/2.8 Hz, 1 H, N($CH_2CH_2)_2$), 2.73 - 2.79 (m, 2 H, N($CH_2CH_2)_2$), 2.86 (dd, J = 16.4/7.2 Hz, 1 H, Th$CH_2$CH), 3.00 (dd, J = 16.0/3.2 Hz, 1 H, Th$CH_2$CH), 3.55 (s, 3 H, O$CH_3$), 3.55 (d, J = 13.2 Hz, 1 H, N$CH_2$Ph), 3.58 (d, J = 13.2 Hz, 1 H, N$CH_2$Ph), 3.85 (s, 3 H, $CO_2CH_3$), 4.90 (dd, J = 7.2/3.2 Hz, 1 H, Th$CH_2$C*H*), 7.21 - 7.36 (m, 5 H, Ph-*H*), 7.53 (s, 1 H, 3'-*H*-Th).

**Example 46: 1-(1-Benzyl-6'methoxy-6',7'dihydrospiro[piperidine-4,4'-thieno[3.2-c]pyranol]-2-yl)-1-phenyl-methanol**

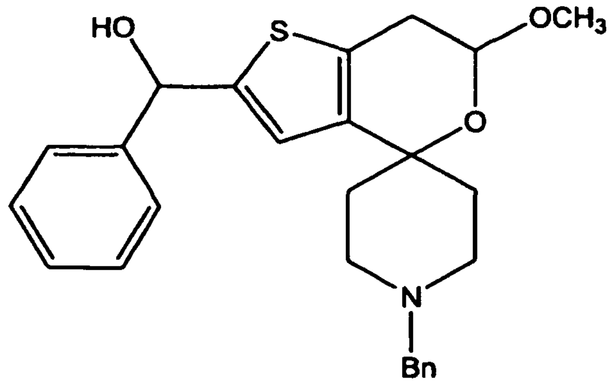

**Example 40** (125 mg, 0.35 mmol) was dissolved under $N_2$ in THF (15 mL) and cooled on ice to 0 °C. A solution of phenylmagnesiumbromide (1 M in THF, 700 μL, 0.7 mmol) was slowly added while stirring and the mixture stirred for a further 15 min. Following that a saturated solution of NaCl (10 mL) was added and extracted with $CH_2Cl_2$ (3 x 5 mL). The organic phase was dried over $Na_2SO_4$, filtered and the solvent removed under vacuum. The crude product was purified using flash-chromatography (2 cm, cHex:EA 1:1, 20 mL, $R_1$ = 0.15). Colourless solid, MP 78-80 °C, yield 150 mg (98 %).

**HPLC:** 99.4 %, $t_R$ = 19.3 min

$C_{26}$H29N0$_3$s (435.6)

**MS (EI):** m/z = 435 [M'], 313 [$M^*$ -$OCH_3$, -$CH_2$Ph], 91 i$^*$$CH_2$Ph].
**IR (Film):** v ($cm^{-1}$) = 3428 (O-H), 3083 (C-H), 1060 (C-O), 697 (C-H).
**$^1$H-NMR ($CDCl_3$):** 6 (ppm) = 1.75 - 1.85 (m, 2 H, N($CH_2$C$H_2$)$_2$), 1.86 - 1.93 (m, 1 H, N($CH_2$C$H_2$)$_2$), 2.00 (td, J = 13.2/4.4 Hz, 1 H, N($CH_2$C$H_2$)$_2$), 2.38 (td, J = 11.8/2.8 Hz,1 H, N(C$H_2$$CH_2$)$_2$), 2.39 (td, J = 11.8/2.8 Hz, 1 H, N(C$H_2$$CH_2$)$_2$), 2.43 - 2.52 (m, 2 H, N(C$H_2$$CH_2$)$_2$), 2.76 (dd, J = 15.6/7.2 Hz, 0.5 H, ThC$H_2$CH), 2.78 (dd, J = 15.6/7.2 Hz, 0.5 H, Th$CH_2$CH), 2.89 (dd, J = 15.6/3.2 Hz, 0.5 H, ThC$H_2$CH), 2.90 (dd, J = 15.6/3.2 Hz, 0.5 H, Th$CH_2$CH), 3.53 (s, 3 H, $OCH_3$) 3.54 (s, 2 H, NC$H_2$Ph), 4.82 - 4.86 (m, 1 H, Th$CH_2$C*H*), 5.93 (s, 1 H, ThC*H*Ph), 6.54 (s, 0.5 H, 3'-*H*-Th), 6.57 (s, 0.5 H, 3'-*H*-Th), 7.22 - 7.45 (m, 10 H, Ph-*H*). A signal for the proton of the OH-group can not be seen. The diastereomers are present in a ratio of 54:46.

**Example 47: 1-(1-Benzyl-6'-methoxy-6',7'-dihydrospiro[piperidine-4,4'-thieno[3.2-c]pyrano]-2-yl)-1-phenyl-methanone**

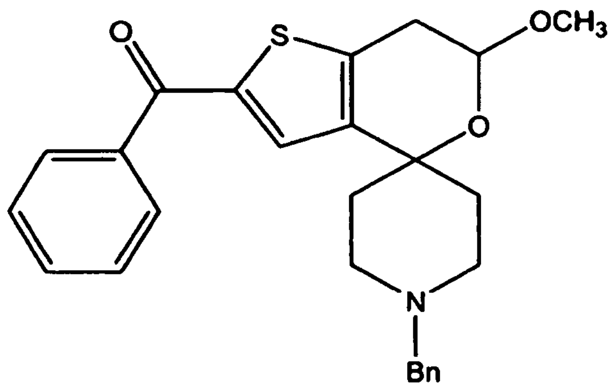

**Example 46** (50 mg, 0.11 mmol) was dissolved in $CH_2Cl_2$ (4 mL) and Molsieb 4 Å" (molecular sieve) (100 mg) added. After adding 1-methylmorpholine-N-oxide (24 mg, 0.2 mmol) and tetra-propylammoniumperruthenate (2.4 mg, 0.07 μmol) it was stirred for 20 min at ambient temperature. For purification a saturated solution of NaCl was added (10

mL) and extracted with $CH_2Cl_2$ (3 x 5 mL). The organic phase was dried over $Na_2SO_4$ and after filtration the solvent was removed under vacuum. The crude product was purified using flash-chromatography (2 cm, cHex:EA 7:3, 10 mL, $R_1$ = 0.2). Colourless solid, MP 91 °C, yield 36 mg (72 %).

**HPLC:** 98.0 %, ambient temperature = 20.2 min

$C_{16}H_{27}BrO_3S$ (433.6)

**MS (EI):** m/z = 433 [$M^+$], 402 [$M^+$ -$OCH_3$], 342 [$M^+$ -$CH_2$Ph].
**IR (Film):** ν ($cm^{-1}$) = 2921 (C-H), 1631 (C=O), 1054 (C-O), 697 (C-H).
**$^1$H-NMR ($CDCl_3$):** δ (ppm) = 1.81 - 1.91 (m, 2 H, N($CH_2C\mathit{H_2})_2$), 1.97 (dd, J = 14.4/2.8 Hz, 1 H, N($CH_2C\mathit{H_2})_2$), 2.04 (td, J = 13.6/4.4 Hz, 1 H, N($CH_2C\mathit{H_2})_2$), 2.43 (td, J = 12.4/2.8 Hz, 1 H, N($C\mathit{H_2}CH_2)_2$), 2.52 (td, J = 12.4/2.8 Hz, 1 H, N($CH_2CH_2)_2$), 2.75 - 2.82 (m, 2 H, N($C\mathit{H_2}CH_2)_2$), 2.92 (dd, J = 16.4/6.8 Hz, 1 H, ThC$\mathit{H_2}$CH), 3.07 (dd, J = 16.4/3.2 Hz, 1 H, ThC$\mathit{H_2}$CH), 3.54 (d, J = 14.0 Hz, 1 H, NC$\mathit{H_2}$Ph), 3.56 (s, 3 H, OC$\mathit{H_3}$), 3.58 (d, J = 14.0 Hz, 1 H, N$CH_2$Ph), 4.93 (dd, J = 6.8/3.2Hz, 1 H, ThC$\mathit{H_2}$CH), 7.19 - 7.33 (m, 5 H, $CH_2$Ph-*H*), 7.33 (s, 1 H, 3'-*H*-Th), 7.49 (t, J = 7.8 Hz, 2 H, meta-*H*-PhCO), 7.59 (t, J = 7.8 Hz, 1 H, para-*H*-PhCO), 7.79 (d, J = 7.8 Hz, 2 H, ortho-H-PhCO).

**BIOLOGICAL ACTIVITY**

**A) In-Vitro**

Some representative compounds of the invention were tested for their activity as sigma (sigma-1 and sigma-2) inhibitors. The following protocols were followed:

**Sigma-1 (Version A)**

Brain membrane preparation and binding assays for the σ1-receptor were performed as described (DeHaven-Hudkins et al., 1992) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.

The radioligand used was [$^3$H]-(+)-pentazocine at 3.0 nM and the final volume was 200 μl. The incubation was initiated with the addition of 100 μl of membrane at a final tissue concentration of approximately 5 mg tissue net weight/mL and the incubation time was 150 min. at 37 °C. After incubation, the membranes were collected onto pretreated glass fiber filterplate (MultiScreen-FC, Millipore), with polyethylenimine 0.1 %. The filters were washed two times with 200 μl of washing buffer (50 mM Tris Cl, pH = 7.4) and then 25 μl of Ecoscint H liquid scintillation cocktail were added. Microplates were allowed to set for several hours and then quantified by liquid scintillation spectrophotometry (1450 Microbeta, Wallac). Nonspecific binding was determined with 1 μM haloperidol.

The Microbeta reader gave the counts per minute (cpm) per each well that were processed in Excel work sheet to obtain means of duplicates. The Specific Binding value was obtained substracting Non-Specific Binding (NSB) from Total Binding (TB).

Percentages of Specific Bound for each different compound concentration were calculated from duplicates cpm means as follow:

$$\frac{\text{Compound - NSB}}{\text{Specific Bound}} \times 100 \quad \text{[Equation 1]}$$

The values were used for non-linear $IC_{50}$ (nM) calculation and graph representation. Inhibition constant ($K_i$) was calculated from $IC_{50}$ according the Cheng-Prussof Equation:

$$K_i = \frac{IC_{50}}{1 + \frac{[L]}{K_d}} \quad \text{[Equation 2]}$$

In which [L] means the radioligand concentration, determinated from the experimental total counts (dpm) using the Specific Activity of the radioligand, and $K_d$ the disotiation constant of the radioligand.

The historical value of the saturation constant $K_d$ of [$^3$H]-(+)pentazocine was 3.1 nM.

**Sigma 1 (Version B)**

In brief the $\sigma_1$-receptor preparation was prepared from guinea pig brain. The brains were homogenized in 5 to 6 times of volume sucrose solution (0.32M) and homogenized. The homogenate was centrifuged at 2900 rpm, 4˚C, 10 min). the supernatant was centrifuged again (23500 x g, 4˚C, 20 min). The pellet was resuspended in Tris-buffer, incubated for 30 min at room temperature and centrifuged f (23500 x g, 4˚C, 20 min). The pellet was resuspended in cold TRIS-buffer and homogenized. Then the protein content was measured (approx. 1.5 mg/mL) and the homogenate frozen at -80˚C for later use.

The radioligand used was [$^3$H]-(+)-Pentazocin in TRIS-buffer. In a volume of 200 μL 50 μL TRIS-buffer, 50 μL compound solution of varying concentration, 50 μL radioligand- solution (8 nM; resulting in 2 nM in the assay) and finally 50 μL of receptor preparation (approx. 1.5 mg /mL) were given into a well of a microplate equipped with a filter. The plate was closed and stirred for 2.5 h at 37 ˚C and 500 rpm. Following that the solvents were removed by a harvester through the filter. After rinsing with $H_2O$ the filter was measured in a scintillation counter ([$^3$H]-protocol).

**Sigma-2 (Version A)**

Brain membrane preparation and binding assays for the σ2-receptor were performed as described (Ronsisvalle et al., 2001) with some modifications. Brain from 6 male Guinea pigs (400g aprox) were carefully dissected on ice and weighed after discarding cerebellum. Homogenates were prepared in 10 volumes of cold 10 mM Tris-HCl, 0.35 M sucrose, pH 7.4 buffer with Polytron PT3000 and the suspension was centrifuged at 1,000 x g for 10 minutes. Supernatants were reserved on ice and each pellet was resuspended in 4 ml of 10 mM Tris-HCl, 0.35 M sucrose, pH 7.4 buffer, homogenized and centrifuged again at 1,000 x g for 10 minutes. All supernatants were mixed and the obtained pool was centrifuged at 33,000 x g for 15 min. Obtained pellets were resuspended in 1.3 ml of 10 mM Tris-HCl, pH 7.4 buffer and incubated at 25˚C during 15 min and centrifuged again at 33,000 x g for 15 min. Obtained pellets were resuspended in 1 ml of 10 mM Tris-HCl, pH 7.4 buffer, pooled and homogenized using Polytron PT3000 at 13500 rpm during 30 sec.

For each experiment, sigma 2 enriched membrane pellets were brought to room temperature and gently resuspended in binding buffer containing 50 mM Tris-HCl, pH 8.0 to obtain 200 μg protein /100 μl.

The radioligand used was [$^3$H]-DTG at 10 nM and the final incubation volume was 200 μl. The incubation was initiated with the addition of 100 μl of membrane (200μg) and the incubation time was 2 hours. at 25 ˚C. NSB (non specific binding) was achieved with 5 μM Di-o-tolylguanidine (DTG) added in 40 μl volume. Sigma 1 receptors were blocked with the sigma 1 ligand (+)-SKF10047 at 400 nM. After incubation, the membranes were collected onto pretreated glass fiber filterplate (MuftiScreen-FC, Millipore), with polyethylenimine 0.5 %. The filters were washed two times with 200 μl of washing buffer (10 mM Tris-HCl, pH 8.0) and then 25 μl of Ecoscint H liquid scintillation cocktail were added. Microplates were allowed to set for several hours and then quantified by liquid scintillation spectrophotometry (1450 Microbeta, Wallac).

The Microbeta reader gave the counts per minute (cpm) per each well that were processed in Excel work sheet to obtain means of duplicates. The Specific Binding value was obtained substracting Non-Specific Binding (NSB) from Total Binding (TB).

Percentages of Specific Bound for each different compound concentration were calculated from duplicates cpm means as follow:

$$\frac{\text{Compound - NSB}}{\text{Specific Bound}} \times 100 \quad \text{[Equation 1]}$$

The values were used for non-linear $IC_{50}$ (nM) calculation and graph representation. Inhibition constant ($K_i$) was calculated from $IC_{50}$ according the Cheng-Prussof Equation:

$$K_i = \frac{IC_{50}}{1 + \frac{[L]}{K_d}} \quad \text{[Equation 2]}$$

In which [L] means the radioligand concentration, determinated from the experimental total counts (dpm) using the Specific Activity of the radioligand, and $K_d$ the disotiation constant of the radioligand.

The historical value of the saturation constant $K_d$ of [$^3$H]-DTG was 23.3 nM.

**References**

DeHaven-Hudkins, D. L., L.C. Fleissner, and F. Y. Ford-Rice, 1992, "Characterization of the binding of [3H](+) pentazocine to σ recognition sites in guinea pig brain", Eur. J. Pharmacol. 227, 371-378.

Ronsisvalle G, Prezzavento O, Marrazzo A, Vittorio F, Bousquet E, Di Toro R, Spampinato S. 2001 Synthesis and binding affinity of cis-(-)- and cis-(+)-N-ethyleneamino-N-nordeoxymetazocine and cis-(-)-N-normetazocine analogues at sigma1, sigma2 and kappa opioid receptors. Eur J Pharm Sci. 12(3), 277-84.

**SIGMA 2 (Version B)**

In brief the $\sigma_2$-Receptorpreparation was prepared from rat liver. The livers were homogenized in 5 to 6 times of volume sucrose solution (0.32M) and homogenized. The homogenate was centrifuged at 2900 rpm, 4°C, 10 min). the supernatant was centrifuged again (31000 x g, 4°C, 20 min). The pellet was resuspended in TRIS-buffer, incubated for 30 min at room temperature while stirring and centrifuged f (31000 x g, 4°C, 20 min). The pellet was resuspended in cold TRIS-buffer pH 8 and homogenized. Then the protein content was measured (approx. 2 mg/mL) and the homogenate frozen at -80°C for later use.

The radioligand used was [$^3$H]-Ditolylguanidin in TRIS-buffer pH 8. The $\sigma_1$-receptor binding sites were masked through (+)-Pentazocin-solution in TRIS-Puffer pH 8.

In a volume of 200 μL 50 μL compound solution of varying concentration, 50 μL (+)-Pentazocin- solution (2 μM; resulting in 500 nM in the assay), 50 μL radioligand-solution (12 nM; resulting in 3 nM in the assay) and finally 50 μL of receptor preparation (approx. 2 mg /mL) were given into a well of a microplate equipped with a filter. The plate was closed and stirred for 2 h at room temperature and 500 rpm. Following that the solvents were removed by a harvester through the filter. After rinsing with $H_2O$ the filter was measured in a scintillation counter ([$^3$H]-protocol).

Some of the results obtained (through the version B) are shown in table (I).

Table (I)

| Example | Formula | Binding σ1 Ki [nM] | Binding σ2 Ki [nM] or +[Inhibition at 1μM] |
|---|---|---|---|
| 2 | 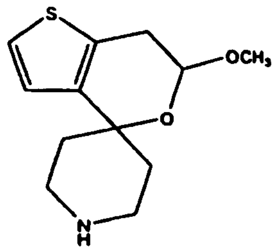 | 602 | 17%+ |
| 3 | 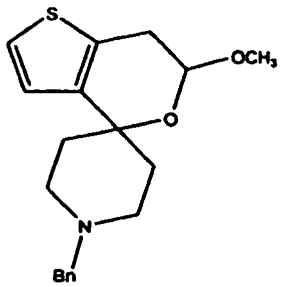 | 0.32 ± 0.16 | 1260 |
| 4 | 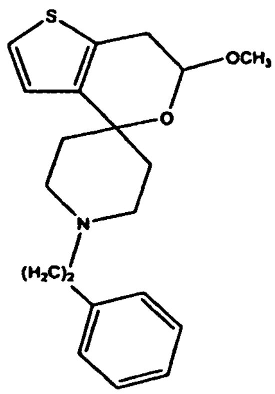 | 5.89 ± 5.10 | 191 |
| 5 | 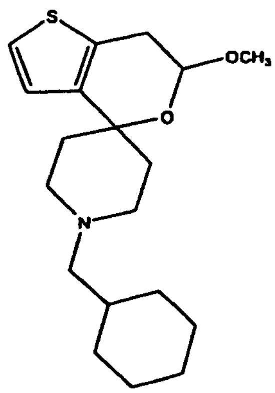 | 0.29 ± 0.079 | 25.4 ± 13.5 |

(continued)

| Example | Formula | Binding σ1 Ki [nM] | Binding σ2 Ki [nM] or +[Inhibition at 1μM] |
|---|---|---|---|
| 6 | 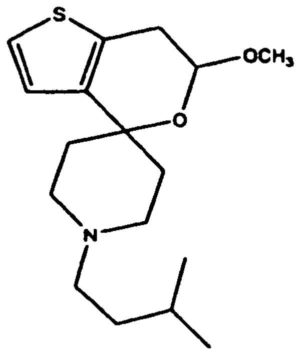 | 0.96 ± 0.53 | 285 |
| 7 | 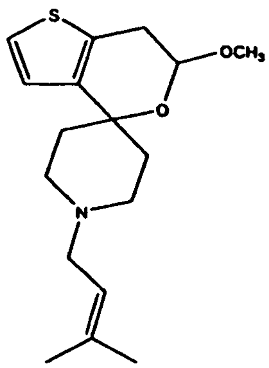 | 0.96 ± 0.25 | 78.1 ± 74.0 |
| 8 | 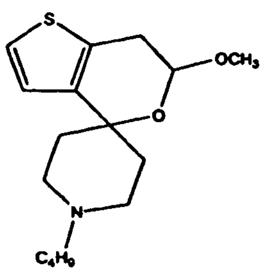 | 4.30 ± 0.44 | 497 ± 294 |
| 9 | 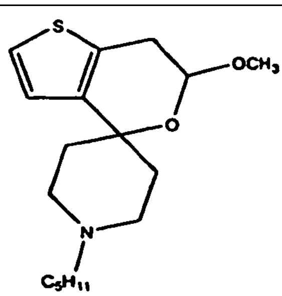 | 1.64 ± 0.53 | 460 |
| 10 | 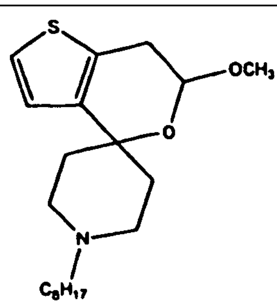 | 4.5 ± 2.0 | 1200 |

(continued)

| Example | Formula | Binding σ1 Ki [nM] | Binding σ2 Ki [nM] or +[Inhibition at 1μM] |
|---|---|---|---|
| 11 | 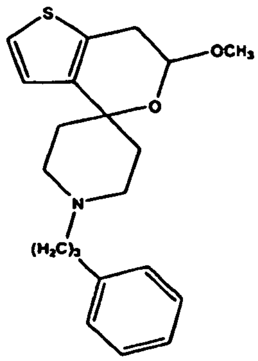 | 2.42 ± 1.66 | 203 |
| 12 | 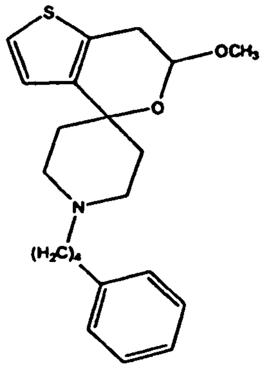 | 7.67 ± 2.15 | 99.2 ± 21.9 |
| 13 | 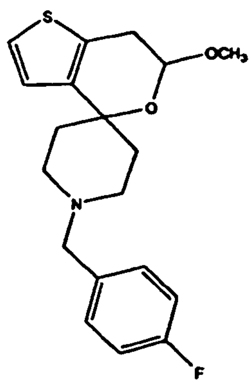 | 3.39 ± 4.54 | 425 |
| 14 | 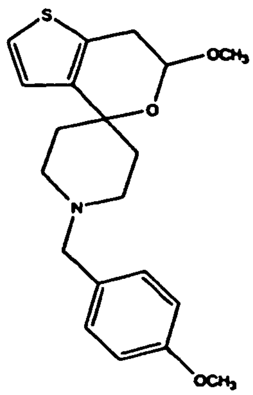 | 2.24 ± 0.99 | 2970 |

(continued)

| Example | Formula | Binding σ1 Ki [nM] | Binding σ2 Ki [nM] or +[Inhibition at 1μM] |
|---|---|---|---|
| 15 | 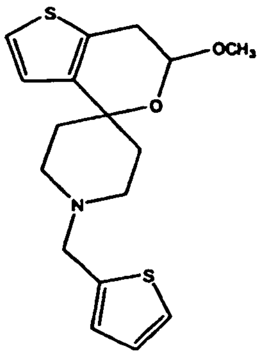 | 2.02 ± 0.61 | 1040 |
| 16 | 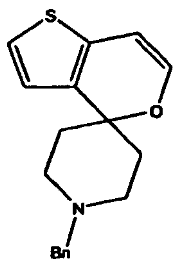 | 1.98 ± 0.89 | 41.9 ± 43.4 |
| 17 | 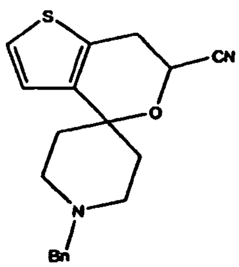 | 1.40 ± 0.32 | 783 |
| 18 | 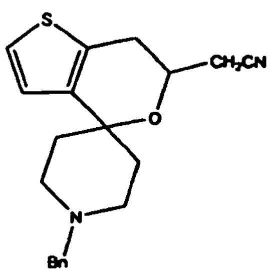 | 3.14 ± 3.33 | 6%* |
| 19 | 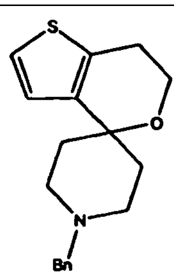 | 0.31 ± 0.11 | 9.6 ± 8.7 |

(continued)

| Example | Formula | Binding σ1 Ki [nM] | Binding σ2 Ki [nM] or +[Inhibition at 1μM] |
|---|---|---|---|
| 20 | 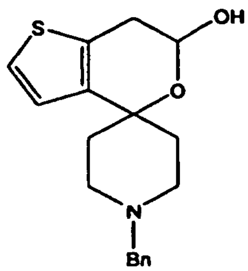 | 3.6 ± 1.16 | 391 |
| 21 | 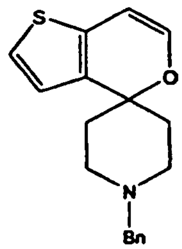 | 1.98 ± 0.89 | 60.8 ± 30.0 |
| 21a | 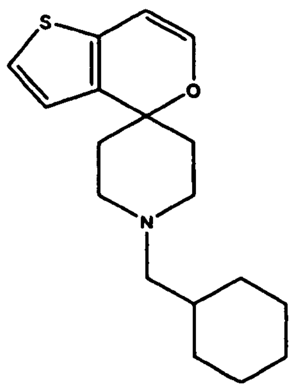 | 1.65 ± 0.15 | 7.32 |
| 22 | 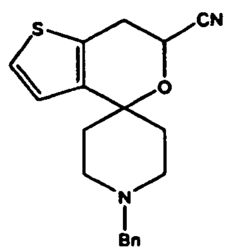 | 1.40 ± 0.32 | 783 |
| 22a | 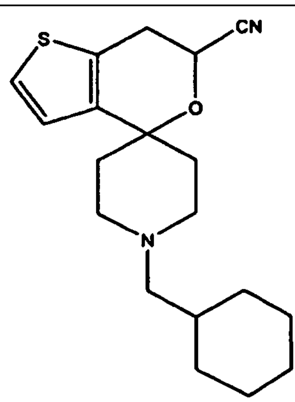 | 0.46 ± 0.07 | 25.8 ± 6.9 |

(continued)

| Example | Formula | Binding σ1 Ki [nM] | Binding σ2 Ki [nM] or +[Inhibition at 1μM] |
|---|---|---|---|
| 23 | 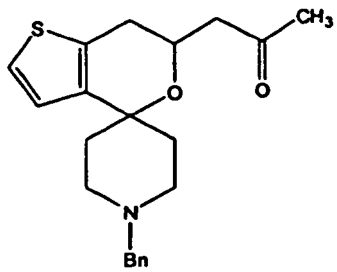 | 9.42 ± 8.0 | 1610 |
| 23a | 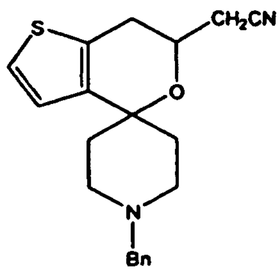 | 3.14 ± 3.33 | 6% |
| 24 | 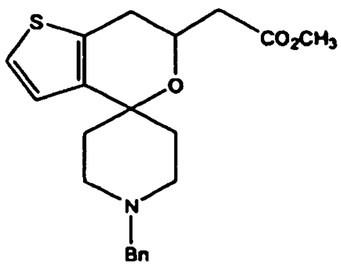 | 12.4 ± 1.2 | 5000 |
| 25 | 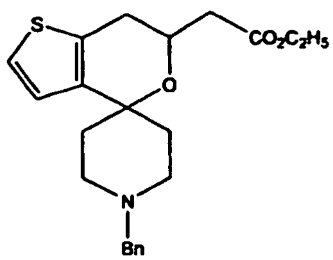 | 20.2 ± 4.9 | 2850 |
| 26 | 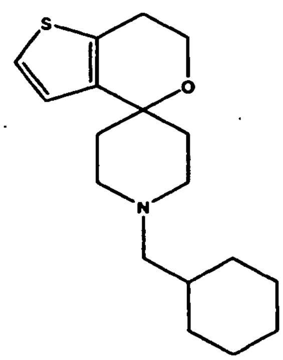 | 0.66 ± 0.27 | 3.27 ± 0.53 |

(continued)

| Example | Formula | Binding σ1 Ki [nM] | Binding σ2 Ki [nM] or +[Inhibition at 1μM] |
|---|---|---|---|
| 26a | 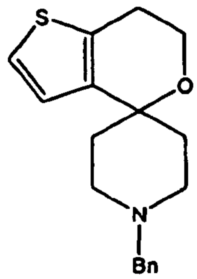 | 0.31 ± 0.11 | 9.6 ± 8.7 |
| 27 | 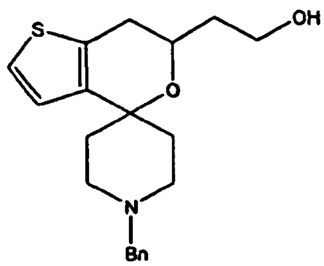 | 27.1 ± 7.5 | 11% |
| REFERENCE 28 | 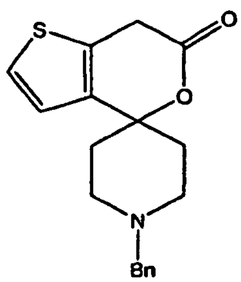 | 296 | 0% |
| 29 | 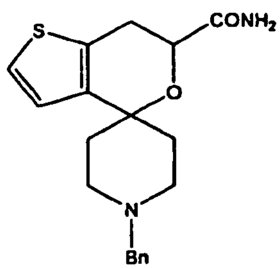 | 350 | 63% |
| 30 | 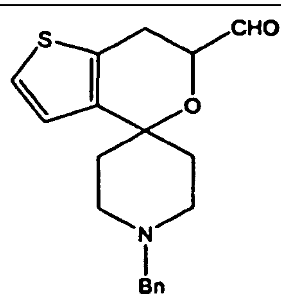 | | |

(continued)

| Example | Formula | Binding σ1 Ki [nM] | Binding σ2 Ki [nM] or +[Inhibition at 1μM] |
|---|---|---|---|
| 31 | 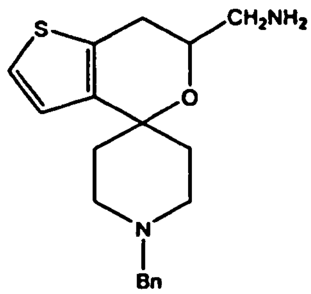 | 98.0 | 7400 |
| 32 | 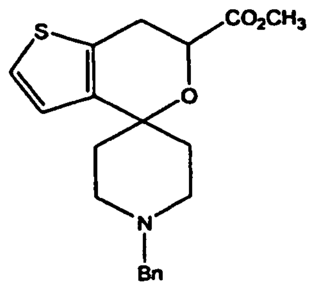 | 29.3 ± 5.5 | 6600 |
| 33 | 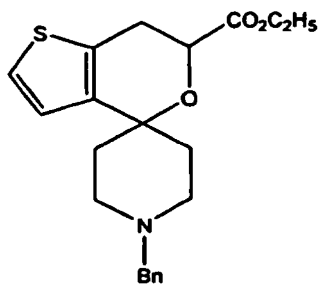 | 144 | 0% |
| 34 | 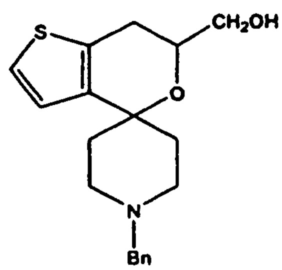 | 5.4 ± 2.55 | 4300 |
| 35 | 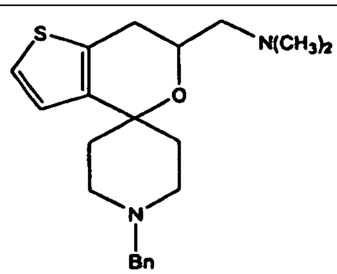 | 62.5 | 8100 |

(continued)

| Example | Formula | Binding σ1 Ki [nM] | Binding σ2 Ki [nM] or +[Inhibition at 1μM] |
|---|---|---|---|
| 36 | 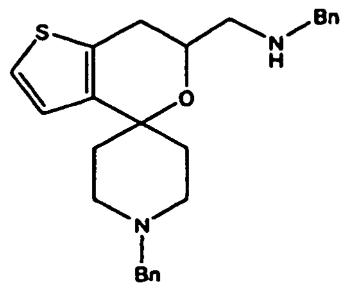 | 196 ± 41.2 | 621 |
| 37 | 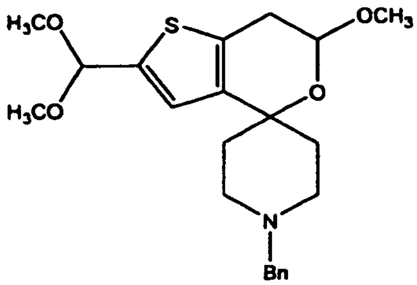 | 2.33 ± 0.65 | 4280 |
| 38 | 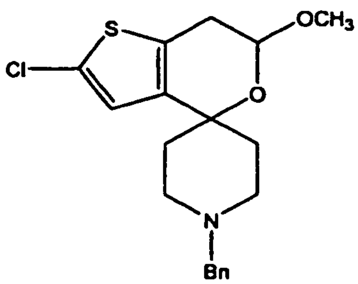 | 1.25 ± 0.11 | 49% |
| 39 | 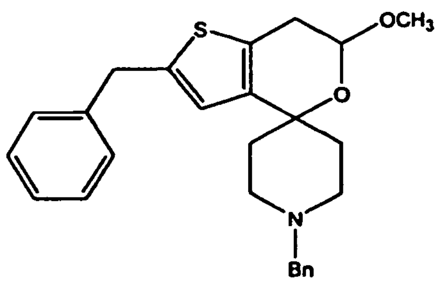 | 3.26 ± 1.15 | 411 |
| 40 | 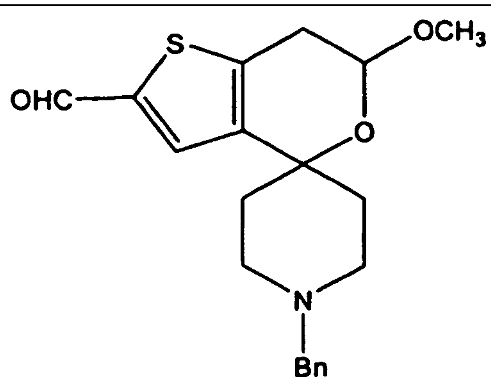 | | |

(continued)

| Example | Formula | Binding σ1 Ki [nM] | Binding σ2 Ki [nM] or +[Inhibition at 1μM] |
|---|---|---|---|
| 41 | 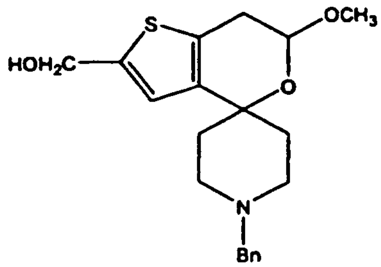 | 5.84 ± 2.03 | 4650 |
| 42 | 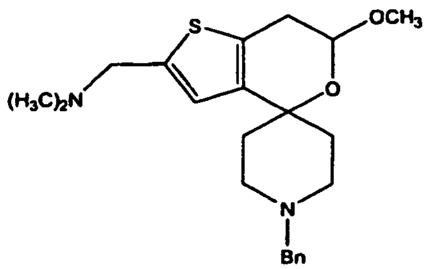 | 16.8 ± 1.10 | 898 |
| 43 | 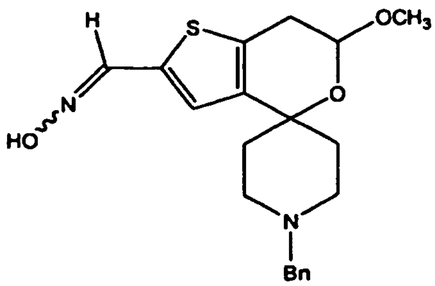 | 38.5 ± 4.6 | 2% |
| 44 | 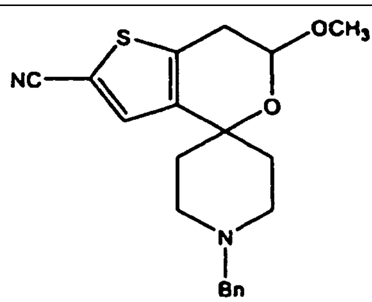 | 1.10 ± 0.15 | 16% |
| 45 | 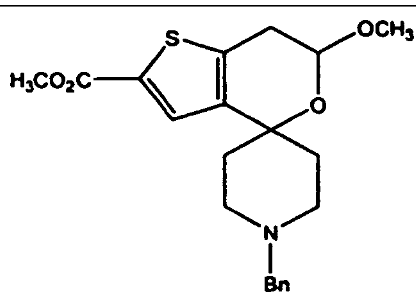 | 10.9 ± 2.2 | 45% |

(continued)

| Example | Formula | Binding σ1 Ki [nM] | Binding σ2 Ki [nM] or +[Inhibition at 1μM] |
|---|---|---|---|
| 46 | 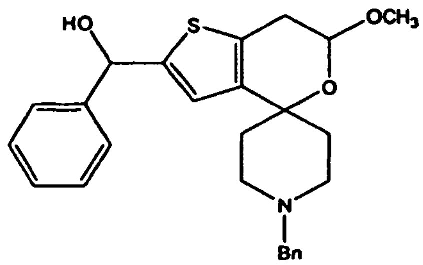 | 3.59 ± 2.51 | 882 ± 17 |
| 47 | 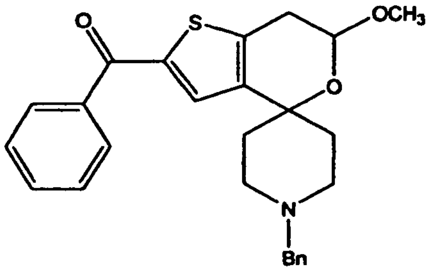 | 3.74 ± 2.37 | 427 ± 163 |

Some other results obtained for Sigma-1 (by version A) are shown in table (II).

Table (II)

| Example | Binding σ1; Ki [nM] |
|---|---|
| **3** | 2.4 ± 0.3 |
| **5** | 2.2 ± 0.3 |
| **6** | 1.3 ± 0.5 |
| **8** | 5.5 ± 1.2 |
| **9** | 2.9 ± 0.5 |
| **13** | 2.3 ± 0.4 |
| **17** | 4.5 ± 0.5 |
| **19** | 2.1 t 0.4 |

**B)** **<u>In-Vivo</u>**

**EFFECT ON CAPSAICIN IN DEVELOPMENT OF MECHANICAL ALLODYNIA**

This model uses the von-Frey Filaments and is a model to test the effects or symptoms of neuropathic pain, allodynia etc.

interest of the model:

- The injection of 1 μg of capsaicin to experimental animals produces acute pain followed by hyperalgesia/allodynia
- The mechanisms involved in capsaicin-induced acute pain and hyperalgesia are relatively well known (mainly activation of peripheral nociceptors and sensitization of spinal cord neurons, respectively)

The test protocol for all tests with of Frey filaments: After habituation mice were first treated with the test-compound (or solvent in controls). Then 1 μg capsaicin (1% DMSO) is injected into their paw resulting in developing pain in the effected paw. The effected paw is then treated with a mechanical stimulus and the latency time before the paw is withdrawn is measured.

Using this pharmacological test the compounds according to example 22 and example 25 showed a clear antiallodynic effect. They were tested at 16 mg/kg i.p. in 8 animals each and respectively achieved 45.67 ± 9.62 % and 44.28 ± 9.49 % of pain relief.

## Claims

1. Compound of general formula (I) or (Ia),

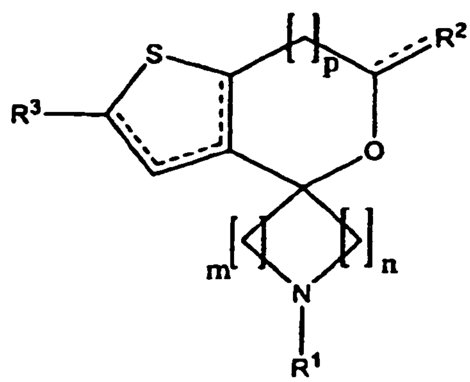

or

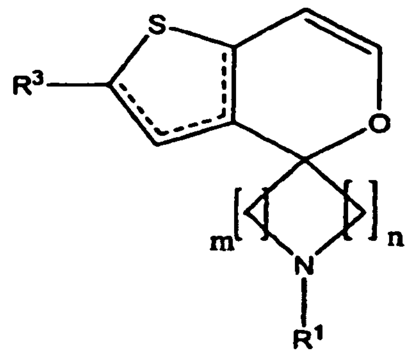

(I) (Ia)

wherein
m is selected from 1, 2 or 3, n is selected from 1, 2 or 3, and m + n is either 3, 4 or 5;
p is selected from 0 or 1;
the dotted lines <u>-----</u> are either a double or a single bond;
R' is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
$R^2$ is selected from hydrogen; an optionally at least monosubstituted, linear or branched $C_{1-8}$-aliphatic group; $(CH_2)_qCN$ with q being 0, 1, 2, 3 or 4; O-R with R being H or an optionally at least monosubstituted, linear or branched $C_{1-6}$-aliphatic group; $(CH_2)_sCOR^*$; $(CH_2)_sNR^*R^{**}$; $CONR^*R^{**}$; $(CH_2)_sCO_2R^*$; CH=NOR*; $(CH_2)_sOR^*$;
with
R* being H; or an optionally at least monosubstituted, linear or branched $C_{1-6}$-aliphatic group;
R** being an optionally at least monosubstituted, linear or branched $C_{1-8}$-aliphatic group; or an optionally at least monosubstituted aryl or alkyl-aryl;
s being 0, 1, or 2;
$R^3$ is selected from hydrogen; halogen; an optionally at least monosubstituted, linear or branched $C_{1-18}$-aliphatic group; CN; CH(=NOH); $NO_2$; $SO_3H$; COR'''; $(CH_2)$,NR'R"; CH(OR')(OR"); $CO_2R'$; an optionally at least monosubstituted alkyl-aryl with alkyl optionally being substituted by OH; an optionally at least monosubstituted alkyl-heterocyclyl with alkyl optionally being substituted by OH or forming a C(O)-keto-group; or an optionally at least monosubstituted alkyl-cycloalkyl with alkyl optionally being substituted by OH;
with
R' being H; or an optionally at least monosubstituted, linear or branched $C_{1-8}$-aliphatic group;
R" being an optionally at least monosubstituted, linear or branched $C_{1-6}$-aliphatic group;
R"' being H; an optionally at least monosubstituted, linear or branched $C_{1-8}$-aliphatic group; or an optionally at least monosubstituted aryl, an optionally at least monosubstituted heterocyclyl, or an optionally at least monosubstituted cycloalkyl;
r being 0, 1, or 2;

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
with the proviso that if $R^2$ and $R^3$ are both hydrogen; R' may not be methyl.

**2.** Compound according to claim 1, having a general formula **Ic,**

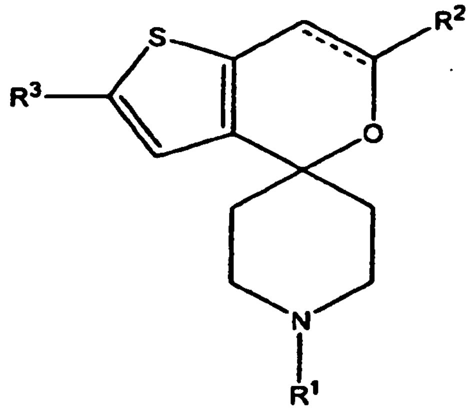

**(Ic)**

wherein
the dotted lines ----- are either a double or a single bond;
$R^1$ is selected from hydrogen; an optionally at least monosubstituted, linear or branched $C_{1-18}$-aliphatic group; an optionally at least monosubstituted aryl; an optionally at least monosubstituted heterocyclyl; an optionally at least monosubstituted cycloalkyl; an optionally at least monosubstituted alkyl-aryl; an optionally at least monosubstituted alkyl-heterocyclyl; or an optionally at least monosubstituted alkyl-cycloalkyl;
$R^2$ is selected from hydrogen; an optionally at least monosubstituted, linear or branched $C_{1-8}$-aliphatic group; $(CH_2)_qCN$ with q being 0, 1, 2, 3 or 4; O-R with R being H or an optionally at least monosubstituted, linear or branched $C_{1-6}$-aliphatic group; $(CH_2)_sCOR^*$; $(CH_2)_sNR^*R^{**}$; $CONR^*R^{**}$; $(CH_2)_sCO_2R^*$; $CH{=}NOR^*$; $(CH_2)_sOR^*$;
with
R* being H; or an optionally at least monosubstituted, linear or branched $C_{1-6}$-aliphatic group;
R** being an optionally at least monosubstituted, linear or branched $C_{1-6}$-aliphatic group; or an optionally at least monosubstituted aryl or alkyl-aryl;
s being 0, 1, or 2;
$R^3$ is selected from hydrogen; halogen; an optionally at least monosubstituted, linear or branched $C_{1-18}$-aliphatic group; CN; CH(=NOH); $NO_2$; $SO_3H$; COR'''; $(CH_2)_rNR'R''$; CH(OR')(OR''); $CO_2R'$; an optionally at least monosubstituted alkyl-aryl with alkyl optionally being substituted by OH; an optionally at least monosubstituted alkyl-heterocyclyl with alkyl optionally being substituted by OH or forming a C(O)-keto-group; or an optionally at least monosubstituted alkyl-cycloalkyl with alkyl optionally being substituted by OH;
with
R' being H; or an optionally at least monosubstituted, linear or branched $C_{1-6}$-aliphatic group;
R'' being an optionally at least monosubstituted, linear or branched $C_{1-6}$-aliphatic group;
R''' being H; an optionally at least monosubstituted, linear or branched $C_{1-6}$-aliphatic group; or an optionally at least monosubstituted aryl, an optionally at least monosubstituted heterocyclyl, or an optionally at least monosubstituted cycloalkyl;
r being 0, 1, or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof;
with the proviso that if $R^2$ and $R^3$ are both hydrogen, R' may not be methyl.

**3.** Compound according to any of claims 1 or 2, **characterized in that** R' is selected from H; an optionally at least monosubstituted, linear or branched $C_{1-18}$-aliphatic group; an optionally at least monosubstituted alkyl-aryl; an

optionally at least monosubstituted alkyl-heterocyclyl; or an optionally at least monosubstituted alkyl-cycloalkyl.

4. Compound according to any of claims 1 to 3, **characterized in that** $R^2$ is selected from hydrogen; an optionally at least monosubstituted, linear or branched $C_{1-6}$-aliphatic group; or OR with R being H or an optionally at least monosubstituted, linear or branched $C_{1-6}$-aliphatic group; $(CH_2)_q$-CN, $(CH_2)$q-NH-benzyl; $(CH_2)_q$-$N(R^{22})_2$; $(CH_2)_q$-C(O)-$N(R^{22})_2$; $(CH_2)_q$-C(O)-$R^{22}$ or $(CH_2)_q$-C(O)-O-$R^{22}$, with q being 0, or 1 and $R^{22}$ being H; or an optionally at least monosubstituted, linear or branched $C_{1-6}$-aliphatic group; and/or
**characterized in that** $R^3$ is selected from hydrogen; halogen; CN; CH(=NOH); an optionally at least monosubstituted, linear or branched $C_{1-10}$-aliphatic group; $CH_2$-$R^{33}$ ; C(O)-$R^{33}$; C(O)O-$R^{33}$; CHOH-$R^{33}$; $CH_2N(R^{33})_2$ with $R^{33}$ being H; or an optionally at least monosubstituted, linear or branched $C_{1-6}$-aliphatic group; or an optionally at least monosubstituted aryl.

5. Compound according to claim 1, **characterized in that** m and n are selected from 1 or 2 and m+n are selected from 3 or 4.

6. Compound according to any of claims 1 or 2, having a general formula Id,

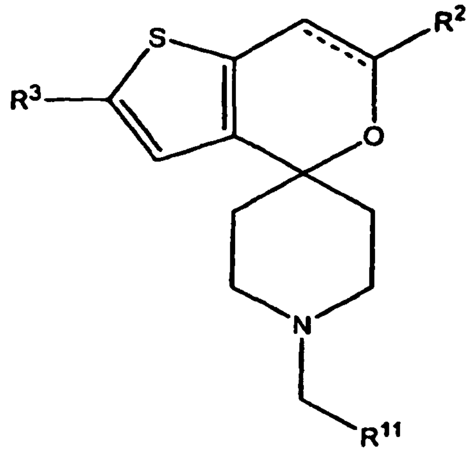

**(Id)**

wherein
the bond with the dotted line ........ either is a double or a single bond:
R" is selected from an optionally at least monosubstituted, linear or branched $C_{1-9}$-aliphatic group; an optionally at least monosubstituted aryl or alkyl-aryl; an optionally at least monosubstituted heterocyclyl or alkyl-heterocyclyl; an optionally at least monosubstituted cycloalkyl or alkyl-cycloalkyl;
$R^2$ is selected from hydrogen; an optionally at least monosubstituted, linear or branched $C_{1-6}$-aliphatic group; or OR with R being H or an optionally at least monosubstituted, linear or branched $C_{1-6}$-aliphatic group; $(CH_2)_q$-CN, $(CH_2)_q$-NH-benzyl; $(CH_2)_q$-$N(R^{22})_2$; $(CH_2)_q$-C(O)-$N(R^{22})_2$; $(CH_2)_q$-C(O)-$R^{22}$ or $(CH_2)_q$-C(O)-$OR^{22}$, with q being 0, or 1 and $R^{22}$ being H; or an optionally at least monosubstituted, linear or branched $C_{1-6}$-aliphatic group;
$R^3$ is selected from hydrogen; halogen; CN; CH(=NOH); an optionally at least monosubstituted, linear or branched $C_{1-10}$-aliphatic group; $CH_2$-$R^{33}$; C(O)-$R^{33}$; C(O)O-$R^{33}$; CHOH-$R^{33}$; $CH_2N(R^{33})_2$ with $R^{33}$ being H; an optionally at least monosubstituted, linear or branched $C_{1-6}$-aliphatic group; or an optionally at least monosubstituted aryl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

7. Compound according to claim 6, **characterized in that** $R^{11}$ is selected from an optionally at least monosubstituted, linear or branched $C_{1-9}$-aliphatic group; an optionally at least monosubstituted aryl or alkyl-aryl; an optionally at least monosubstituted heterocyclyl; or an optionally at least monosubstituted cycloalkyl; preferably is phenyl, p-methoy-phenyl, cyclohexyl, thiophene, benzyl; branched or linear $C_{3-7}$-alkyl; or branched or linear $C_{3-7}$-alkenyl; and/or
**characterized in that** $R^2$ is selected from hydrogen; an unsubstituted or by at least one of OH, F, or Cl substituted,

linear or branched $C_{1-6}$-aliphatic group; or OR with R being H or CH3; $(CH_2)_q$-CN, $(CH_2)_q$-NH-benzyl; $(CH_2)_q$-N$(R^{22})_2$; $(CH_2)$q-C(O)-N$(R^{22})_2$; $(CH_2)_q$-C(O)-$R^{22}$ or $(CH_2)_q$-C(O)-O-$R^{22}$, with q being 0, or 1 and $R^{22}$ being H, $CH_3$, or $C_2H_5$ ; and/or

**characterized in that** $R^3$ is selected from hydrogen; halogen; CN; CH(=NOH); a linear or branched, unsubstituted or by at least one of OH, Cl, or F substituted $C_{1-8}$-aliphatic group; $CH_2R^{33}$; C(O)-$R^{33}$; C(O)O-$R^{33}$; CHOH-R $^{33}$; $CH_2N(R^{33})_2$ with $R^{33}$ being H; $CH_3$; $C_2H_5$; or phenyl.

**8.** Compound according to claim 1, **characterized in that** the compound is selected from

- 6'-Methoxy-6',7'-dihydrospiro[piperidin-4,4'-thieno[3,2-c]pyran;
- 1-Benzyl-6'-methoxy-6',7'-dihydrospiro[piperidin-4,4'-thieno[3.2-c]pyran];
- 6'-Methoxy-1-(2-phenylethyl)-6',7'-dihydrospiro[piperidin-4,4'-thieno[3.2-c]pyran];
- 1-(Cyclohexylmethyl)-6'-Methoxy -6',7'-dihydrospiro[piperidin-4,4'-thieno[3.2-c]pyran];
- 6'-Methoxy-1-(3-methylbutyl)-6',7'-dihydrospiro[piperidin-4,4'-thieno[3.2-c]pyran];
- 6'-Methoxy-1-(3-methylbut-2-enyl)-6',7'-dihydrospiro[piperidin-4,4'-thieno[3.2-c]pyran];
- 1-Butyl-6'-methoxy-6',7'-dihydrospiro[piperidin-4,4'-thieno[3.2-c]pyran];
- 6'-Methoxy-1-pentyl-6',7'-dihydrospiro[piperidin-4.4'-thieno[3.2-c]pyran];
- 6'-Methoxy-1-octyl -6',7'-dihydrospiro[piperidin-4,4'-thieno[3.2-c]pyran];
- 3'-Methoxy-1-(3-phenylpropyl)-6',7'-dihydrospiro[piperidin-4,4'thieno[3.2-c]pyran];
- 3'-Methoxy-1-(4-phenylbutyl)-6',7'-dihydrospiro[piperidin-4,4'-thieno[3.2-c]pyran];
- 1-(4-Fluorbenzyl)-6'-Methoxy-6',7'-dihydrospiro[piperidin-4,4'-thieno[3.2-c]pyran];
- 6'-Methoxy-1-(4-methoxybenzyl)-6',7'-dihydrospiro[piperidin-4,4'-thieno[3.2-c]pyran];
- 6'-Methoxy-1-(thien-2-ylmethyl)-6',7'dihydrospiro[piperidin-4,4'-thieno[3.2-c]pyran];
- 1-Benzylspiro[piperidin-4,4'-thieno[3,2-c]pyran];
- 1-Benzyl-6',7'-dihydrospiro[piperidin-4,4'-thieno[3,2-c]pyran]-6'-carbonitril;
- {1-Benzyl-6',7'-dihyrospiro[piperidin-4,4'-thieno[3,2-c]pyran]-6'-yl}acetonitril;
- 1-Benzyl-6',7'-dihydrospiro[piperidin-4,4'-thieno[3,2-c]pyran];
- 1-Benzyl-6',7'-dihydrospiro[piperidin-4,4'-thieno[3,2-c]pyran]-6'-ol
- 1-(Cyclohexylmethyl)spiro[piperidine-4,4'-thieno[3,2-c]pyrano]
- 1-(Cyclohexylmethyl)-6',7'-dihydrospiro[piperidine-4.4'-thieno[3.2-c]pyranol-6'-carbonitrile
- 1-(1-Benzyl-6',7'-dihydrospiro[piperidine-4,4'-thieno[3,2-c]pyrano]-6'-yl)acetone
- Methyl-2-(1-benzyl-6',7'-dihyrospiro[piperidin-4,4'-thieno[3,2-c]pyrano]-6'-yl)-acetate
- Ethyl-2-(1-benzyl-6',7'-dihyrospiro[piperidine-4,4'-thieno[3,2-c]pyrano]-6'-yl)acetate
- 1-(Cyclohexylmethyl)-6',7'-dihydrospiro[piperidine-4,4'-thieno[3,2-c]pyrano]
- 2-(1-Benzyl-6',7'-dihyrospiro[piperidine-4,4'-thieno[3,2-c]pyrano]-6'-yl)ethanol
- 1-Benzyl-6',7'-dihyrospiro[piperidine-4,4'thieno[3,2-c]pyrano]-6'-carboxamide
- 1-Benzyl-6',7'-dihyrospiro[piperidine-4,4'-thieno[3,2-c]pyrano]-6'-carbaldehyde
- (1-Benzyl-6',7'-dihydrospiro[piperidine-4,4'-thieno[3,2-c]pyrano]-6'-yl)methanamine
- Methyl-1-benzyl-6',7'-dihyrospiro[piperidine-4,4'-thieno[3,2-c]pyrano]-6'-carboxylate
- Etyhl-1-benzyt-6',7'-dihyrospiro[piperidine-4,4'-thieno[3,2-c]pyrano]-6'-carboxylate
- (1-Benzyl-6',7'-dihyrospiro[piperidine-4,4'-thieno[3,2-c]pyrano]-6'-yl)methanol
- (1-Benzyl-6',7'-dihydrospiro[piperidine-4,4'-thieno[3,2-c]pyrano]-6'-yl)-N,N-dimethylmethanamine
- (1-Benzyl-6',7'-dihydrospiro[piperidine-4,4'-thieno[3,2-c]pyrano]-6'-yl)-N-benzyl-methanamine
- (1-Benzyl-6'-methoxy-6',7'-dihydrospiro[piperidine-4,4'-thieno[3.2-c]pyrano]-2-carbaldehyde-dimethylacetal
- 1-Benzyl-2'-chloro-6'-methoxy-6',7'-dihydrospiro[piperidine-4,4'-thieno[3.2-c]-pyrano]
- 1,2'-Dibenzyl-6'-methoxy-6',7'-dihydrospiro[piperidine-4,4'-thieno[3.2-c]pyrano]
- (1-Benzyl-6'-methoxy-6',7'-dihydrospiro[piperidne-4,4'-thieno[3.2-c]pyrano]-2-carbaldehyde
- (1-Benzyl-6'-methoxy-6',7'-dihydrospiro[piperidine-4,4'-thieno[3.2-c]pyrano]-2'-yl)-methanol
- (1-Benzyl-6'-methoxy-6',7'-dihydrospiro[piperidine-4,4'-thieno[3.2-c]pyrano]-2'-yl)-N,N-dimethylmethanamine
- (E/Z)-1-Benzyl-6'-methoxy-6',7'-dihydrospiro[piperidine-4,4'-thieno[3,2-c]pyrano]-2-carbaldehydoxim
- 1-Benzyl-6'-methoxy-6',7'-dihydrospiro[piperidine-4,4'-thieno[3,2-c]pyrano]-2-carbonitrile
- Methyl-1-benzyl-6'-methoxy-6',7'-dihydrospiro[piperidine-4,4'-thieno[3,2-c]pyrano] -2-carboxylate
- 1-(1-Benzyl-6'-methoxy-6',7'-dihydrospiro[piperidine-4,4'-thieno[3.2-c]pyrano]-2-yl)-1-phenylmethanol; or
- 1-(1-Benzyl-6'-methoxy-6',7'-dihydrospiro[piperidine-4,4'-thieno[3.2-c]pyrano]-2-yl)-1-phenylmethanone;

optionally in form of one of the stereoisomers, preferably enantiomers or diastereomers, a racemate or in form of a mixture of at least two of the stereoisomers, preferably enantiomers and/or diastereomers, in any mixing ratio, or

a corresponding salt thereof, or a corresponding solvate thereof.

**9.** Compound according to claim 1, having a general formula Ib,

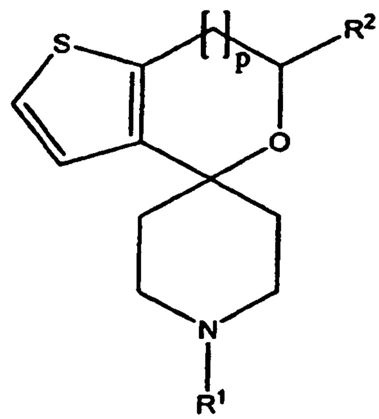

(Ib)

wherein
p is selected from 0 or 1;
R' is selected from hydrogen; $C_{1-10}$-alkyl, linear or branched; $C_{2-8}$-alkenyl, linear or branched; optionally at least mono-substituted $C_{1-6}$-alkyl-aryl; optionally at least mono-substituted $C_{1-6}$-alkyl-heterocyclyl; or optionally at least mono-substituted $C_{1-6}$-alkyl-$C_{4-8}$-cycloalkyl;
$R^2$ is selected from H; CN; $CH_2CN$; OH or a linear or branched $OC_{1-4}$-alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**10.** Process for the production of a compound according to claim 1, wherein a compound of formula (III)

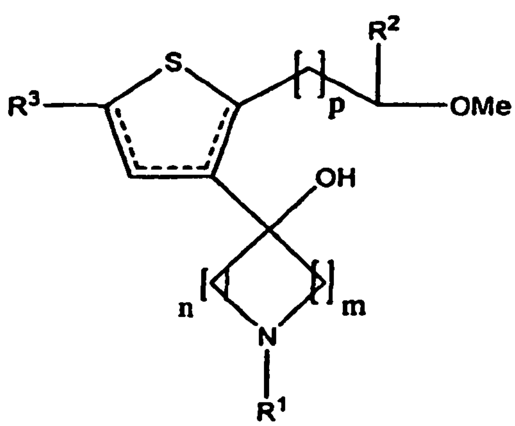

(III)

wherein $R^1$, $R^2$, $R^3$, m, n and p are as defined in claim 1 is reacted with p-toluol sulphonic acid to form acompound according the formula (I); preferably
wherein

- $R^2$ is $OCH_3$; and/or
- $R^3$ is H.

**11.** Process for the production of a compound of claim 9 according to formula Ib, wherein a compound of formula (IIIb)

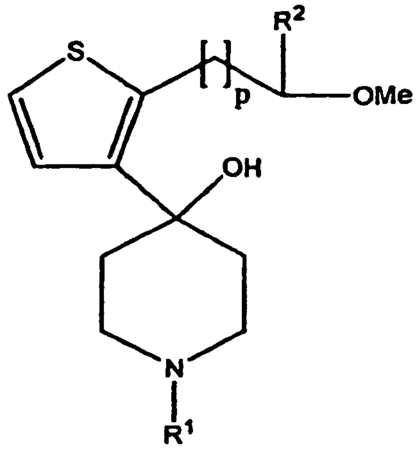

(IIIb)

, wherein $R^1$, $R^2$ and p are as defined in claim 1 is reacted with p-toluol sulphonic acid to form a compound according the formula (Ib); preferably
wherein

- $R^2$ is $OCH_3$ and/or
- p is 1.

**12.** Process according to claims 10, wherein as a next step a compound according to formula (I) in which $R^3$ is C(O) OR' is reacted with a alkaline solution in water to form a compound according to formulas (I) with $R^1$ being H.

**13.** A pharmaceutical composition which comprises a compound as defined in any of claims 1 to 9 or a pharmaceutically acceptable salt or solvate thereof, and a pharmaceutically acceptable carrier, adjuvant or vehicle.

**14.** Use of a compound as defined in any of claims 1 to 9 in the production of a medicament for the treatment or prophylaxis of a sigma receptor mediated disease or condition.

**15.** Use according to claim 14 wherein the disease is diarrhoea, lipoprotein disorders, metabolic syndrome, treatment of elevated triglyceride levels, chylomicronemia, hyperlipoproteinemia; hyperlipidemia, especially mixed hyperlipidemia; hypercholesterolemia, dysbetalipoproteinemia, hypertriglyceridemia including both the sporadic and familial disorder (inherited hypertriglyceridemia), migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine, tardive diskinesia, ischemic stroke, epilepsy, stroke, depression, stress, psychotic condition, schizophrenia; inflammation, autoimmune diseases or cancer; or
wherein the disease is pain, especially neuropathic pain, inflammatory pain or other pain conditions, allodynia and/or hyperalgesia, especially mechanical allodynia.

## Patentansprüche

**1.** Verbindung der allgemeinen Formel (I) oder (Ia),

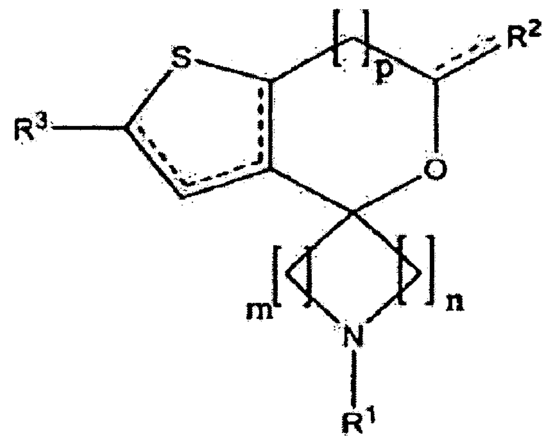

(I)

oder

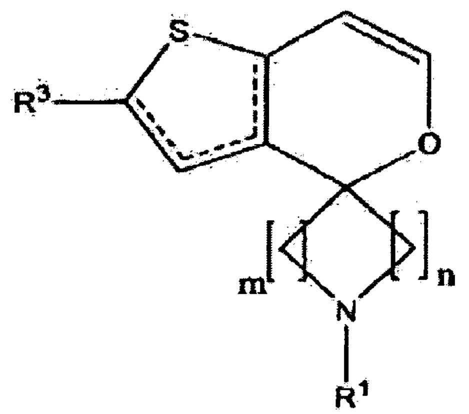

(Ia)

wobei

m ausgewählt ist aus 1, 2 oder 3, n ausgewählt ist aus 1, 2 oder 3 und m + n entweder 3, 4 oder 5 ist;

p ausgewählt ist aus 0 oder 1;

die gepunkteten Linien entweder eine Doppel- oder eine Einfachbindung sind;

$R^1$ ausgewählt ist aus Wasserstoff; einer wahlweise mindestens monosubstituierten, linearen oder verzweigten aliphatischen $C_{1-18}$-Gruppe; einem wahlweise mindestens monosubstituierten Aryl; einem wahlweise mindestens monosubstituierten Heterocyclyl; einem wahlweise mindestens monosubstituierten Cycloalkyl; einem wahlweise mindestens monosubstituierten Alkylaryl; einem wahlweise mindestens monosubstituierten Alkylheterocyclyl; oder einem wahlweise mindestens monosubstituierten Alkylcycloalkyl;

$R^2$ ausgewählt ist aus Wasserstoff; einer wahlweise mindestens monosubstituierten, linearen oder verzweigten aliphatischen $C_{1-6}$-Gruppe; $(CH_2)_qCN$, wobei q gleich 0, 1, 2, 3 oder 4 ist; O-R, wobei R H oder eine wahlweise mindestens monosubstituierte, lineare oder verzweigte aliphatische $C_{1-6}$-Gruppe ist; $(CH_2)_sCOR^*$; $(CH_2)_sNR^*R^{**}$; $CONR^*R^{**}$; $(CH_2)_sCO_2R^*$; $CH{=}NOR^*$; $(CH_2)_sOR^*$;

wobei

$R^*$ H ist; oder eine wahlweise mindestens monosubstituierte, lineare oder verzweigte aliphatische $C_{1-6}$-Gruppe;

$R^{**}$ eine wahlweise mindestens monosubstituierte, lineare oder verzweigte aliphatische $C_{1-6}$-Gruppe ist; oder ein wahlweise mindestens monosubstituiertes Aryl oder Alkylaryl ist;

s 0, 1 oder 2 ist;

$R^3$ ausgewählt ist aus Wasserstoff; Halogen; einer wahlweise monosubstituierten, linearen oder verzweigten ali-

phatischen $C_{1-18}$-Gruppe; CN; CH (=NOH) ; $NO_2$; $SO_3H$; COR'''; $(CH_2)_rNR'R'''$; CH(OR')(OR''); $CO_2R'$; einem wahlweise mindestens monosubstituierten Alkylaryl, wobei Alkyl wahlweise durch OH substituiert ist; einem wahlweise mindestens monosubstituiertes Alkylheterocyclyl, wobei Alkyl wahlweise durch OH substituiert ist oder eine C(O)-Ketogruppe bildet; oder einem wahlweise mindestens monosubstituierten Alkylcycloalkyl, wobei Alkyl wahlweise durch OH substituiert ist;
wobei
R' H oder eine wahlweise mindestens monosubstituierte, lineare oder verzweigte aliphatische $C_{1-6}$-Gruppe ist;
R'' eine wahlweise mindestens monosubstituierte, lineare oder verzweigte aliphatische $C_{1-6}$-Gruppe ist;
R''' H; eine wahlweise mindestens monosubstituierte, lineare oder verzweigte aliphatische $C_{1-6}$-Gruppe; oder ein wahlweise mindestens monosubstituiertes Aryl, ein wahlweise mindestens monosubstituiertes Heterocyclyl oder ein wahlweise mindestens monosubstituiertes Cycloalkyl ist;
r 0, 1 oder 2 ist;
wahlweise in Form eines der Stereoisomere, vorzugsweise Enantiomere oder Diastereomere, eines Racemats oder in Form einer Mischung von mindestens zwei der Stereoisomere, vorzugsweise Enantiomere und/oder Diastereomere, in einem beliebigen Mischverhältnis, oder eines entsprechenden Salzes davon oder eines entsprechenden Solvats davon;
mit der Maßgabe, dass, wenn $R^2$ und $R^3$ beide Wasserstoff sind, $R^1$ nicht Methyl sein kann.

2. Verbindung nach Anspruch 1 mit der allgemeinen Formel Ic,

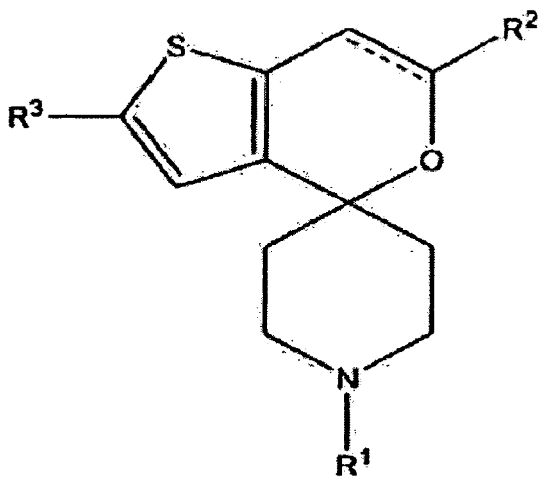

(Ic)

wobei
die gepunkteten Linien entweder eine Doppel- oder eine Einfachbindung sind;
$R^1$ ausgewählt ist aus Wasserstoff; einer wahlweise mindestens monosubstituierten, linearen oder verzweigten aliphatischen $C_{1-18}$-Gruppe; einem wahlweise mindestens monosubstituierten Aryl; einem wahlweise mindestens monosubstituierten Heterocyclyl; einem wahlweise mindestens monosubstituierten Cycloalkyl; einem wahlweise mindestens monosubstituierten Alkylaryl; einem wahlweise mindestens monosubstituierten Alkylheterocyclyl; oder einem wahlweise mindestens monosubstituierten Alkylcycloalkyl;
$R^2$ ausgewählt ist aus Wasserstoff; einer wahlweise mindestens monosubstituierten, linearen oder verzweigten aliphatischen $C_{1-6}$-Gruppe; $(CH_2)_qCN$, wobei q gleich 0, 1, 2, 3 oder 4 ist; O-R, wobei R H oder eine wahlweise mindestens monosubstituierte, lineare oder verzweigte aliphatische $C_{1-6}$-Gruppe ist; $(CH_2)_sCOR^*$; $(CH_2)_sNR^*R^{**}$; $CONR^*R^{**}$; $(CH_2)_sCO_2R^*$; CH=NOR*; $(CH_2)_sOR^*$;
wobei
R* H oder eine wahlweise mindestens monosubstituierte, lineare oder verzweigte aliphatische $C_{1-6}$-Gruppe ist;
R** eine wahlweise mindestens monosubstituierte, lineare oder verzweigte aliphatische $C_{1-6}$-Gruppe; oder ein wahlweise mindestens monosubstituiertes Aryl oder Alkylaryl ist;
s 0, 1 oder 2 ist;
$R^3$ ausgewählt ist aus Wasserstoff; Halogen; einer wahlweise monosubstituierten, linearen oder verzweigten aliphatischen $C_{1-18}$-Gruppe; CN; CH(=NOH) ; $NO_2$; $SO_3H$; COR'''; $(CH_2)_rNR'R''$; CH(OR')(OR''); $CO_2R'$; einem wahl-

weise mindestens monosubstituierten Alkylaryl, wobei Alkyl wahlweise durch OH substituiert ist; einem wahlweise mindestens monosubstituierten Alkylheterocyclyl, wobei Alkyl wahlweise durch OH substituiert ist oder eine C(O)-Ketogruppe bildet; oder einem wahlweise mindestens monosubstituierten Alkylcycloalkyl, wobei Alkyl wahlweise durch OH substituiert ist;
wobei
R' H oder eine wahlweise mindestens monosubstituierte, lineare oder verzweigte aliphatische $C_{1-6}$-Gruppe ist;
R" eine wahlweise mindestens monosubstituierte, lineare oder verzweigte aliphatische $C_{1-6}$-Gruppe ist;
R"' H; eine wahlweise mindestens monosubstituierte, lineare oder verzweigte aliphatische $C_{1-6}$-Gruppe; oder ein wahlweise mindestens monosubstituiertes Aryl, ein wahlweise mindestens monosubstituiertes Heterocyclyl oder ein wahlweise mindestens monosubstituiertes Cycloalkyl ist;
r 0, 1 oder 2 ist;
wahlweise in Form eines der Stereoisomere, vorzugsweise Enantiomere oder Diastereomere, eines Racemats oder in Form einer Mischung von mindestens zwei der Stereoisomere, vorzugsweise Enantiomere und/oder Diastereomere, in einem beliebigen Mischverhältnis, oder eines entsprechenden Salzes davon oder eines entsprechenden Solvats davon;
mit der Maßgabe, dass, wenn $R^2$ und $R^3$ beide Wasserstoff sind, $R^1$ nicht Methyl sein kann.

3. Verbindung nach einem der Ansprüche 1 oder 2, **dadurch gekennzeichnet, dass** $R^1$ ausgewählt ist aus H; einer wahlweise monosubstituierten, linearen oder verzweigten aliphatischen $C_{1-18}$-Gruppe; einem wahlweise mindestens monosubstituierten Alkylaryl; einem wahlweise mindestens monosubstituierten Alkylheterocyclyl; oder einem wahlweise mindestens monosubstituierten Alkylcycloalkyl.

4. Verbindung nach einem der Ansprüche 1 bis 3, **dadurch gekennzeichnet, dass** $R^2$ ausgewählt ist aus Wasserstoff; einer wahlweise mindestens monosubstituierten, linearen oder verzweigten aliphatischen $C_{1-6}$-Gruppe; oder OR, wobei R H oder eine wahlweise mindestens monosubstituierte, lineare oder verzweigte aliphatische $C_{1-6}$-Gruppe ist; $(CH_2)_q$-CN, $(CH_2)_q$-NH-Benzyl; $(CH_2)_q$-$N(R^{22})_2$ ; $(CH_2)_q$-C(O)-$N(R^{22})_2$; $(CH_2)_q$-C(O)-$R^{22}$ oder $(CH_2)_q$-C(O)-O-$R^{22}$, wobei q 0 oder 1 ist und $R^{22}$ H oder eine wahlweise mindestens monosubstituierte, lineare oder verzweigte aliphatische $C_{1-6}$-Gruppe ist; und/oder
**dadurch gekennzeichnet, dass** $R^3$ ausgewählt ist aus Wasserstoff; Halogen; CN; CH(=NOH); einer wahlweise mindestens monosubstituierten, linearen oder verzweigten aliphatischen $C_{1-10}$-Gruppe; $CH_2$-$R^{33}$ ; C(O)-$R^{33}$; C(O) O-$R^{33}$; CHOH-$R^{33}$; $CH_2N(R^{33})_2$, wobei $R^{33}$ H oder eine wahlweise mindestens monosubstituierte, lineare oder verzweigte aliphatische $C_{1-6}$-Gruppe ist; oder einem wahlweise mindestens monosubstituierten Aryl.

5. Verbindung nach Anspruch 1, **dadurch gekennzeichnet, dass** m und n ausgewählt sind aus 1 oder 2 und m+n ausgewählt sind aus 3 oder 4.

6. Verbindung nach einem der Ansprüche 1 oder 2 mit der allgemeinen Formel Id,

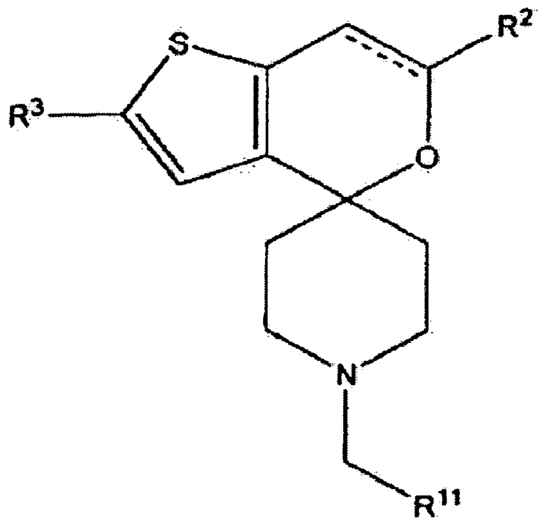

(Id)

wobei

die Bindung mit der gepunkteten Linie ....... entweder eine Doppel- oder eine Einfachbindung ist;

$R^{11}$ ausgewählt ist aus einer wahlweise mindestens monosubstituierten, linearen oder verzweigten aliphatischen $C_{1-9}$-Gruppe; einem wahlweise mindestens monosubstituierten Aryl oder Alkylaryl; einem wahlweise mindestens monosubstituierten Heterocyclyl oder Alkylheterocyclyl; einem wahlweise mindestens monosubstituierten Cycloalkyl oder Alkylcycloalkyl;

$R^2$ ausgewählt ist aus Wasserstoff; einer wahlweise mindestens monosubstituierten, linearen oder verzweigten aliphatischen $C_{1-6}$-Gruppe; oder OR, wobei R H oder eine wahlweise mindestens monosubstituierte, lineare oder verzweigte aliphatische $C_{1-6}$-Gruppe ist; $(CH_2)_q$-CN, $(CH_2)_q$-NH-Benzyl; $(CH_2)_q$-$N(R^{22})_2$; $(CH_2)_q$-C(O)-$N(R^{22})_2$; $(CH_2)_q$-C(O)-$R^{22}$ oder $(CH_2)_q$-C(O)-O-$R^{22}$, wobei q 0 oder 1 ist und $R^{22}$ H oder eine wahlweise mindestens monosubstituierte, lineare oder verzweigte aliphatische $C_{1-6}$-Gruppe ist;

$R^3$ ausgewählt ist aus Wasserstoff; Halogen; CN; CH(=NOH); einer wahlweise mindestens monosubstituierten, linearen oder verzweigten aliphatischen $C_{1-10}$-Gruppe; $CH_2$-$R^{33}$; C(O)-$R^{33}$; C(O)O-$R^{33}$; CHOH-$R^{33}$; $CH_2N(R^{33})_2$, wobei $R^{33}$ H oder eine wahlweise mindestens monosubstituierte, lineare oder verzweigte aliphatische $C_{1-6}$-Gruppe ist; oder einem wahlweise mindestens monosubstituierten Aryl;

wahlweise in Form eines der Stereoisomere, vorzugsweise Enantiomere oder Diastereomere, eines Racemats oder in Form einer Mischung von mindestens zwei der Stereoisomere, vorzugsweise Enantiomere und/oder Diastereomere, in einem beliebigen Mischverhältnis, oder eines entsprechenden Salzes davon oder eines entsprechenden Solvats davon.

**7.** Verbindung nach Anspruch 6, **dadurch gekennzeichnet, dass** $R^{11}$ ausgewählt ist aus einer wahlweise mindestens monosubstituierten, linearen oder verzweigten aliphatischen $C_{1-9}$-Gruppe; einem wahlweise mindestens monosubstituierten Aryl oder Alkylaryl; einem wahlweise mindestens monosubstituierten Heterocyclyl; oder einem wahlweise mindestens monosubstituierten Cycloalkyl; vorzugsweise Phenyl, p-Methoxyphenyl, Cyclohexyl, Thiophen, Benzyl; verzweigtes oder lineares $C_{3-7}$-Alkyl; oder verzweigtes oder lineares $C_{3-7}$-Alkenyl ist; und/oder

**dadurch gekennzeichnet, dass** $R^2$ ausgewählt ist aus Wasserstoff; einer unsubstituierten oder durch mindestens einen von OH, F oder Cl substituierten, linearen oder verzweigten aliphatischen $C_{1-6}$-Gruppe; oder OR, wobei R H oder CH3 ist; $(CH_2)_q$-CN, $(CH_2)_q$-NH-Benzyl; $(CH_2)_q$-$N(R^{22})_2$; $(CH_2)_q$-C(O)-$N(R^{22})_2$; $(CH_2)_q$-C(O)-$R^{22}$ oder $(CH_2)$ qC(O)-O-$R^{22}$, wobei q 0 oder 1 ist und $R^{22}$ H, $CH_3$ oder $C_2H_5$ ist; und/oder

**dadurch gekennzeichnet, dass** $R^3$ ausgewählt ist aus Wasserstoff; Halogen; CN; CH(=NOH); einer linearen oder verzweigten, unsubstituierten oder durch mindestens einen von OH, Cl oder F substituierten aliphatischen $C_{1-6}$-Gruppe; $CH_2$-$R^{33}$; C(O)-$R^{33}$; C(O)O-$R^{33}$; CHOH-$R^{33}$; $CH_2N(R^{33})_2$, wobei $R^{33}$ H ist; $CH_3$; $C_2H_5$; oder Phenyl.

**8.** Verbindung nach Anspruch 1, **dadurch gekennzeichnet, dass** die Verbindung ausgewählt ist aus

- 6'-Methoxy-6',7'-dihydrospiro[piperidin-4,4'-thieno[3,2-c]pyran];
- 1-Benzyl-6'-methoxy-6',7'-dihydrospiro[piperidin-4,4'-thieno[3,2-c]pyran];

6'-Methoxy-1-(2-phenylethyl)-6',7'-dihydrospiro[piperidin-4,4'-thieno[3,2- c]pyran];
- 1-(Cyclohexylmethyl)-6'-Methoxy-6',7'-dihydrospiro[piperidin-4,4'-thieno[3,2-c]pyran];

6'-Methoxy-1-(3-methylbutyl)-6',7'-dihydrospiro[piperidin-4,4'-thieno[3,2-c]pyran];
- 6'-Methoxy-1-(3-methylbut-2-enyl)-6',7'-dihydrospiro[piperidin-4,4'-thieno[3,2-c]pyran];
- 1-Butyl-6'-methoxy-6',7'-dihydrospiro[piperidin-4,4'-thieno[3,2-c]pyran];

6'-Methoxy-1-pentyl-6',7'-dihydrospiro[piperidin-4,4'-thieno[3,2-c]pyran];
6'-Methoxy-1-octyl-6',7'-dihydrospiro[piperidin-4,4'-thieno[3,2-c]pyran];
3'-Methoxy-1-(3-phenylpropyl)-6',7'-dihydrospiro[piperidin-4,4'-thieno[3,2-c]pyran];
3'-Methoxy-1-(4-phenylbutyl)-6',7'-dihydrospiro[piperidin-4,4'-thieno[3,2-c]pyran];
1-(4-Fluorbenzyl)-6'-Methoxy-6',7'-dihydrospiro[piperidin-4,4'-thieno[3,2-c]pyran];
6'-Methoxy-1-(4-methoxybenzyl)-6',7'-dihydrospiro[piperidin-4,4'-thieno[3,2-c]pyran];
6'-Methoxy-1-(thien-2-ylmethyl)-6',7'-dihydrospiro[piperidin-4,4'-thieno[3,2-c]pyran];
1-Benzylspiro[piperidin-4,4'-thieno[3,2-c]pyran];
1-Benzyl-6',7'-dihydrospiro[piperidin-4,4'-thieno[3,2-c]pyran]-6'-carbonitril;
{1-Benzyl-6',7'-dihyrospiro[piperidin-4,4'-thieno[3,2-c]pyran]-6'-yl}acetonitril;
1-Benzyl-6',7'-dihydrospiro[piperidin-4,4'-thieno[3,2-c]pyran];
1-Benzyl-6',7'-dihydrospiro[piperidin-4,4'-thieno[3,2-c[pyran]-6'-ol
1-(Cyclohexylmethyl)spiro[piperidin-4,4'-thieno[3,2-c]pyrano]
1-(Cyclohexylmethyl)-6',7'-dihydrospiro[piperidin-4,4'-thieno[3,2-c]pyrano]-6'-carbonitril
1-(1-Benzyl-6',7'-dihydrospiro[piperidin-4,4'-thieno[3,2-c]pyrano]-6'-yl)aceton
Methyl-2-(1-benzyl-6',7'-dihydrospiro[piperidin-4,4'-thieno[3,2-c]pyrano]-6'-yl)-acetat

Ethyl-2-(1-benzyl-6',7'-dihydrospiro[piperidin-4,4'-thieno[3,2-c]pyrano]-6'-yl)acetat
1-(Cyclohexylmethyl)-6',7'-dihydrospiro[piperidin-4,4'-thieno[3,2-c]pyrano]
2-(1-Benzyl-6',7'-dihydrospiro[piperidin-4,4'-thieno[3,2-c]pyrano]-6'-yl)ethanol
1-Benzyl-6',7'-dihydrospiro[piperidin-4,4'-thieno[3,2-c]pyrano]-6'-carboxamid
1-Benzyl-6',7'-dihydrospiro[piperidin-4,4'-thieno[3,2-c]pyrano]-6'-carbaldehyd
(1-Benzyl-6',7'-dihydrospiro[piperidin-4,4'-thieno[3,2-c]pyrano]-6'-yl)methanamin
Methyl-1-benzyl-6',7'-dihydrospiro[piperidin-4,4'-thieno[3,2-c]pyrano]-6'-carboxylat
Etyhl-1-benzyl-6',7'-dihydrospiro[piperidin-4,4'-thieno[3,2-c]pyrano]-6'-carboxylat
(1-Benzyl-6',7'-dihydrospiro[piperidin-4,4'-thieno[3,2-c]pyrano]-6'-yl-methanol
(1-Benzyl-6',7'-dihydrospiro[piperidin-4,4'-thieno[3,2-c]pyrano]-6'-yl-N,N-dimethylmethanamin
(1-Benzyl-6',7'-dihydrospiro[piperidin-4,4'-thieno[3,2-c]pyrano]-6'-yl)-N-benzyl-methanamin
(1-Benzyl-6'-methoxy-6',7'-dihydrospiro[piperidin-4,4'-thieno[3,2-c]pyrano]-2-carbaldehyddimethylacetal
1-Benzyl-2'-chloro-6'-methoxy-6',7'-dihydrospiro[piperidin-4,4'-thieno[3,2-c]pyrano]
1,2'-Dibenzyl-6'-methoxy-6',7'-dihydrospiro[piperidin-4,4'-thieno[3,2-c]pyrano]
(1-Benzyl-6'-methoxy-6',7'-dihydrospiro[piperidin-4,4'-thieno[3,2-c]pyrano]-2-carbaldehyd
(1-Benzyl-6'-methoxy-6',7'-dihydrospiro[piperidin-4,4'-thieno[3,2-c]pyrano]-2'-yl)-methanol
(1-Benzyl-6'-methoxy-6',7'-dihydrospiro[piperidin-4,4'-thieno[3,2-c]pyrano]-2'-yl)-N,N-dimethylmethanamin
(E/Z)-1-Benzyl-6'-methoxy-6',7'-dihydrospiro[piperidin-4,4'-thieno[3,2-c]pyrano]-2-carbaldehydoxim
1-Benzyl-6'-methoxy-6',7'-dihydrospiro[piperidin-4,4'-thieno[3,2-c]pyrano]-2-carbonitril
- Methyl-1-benzyl-6'-methoxy-6',7'-dihydrospiro[piperidin-4,4'-thieno[3,2-c]pyrano]-2-carboxylat
- 1-(1-Benzyl-6'-methoxy-6',7'-dihydrospiro[piperidin-4,4'-thieno[3,2-c]pyrano]-2-yl)-1-phenylmethanol; oder
- 1-(1-Benzyl-6'-methoxy-6',7'-dihydrospiro[piperidin-4,4'-thieno[3,2-c]pyrano]-2-yl)-1-phenylmethanon;

wahlweise in Form eines der Stereoisomere, vorzugsweise Enantiomere oder Diastereomere, eines Racemats oder in Form einer Mischung von mindestens zwei der Stereoisomere, vorzugsweise Enantiomere und/oder Diastereomere, in einem beliebigen Mischverhältnis, oder eines entsprechenden Salzes davon oder eines entsprechenden Solvats davon.

**9.** Verbindung nach Anspruch 1 mit der allgemeinen Formel Ib,

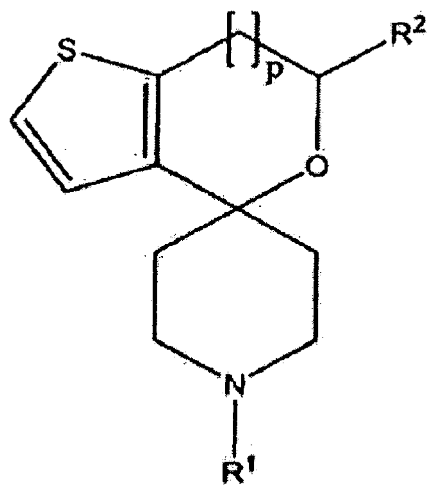

**(Ib)**

wobei

p ausgewählt ist aus 0 oder 1;

$R^1$ ausgewählt ist aus Wasserstoff; linearem oder verzweigtem $C_{1-10}$-Alkyl; linearem oder verzweigtem $C_{2-8}$-Alkenyl; wahlweise mindestens monosubstituiertem $C_{1-6}$-Alkylaryl; wahlweise mindestens monosubstituiertem $C_{1-6}$-Alkylheterocyclyl; oder wahlweise mindestens monosubstituiertem $C_{1-6}$-Alkyl-$C_{4-8}$-Cycloalkyl;

$R^2$ ausgewählt ist aus H; CN; $CH_2CN$; OH oder einer linearen oder verzweigten $OC_{1-4}$-Alkylgruppe;

wahlweise in Form eines der Stereoisomere, vorzugsweise Enantiomere oder Diastereomere, eines Racemats oder in Form einer Mischung von mindestens zwei der Stereoisomere, vorzugsweise Enantiomere und/oder Diastereomere, in einem beliebigen Mischverhältnis, oder eines entsprechenden Salzes davon oder eines entsprechenden

Solvats davon.

**10.** Verfahren zur Herstellung einer Verbindung nach Anspruch 1, wobei eine Verbindung der Formel (III)

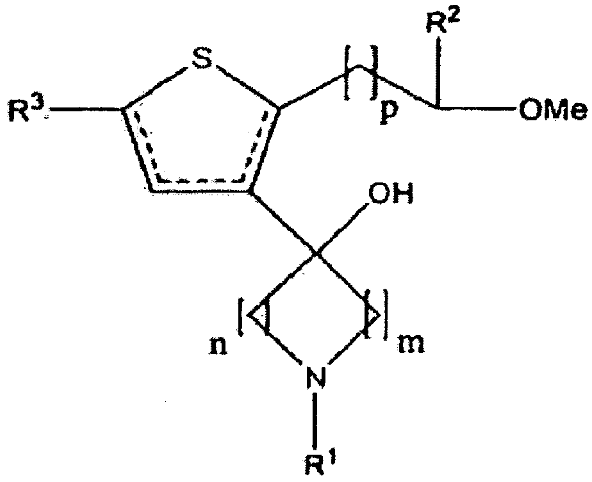

(III)

wobei $R^1$, $R^2$, $R^3$, m, n und p wie in Anspruch 1 definiert sind, mit p-Toluolsulfonsäure umgesetzt wird, um eine Verbindung der Formel (I) zu bilden;
wobei vorzugsweise

- $R^2$ $OCH_3$ ist; und/oder
- $R^3$ H ist.

**11.** Verfahren zur Herstellung einer Verbindung nach Anspruch 9 gemäß Formel Ib, wobei eine Verbindung der Formel (IIIb)

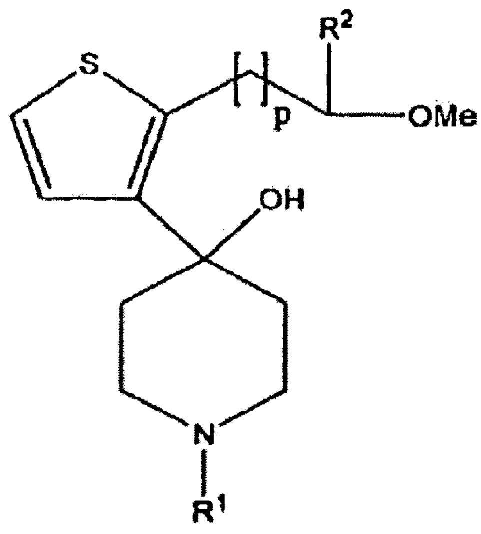

(IIIb)

wobei $R^1$, $R^2$, und p wie in Anspruch 1 definiert sind, mit p-Toluolsulfonsäure umgesetzt wird, um eine Verbindung der Formel (Ib) zu bilden;

wobei vorzugsweise

- $R^2$ $OCH_3$ ist; und/oder
- p 1 ist.

**12.** Verfahren nach Anspruch 10, wobei als ein nächster Schritt eine Verbindung der Formel (I), wobei $R^3$ C(O)OR' ist, mit einer alkalischen Lösung in Wasser umgesetzt wird, um eine Verbindung der Formel (I) zu bilden, wobei $R^1$ H ist.

**13.** Pharmazeutische Zusammensetzung, umfassend eine Verbindung nach einem der Ansprüche 1 bis 9 oder ein pharmazeutisch annehmbares Salz oder Solvat davon und einen pharmazeutisch annehmbaren Trägerstoff, Adjuvans oder Trägersubstanz.

**14.** Verwendung einer Verbindung nach einem der Ansprüche 1 bis 9 bei der Herstellung eines Arzneimittels für die Behandlung oder Prophylaxe einer durch Sigma-Rezeptor vermittelten Krankheit oder Zustands.

**15.** Verwendung nach Anspruch 14, wobei die Krankheit Durchfall, Lipoproteinstörungen, metabolisches Syndrom, Behandlung von erhöhten Triglyceridwerten, Chylomikronämie, Hyperlipoproteinämie; Hyperlipidämie, insbesondere gemischte Hyperlipidämie; Hypercholesterinämie, Dysbetalipoproteinämie, Hypertriglyceridämie, einschließlich der sporadischen und familiären Störung (angeborene Hypertriglyceridämie), Migräne, Fettsucht, Arthritis, Bluthochdruck, Arrhythmie, Geschwür, Lern-, Gedächtnis- und Aufmerksamkeitsdefizite, kognitive Störungen, neurodegenerative Krankheiten, demyelinisierende Krankheiten, Abhängigkeit von Drogen und chemischen Substanzen, einschließlich Kokain, Amphetamin, Ethanol und Nikotin, tardive Dyskinesie, ischämischer Schlaganfall, Epilepsie, Schlaganfall, Depression, Stress, psychotischer Zustand, Schizophrenie; Entzündung, Autoimmunkrankheiten oder Krebs ist; oder
wobei die Krankheit Schmerz ist, insbesondere neuropathischer Schmerz, Entzündungsschmerz oder andere Schmerzzustände, Allodynie und/oder Hyperalgesie, insbesondere mechanische Allodynie.

## Revendications

**1.** Composé de formule générale (I) ou (Ia)

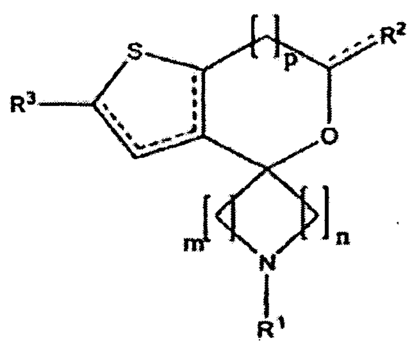

(I)

ou

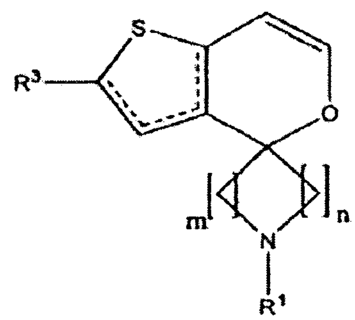

(Ia)

dans lesquelles
m est choisi parmi 1, 2 ou 3, n est choisi parmi 1, 2 ou 3, et m + n est égal à 3, 4 ou 5 ;
p est choisi parmi 0 ou 1 ;
les lignes pointillées ........ sont des doubles ou simples liaisons ;
$R^1$ est choisi parmi un atome d'hydrogène ; un groupe aliphatique en $C_1$-$C_{18}$ linéaire ou ramifié, facultativement au moins monosubstitué ; un groupe aryle facultativement au moins monosubstitué ; un groupe hétérocyclyle facultativement au moins monosubstitué ; un groupe cycloalkyle facultativement au moins monosubstitué ; un groupe alkylaryle facultativement au moins monosubstitué ; un groupe alkylhétérocyclyle facultativement au moins monosubstitué ; ou un groupe alkylcycloalkyle facultativement au moins monosubstitué ;
$R^2$ est choisi parmi un atome d'hydrogène ; un groupe aliphatique en $C_1$-$C_6$ linéaire ou ramifié, facultativement au moins monosubstitué ; $(CH_2)_qCN$ où q est 0, 1, 2, 3 ou 4 ; O-R où R est H ou un groupe aliphatique en $C_1$-$C_6$ linéaire

ou ramifié, facultativement au moins monosubstitué ; $(CH_2)_sCOR^*$ ; $(CH_2)_sNR^*R^{**}$ ; CONR*R** ; $(CH_2)_sCO2R^*$; CH=NOR* ; $(CH_2)_sOR^*$ ;

où

R* est H ; ou un groupe aliphatique en $C_1$-$C_6$ linéaire ou ramifié, facultativement au moins monosubstitué ;

R** est un groupe aliphatique en $C_1$-$C_6$ linéaire ou ramifié, facultativement au moins monosubstitué ; ou un groupe aryle ou alkylaryle facultativement au moins monosubstitué ;

s est 0, 1 ou 2 ;

$R^3$ est choisi parmi un atome d'hydrogène ; un atome d'halogène ; un groupe aliphatique en $C_1$-$C_{18}$ linéaire ou ramifié, facultativement au moins monosubstitué ; CN ; CH (=NOH) ; $NO_2$ ; $SO_3H$ ; COR"' ; $(CH_2)_rNR'R''$ ; CH(OR') (OR") ; $CO_2R'$ ; un groupe alkylaryle facultativement au moins monosubstitué avec un groupe alkyle facultativement substitué par OH ; un groupe alkylhétérocyclyle facultativement au moins monosubstitué avec un groupe alkyle facultativement substitué par OH ou formant un groupe C(O)céto ; ou un groupe alkylcycloalkyle facultativement au moins monosubstitué avec un groupe alkyle facultativement substitué par OH ;

où

R' est H ; ou un groupe aliphatique en $C_1$-$C_6$ linéaire ou ramifié, facultativement au moins monosubstitué ;

R" est un groupe aliphatique en $C_1$-$C_6$ linéaire ou ramifié, facultativement au moins monosubstitué ;

R"' est H ; un groupe aliphatique en $C_1$-$C_6$ linéaire ou ramifié, facultativement au moins monosubstitué ; ou un groupe aryle facultativement au moins monosubstitué, un groupe hétérocyclyle facultativement au moins monosubstitué ou un groupe cycloalkyle facultativement au moins monosubstitué ;

r est 0, 1 ou 2 ;

facultativement sous la forme d'un isomère choisi parmi les stéréoisomères, de préférence les énantiomères ou les diastéréoisomères, d'un racémate, ou sous la forme d'un mélange d'au moins deux des stéréoisomères, de préférence des énantiomères et/ou des diastéréoisomères, selon n'importe quel rapport de mélange, ou d'un sel correspondant de ceux-ci, ou d'un solvate correspondant de ceux-ci ;

à condition que si $R^2$ et $R^3$ sont tous les deux un atome d'hydrogène, $R^1$ ne puisse pas être un groupe méthyle.

**2.** Composé selon la revendication 1, répondant à la formule générale (Ic)

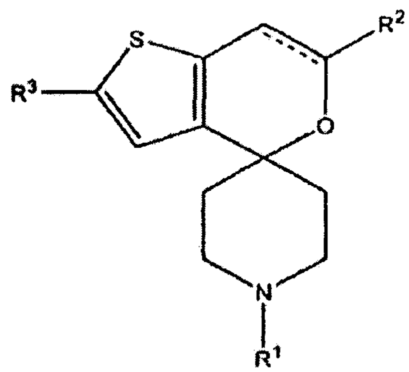

**(Ic)**

dans laquelle

la ligne pointillée ········ est une double ou simple liaison ;

$R^1$ est choisi parmi un atome d'hydrogène ; un groupe aliphatique en $C_1$-$C_{18}$ linéaire ou ramifié, facultativement au moins monosubstitué ; un groupe aryle facultativement au moins monosubstitué ; un groupe hétérocyclyle facultativement au moins monosubstitué ; un groupe cycloalkyle facultativement au moins monosubstitué ; un groupe alkylaryle facultativement au moins monosubstitué ; un groupe alkylhétérocyclyle facultativement au moins monosubstitué ; ou un groupe alkylcycloalkyle facultativement au moins monosubstitué ;

$R^2$ est choisi parmi un atome d'hydrogène ; un groupe aliphatique en $C_1$-$C_6$ linéaire ou ramifié, facultativement au moins monosubstitué ; $(CH_2)_qCN$ où q est 0, 1, 2, 3 ou 4 ; O-R où R est H ou un groupe aliphatique en $C_1$-$C_6$ linéaire ou ramifié, facultativement au moins monosubstitué ;

$(CH_2)_sCOR^*$ ; $(CH_2)_sNR^*R^{**}$; CONR*R** ; $(CH_2)_sCO_2R^*$ ; CH=NOR* ; $(CH_2)_sOR^*$ ;

où

R* est H ; ou un groupe aliphatique en $C_1$-$C_6$ linéaire ou ramifié, facultativement au moins monosubstitué ;

R** est un groupe aliphatique en $C_1$-$C_6$ linéaire ou ramifié, facultativement au moins monosubstitué ; ou un groupe

aryle ou alkylaryle facultativement au moins monosubstitué ;
s est 0, 1 ou 2 ;
$R^3$ est choisi parmi un atome d'hydrogène ; un atome d'halogène ; un groupe aliphatique en $C_1$-$C_{18}$ linéaire ou ramifié, facultativement au moins monosubstitué ; CN ; CH(=NOH) ; $NO_2$ ; $SO_3H$ ; COR"' ; $(CH_2)_r$NR'R" ; CH(OR')(OR") ; $CO_2$R' ; un groupe alkylaryle facultativement au moins monosubstitué avec un groupe alkyle facultativement substitué par OH ; un groupe alkylhétérocyclyle facultativement au moins monosubstitué avec un groupe alkyle facultativement substitué par OH ou formant un groupe C(O)céto ; ou un groupe alkylcycloalkyle facultativement au moins monosubstitué avec un groupe alkyle facultativement substitué par OH ;
où
R' est H ; ou un groupe aliphatique en $C_1$-$C_6$ linéaire ou ramifié, facultativement au moins monosubstitué ;
R" est un groupe aliphatique en $C_1$-$C_6$ linéaire ou ramifié, facultativement au moins monosubstitué ;
R"' est H ; un groupe aliphatique en $C_1$-$C_6$ linéaire ou ramifié, facultativement au moins monosubstitué ; ou un groupe aryle facultativement au moins monosubstitué, un groupe hétérocyclyle facultativement au moins monosubstitué ou un groupe cycloalkyle facultativement au moins monosubstitué ;
r est 0, 1 ou 2 ;
facultativement sous la forme d'un isomère choisi parmi les stéréoisomères, de préférence les énantiomères ou les diastéréoisomères, d'un racémate, ou sous la forme d'un mélange d'au moins deux des stéréoisomères, de préférence des énantiomères et/ou des diastéréoisomères, selon n'importe quel rapport de mélange, ou d'un sel correspondant de ceux-ci, ou d'un solvate correspondant de ceux-ci ;
à condition que si $R^2$ et $R^3$ sont tous les deux un atome d'hydrogène, $R^1$ ne puisse pas être un groupe méthyle.

**3.** Composé selon la revendication 1 ou 2, **caractérisé en ce que** $R^1$ est choisi parmi H ; un groupe aliphatique en $C_1$-$C_{18}$ linéaire ou ramifié, facultativement au moins monosubstitué ; un groupe alkylaryle facultativement au moins monosubstitué ; un groupe alkylhétérocyclyle facultativement au moins monosubstitué ; ou un groupe alkylcycloalkyle facultativement au moins monosubstitué.

**4.** Composé selon l'une quelconques des revendications 1 à 3, **caractérisé en ce que** $R^2$ est choisi parmi un atome d'hydrogène ; un groupe aliphatique en $C_1$-$C_6$ linéaire ou ramifié, facultativement au moins monosubstitué ; ou OR où R est H ou un groupe aliphatique en $C_1$-$C_6$ linéaire ou ramifié, facultativement au moins monosubstitué ; $(CH_2)_q$-CN ; $(CH_2)_q$-NH-benzyle ; $(CH_2)_q$-N$(R^{22})_2$ ; $(CH_2)_q$-C(O)-N$(R^{22})_2$ ; $(CH_2)_q$-C(O)-$R^{22}$ ou $(CH_2)_q$-C(O)-O-$R^{22}$, où q est 0 ou 1 et $R^{22}$ est H ; ou un groupe aliphatique en $C_1$-$C_6$ linéaire ou ramifié, facultativement au moins monosubstitué ; et/ou
**caractérisé en ce que** $R^3$ est choisi parmi un atome d'hydrogène ; un atome d'halogène ; CN ; CH(=NOH) ; un groupe aliphatique en $C_1$-$C_{10}$ linéaire ou ramifié, facultativement au moins monosubstitué ; $CH_2$-$R^{33}$ ; C(O)-$R^{33}$ ; C(O)O-$R^{33}$ ; CHOH-$R^{33}$ ; $CH_2N(R^{33})_2$ où $R^{33}$ est H ; ou un groupe aliphatique en $C_1$-$C_6$ linéaire ou ramifié, facultativement au moins monosubstitué ; ou un groupe aryle facultativement au moins monosubstitué.

**5.** Composé selon la revendication 1, **caractérisé en ce que** n et m sont choisis parmi 1 ou 2 et m + n est choisi parmi 3 ou 4.

**6.** Composé selon la revendication 1 ou 2, répondant à la formule générale (Id)

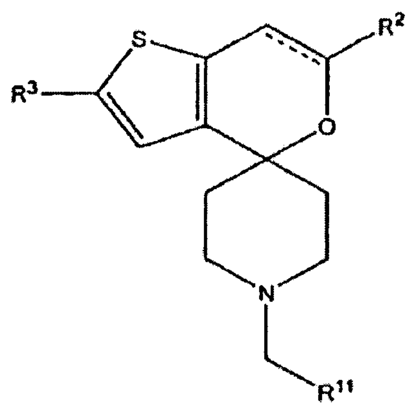

(Id)

dans laquelle
la ligne pointillée ⋯⋯ est une double ou simple liaison ;
$R^1$ est choisi parmi un groupe aliphatique en $C_1$-$C_9$ linéaire ou ramifié, facultativement au moins monosubstitué ; un groupe aryle ou alkylaryle facultativement au moins monosubstitué ; un groupe hétérocyclyle ou alkylhétérocyclyle facultativement au moins monosubstitué ; un groupe cycloalkyle ou alkylcycloalkyle facultativement au moins monosubstitué ;
$R^2$ est choisi parmi un atome d'hydrogène ; un groupe aliphatique en $C_1$-$C_6$ linéaire ou ramifié, facultativement au moins monosubstitué ; ou OR où R est H ou un groupe aliphatique en $C_1$-$C_6$ linéaire ou ramifié, facultativement au moins monosubstitué ; $(CH_2)_q$-CN ; $(CH_2)_q$-NH-benzyle ; $(CH_2)_q$-$N(R^{22})_2$; $(CH_2)_q$-C(O)-$N(R^{22})_2$, $(CH_2)_q$-C(O)-$R^{22}$ ou $(CH_2)_q$-C(O)-O-$R^{22}$, où q est 0 ou 1 et $R^{22}$ est H ; ou un groupe aliphatique en $C_1$-$C_6$ linéaire ou ramifié, facultativement au moins monosubstitué
$R^3$ est choisi parmi un atome d'hydrogène ; un atome d'halogène ; CN ; CH(=NOH) ; un groupe aliphatique en $C_1$-$C_{10}$ linéaire ou ramifié, facultativement au moins monosubstitué ; $CH_2$-$R^{33}$; C(O)-$R^{33}$ ; C(O)O-$R^{33}$ ; CHOH-$R^{13}$ ; $CH_2N(R^{33})_2$ où $R^{33}$ est H ; un groupe aliphatique en $C_1$-$C_6$ linéaire ou ramifié, facultativement au moins monosubstitué ; ou un groupe aryle facultativement au moins monosubstitué ;
facultativement sous la forme d'un isomère choisi parmi les stéréoisomères, de préférence les énantiomères ou les diastéréoisomères, d'un racémate, ou sous la forme d'un mélange d'au moins deux des stéréoisomères, de préférence des énantiomères et/ou des diastéréoisomères, selon n'importe quel rapport de mélange, ou d'un sel correspondant de ceux-ci, ou d'un solvate correspondant de ceux-ci.

**7.** Composé selon la revendication 6, **caractérisé en ce que** $R^{11}$ est choisi parmi un groupe aliphatique en $C_1$-$C_9$ linéaire ou ramifié, facultativement au moins monosubstitué ; un groupe aryle ou alkylaryle facultativement au moins monosubstitué ; un groupe hétérocyclyle facultativement au moins monosubstitué ; ou un groupe cycloalkyle facultativement au moins monosubstitué ; de préférence un groupe phényle, p-méthoxyphényle, cyclohexyle, thiophène, benzyle ; un groupe alkyle en $C_3$-$C_7$ linéaire ou ramifié ; ou un groupe alcényle en $C_3$-$C_7$ linéaire ou ramifié ; et/ou
**caractérisé en ce que** $R^2$ est choisi parmi un atome d'hydrogène ; un groupe aliphatique en $C_1$-$C_6$ linéaire ou ramifié, non substitué ou substitué par au moins l'un de OH, F ou Cl ; ou OR où R est H ou $CH_3$ ; $(CH_2)_q$-CN ; $(CH_2)_q$-NH-benzyle ; $(CH_2)_q$-$N(R^{22})_2$ ; $(CH_2)_q$-C(O)-$N(R^{22})_2$ ; $(CH_2)_q$-C(O)-$R^{22}$ ou $(CH_2)_q$-C(O)-O-$R^{22}$, où q est 0 ou 1 et $R^{22}$ est H ; $CH_3$ ou $C_2H_5$ ; et/ou
**caractérisé en ce que** $R^3$ est choisi parmi un atome d'hydrogène ; un atome d'halogène ; CN ; CH(=NOH) ; un groupe aliphatique en $C_1$-$C_6$ linéaire ou ramifié, non substitué ou substitué par au moins l'un de OH, F ou Cl ; $CH_2$-$R^{33}$ ; C(O)-$R^{33}$ ; C(O)O-$R^{33}$ ; CHOH-$R^{33}$ ; $CH_2N(R^{33})_2$ où $R^{33}$ est H ; $CH_3$ ; $C_2H_5$ ; ou un groupe phényle.

**8.** Composé selon la revendication 1, **caractérisé en ce que** le composé est choisi parmi

- 6'-méthoxy-6',7'-dihydrospiro[pipéridin-4,4'-thiéno[3,2-c]pyrane ;
- 1-benzyl-6'-méthoxy-6',7'-dihydrospiro[pipéridin-4,4'-thiério[3.2-c]pyrane] ;
- 6'-méthoxy-1-(2-phényléthyl)-6',7'-dihydrospiro[pipéridin-4,4'-thiéno[3.2-c]pyrane] ;
- 1-(cyclohexylméthyl)-6'-méthoxy-6',7'-dihydrospiro[pipéridin-4,4'-thiéno[3,2-c]pyrane] ;
- 6'-méthoxy-1-(3-méthylbutyl)-6',7'-dihydrospiro[pipéridin-4,4'-thiéno[3.2-c]pyrane] ;
- 6'-méthoxy-1-(3-méthylbut-2-ényl)-6',7'-dihydrospiro[pipéridin-4,4'-thiéno[3.2-c]pyrane] ;
- 1-butyl-6'-méthoxy-6',7'-dihydrospiro[pipéridin-4,4'-thiéno[3,2-c]pyrane] ;
- 6'-méthoxy-1-pentyl-6',7'-dihydrospiro[pipéridin-4,4'-thiéno[3.2-c]pyrane] ;
- 6'-méthoxy-1-octyl-6',7'-dihydrospiro[pipéridin-4,4'-thiéno[3.2-c]pyrane] ;
- 3'-méthoxy-1-(3-phénylpropyl)-6',7'-dihydrospiro[pipéridin-4,4'-thiéno[3,2-c]pyrane] ;
- 3'-méthoxy-1-(4-phénylbutyl)-6',7'-dihydrospiro[pipéridin-4,4'-thiéno[3.2-c]pyrane] ;
- 1-(4-fluorobenzyl)-6'-méthoxy-6',7'-dihydrospiro[pipéridin-4,4'-thiéno[3.2-c]pyrane] ;
- 6'-méthoxy-1-(4-méthoxybenzyl)-6',7'-dihydrospiro[pipéridin-4,4'-thiéno[3.2-c]pyrane] ;
- 6'-méthoxy-1-(thiényl-2-ylméthyl)-6',7'-dihydrospiro[pipéridin-4,4'-thiéno[3.2-c]pyrane] ;
- 1-benzylspiro[pipéridin-4,4'-thiéno[3,2-c]pyrane] ;
- 1-benzyl-6',7'-dihydrospiro[pipéridin-4,4'-thiéno[3,2-c]pyrano]-6'-carbonitrile ;
- {1-benzyl-6',7'-dihyrospiro[pipéridin-4,4'-thiéno[3,2-c]pyrano]-6'-yl}acétonitrile ;
- 1-benzyl-6',7'-dihydrospiro[pipéridin-4,4'-thiéno[3,2-c]pyrane] ;
- 1-benzyl-6',7'-dihydrospiro[pipéridin-4,4'-thiéno[3,2-c]pyran]-6'-ol ;
- 1-(cyclohexylméthyl)spiro[pipéridine-4,4'-thiéno[3,2-c]pyrane] ;
- 1-(cyclohexylméthyl)-6',7'-dihydrospiro[pipéridine-4,4'-thiéno[3,2-c]pyrano]-3'-carbonitrile ;
- 1-(1-benzyl-6',7'-dihydrospiro[pipéridine-4,4'-thiéno[3,2-c]pyrano]-6'-yl)acétone ;
- 2-(1-benzyl-6',7'-dihyrospiro[pipéridin-4,4'-thiéno[3,2-c]pyrano]-6'-yl)-acétate de méthyle ;

- 2-(1-benzyl-6',7'-dihyrospiro[pipéridine-4,4'-thiéno[3,2-c]pyrano]-6'-yl)acétate d'éthyle ;
- 1-(cyclohexylméthyl)-6',7'-dihydrospiro[pipéridine-4,4'-thiéno[3,2-c]pyrane] ;
- 2-(1-benzyl-6',7'-dihydrospiro[pipéridine-4,4'-thiéno[3,2-c]pyrano]-6'-yl)éthanol ;
- 1-benzyl-6',7'-dihydrospiro[pipéridine-4,4'-thiéno[3,2-c]pyrano]-6'-carboxamide ;
- 1-benzyl-6',7'-dihydrospiro[pipéridine-4,4'-thiéno[3.2-c]pyrano]-6'-carbaldéhyde ;
- (1-benzyl-6',7'-dihydrospiro[pipéridine-4,4'-thiéno[3.2-c]pyrano]-6'-yl)méthanamine ;
- 1-benzyl-6',7'-dihydrospiro[pipéridine-4,4'-thiéno[3,2-c]pyrano]-6'-carboxylate de méthyle ;
- 1-benzyl-6',7'-dihydrospiro[pipéridine-4,4'-thiéno[3,2-c]pyrano]-6'-carboxylate d'éthyle ;
- (1-benzyl-6',7'-dihydrospiro[pipéridine-4,4'-thiéno[3,2-c]pyrano]-6'-yl)méthanol ;
- (1-benzyl-6',7'-dihydrospiro[pipéridine-4,4'-thiéno[3,2-c]pyrano]-6'-yl)-N,N-diméthylméthanamine ;
- (1-benzyl-6',7'-dihydrospiro[pipéridine-4,4'-thiéno[3,2-c]pyrano]-6'-yl)-N-benzyl-méthanamine ;
- (1-benzyl-6'-méthoxy-6',7'-dihydrospiro[pipéridine-4,4'-thiéno[3,2-c]pyrano]-2-carbaldéhyde-diméthylacétal ;
- 1-benzyl-2'-chloro-6'-méthoxy-6',7'-dihydrospiro[pipéridine-4,4'-thiéno[3.2-c]pyrane] ;
- 1,2'-dibenzyl-6'-méthoxy-6',7'-dihydrospiro[pipéridine-4,4'-thiéno[3,2-c]pyrane] ;
- (1-benzyl-6'-méthoxy-6',7'-dihydrospiro[pipéridine-4,4'-thiéno[3.2-c]pyrano]-2-carbaldéhyde ;
- (1-benzyl-6'-méthoxy-6',7'-dihydrospiro[pipéridine-4,4'-thiéno[3.2-c]pyrano]-2'-yl)-méthanol ;
- (1-benzyl-6'-méthoxy-6',7'-dihydrospiro[pipéridine-4,4'-thiéno[3.2-c]pyrano]-2'-yl)-N,N-diméthylméthanamine ;
- (E/Z)-1-benzyl-6'-méthoxy-6',7'-dihydrospiro[pipéridine-4,4'-thiéno[3,2-c]pyrano]-2-carbaldéhydoxime ;
- 1-benzyl-6'-méthoxy-6',7'-dihydrospiro[pipéridine-4,4'-thiéno[3.2-c]pyrano]-2-carbonitrile ;
- 1-benzyl-6'-méthoxy-6',7'-dihydrospiro[pipéridine-4,4'-thiéno[3.2-c]pyrano]-2-carboxylate de méthyle ;
- 1-(1-benzyl-6'-méthoxy-6',7'-dihydrospiro[pipéridine-4,4'-thiéno[3.2-c]pyrano]-2-yl)-1-phénylméthanol ; ou
- 1-(1-benzyl-6'-méthoxy-6',7'-dihydrospiro[pipéridine-4,4'-thiéno[3,2-c]pyrano]-2-yl)-1-phénylméthanone ;

facultativement sous la forme d'un isomère choisi parmi les stéréoisomères, de préférence les énantiomères ou les diastéréoisomères, d'un racémate, ou sous la forme d'un mélange d'au moins deux des stéréoisomères, de préférence des énantiomères et/ou des diastéréoisomères, selon n'importe quel rapport de mélange, ou d'un sel correspondant de ceux-ci, ou d'un solvate correspondant de ceux-ci.

**9.** Composé selon la revendication 1, répondant à la formule générale (Ib)

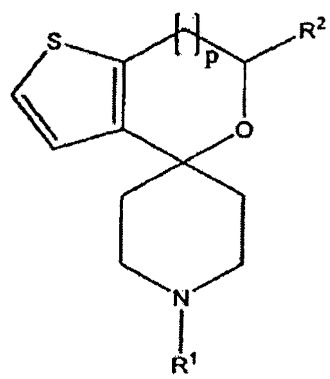

**(Ib)**

dans laquelle
p est choisi parmi 0 ou 1 ;
$R^1$ est choisi parmi un atome d'hydrogène ; un groupe alkyle en $C_1$-$C_{10}$ linéaire ou ramifié ; un groupe alcényle en $C_2$-$C_8$ linéaire ou ramifié ; un groupe (alkyle en $C_1$-$C_6$)aryle facultativement au moins monosubstitué ; un groupe (alkyle en $C_1$-$C_6$)hétérocyclyle facultativement au moins monosubstitué ; ou un groupe (alkyle en $C_1$-$C_6$)cycloalkyle en $C_4$-$C_8$ facultativement au moins monosubstitué ;
$R^2$ est choisi parmi H ; CN ; $CH_2CN$ ; OH ou un groupe O-alkyle en $C_1$-$C_4$ linéaire ou ramifié ;
facultativement sous la forme d'un isomère choisi parmi les stéréoisomères, de préférence les énantiomères ou les diastéréoisomères, d'un racémate, ou sous la forme d'un mélange d'au moins deux des stéréoisomères, de préférence des énantiomères et/ou des diastéréoisomères, selon n'importe quel rapport de mélange, ou d'un sel correspondant de ceux-ci, ou d'un solvate correspondant de ceux-ci.

**10.** Procédé de production d'un composé selon la revendication 1, dans lequel un composé de formule (III)

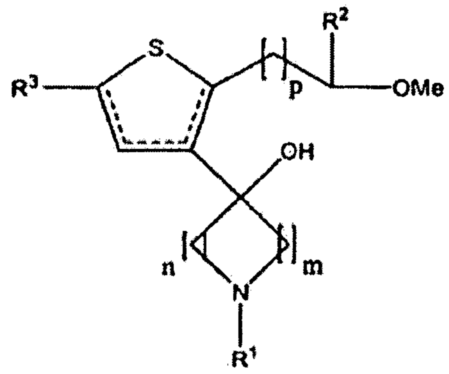

(III)

dans laquelle $R^1$, $R^2$, $R^3$, m, n et p sont tels que définis selon la revendication 1, est mis à réagir avec de l'acide p-toluolsulfonique pour former un composé de formule (I) ; de préférence
dans laquelle
$R^2$ est $OCH_3$ ; et/ou
$R^3$ est H.

**11.** Procédé de production d'un composé de formule (Ib) selon la revendication 9, dans lequel un composé de formule (IIIb)

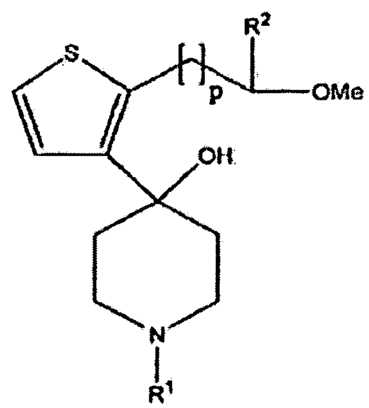

(IIIb)

dans laquelle $R^1$, $R^2$, et p sont tels que définis selon la revendication 1, est mis à réagir avec de l'acide p-toluolsulfonique pour former un composé de formule (Ib) ; de préférence
dans laquelle
$R^2$ est $OCH_3$ ; et/ou
p est 1.

**12.** Procédé selon la revendication 10, dans lequel comme étape suivante, un composé de formule (I) dans laquelle $R^3$ est C(O)OR' est mis à réagir avec une solution alcaline dans de l'eau pour former un composé de formule (I) dans laquelle $R^1$ est H.

**13.** Composition pharmaceutique comprenant un composé tel que défini selon l'une quelconque des revendications 1 à 9 ou un sel ou un solvate pharmaceutiquement acceptable de celui-ci, et un excipient, un adjuvant ou un véhicule pharmaceutiquement acceptable.

**14.** Utilisation d'un composé tel que défini selon l'une quelconque des revendications 1 à 9 dans la préparation d'un médicament pour le traitement ou la prophylaxie d'une maladie ou d'une pathologie médiée par le récepteur sigma.

**15.** Utilisation selon la revendication 14 dans laquelle la maladie est la diarrhée, les troubles lipoprotéiques, le syndrome métabolique, les taux élevés de triglycérides, la chylomicronémie, l'hyperlipoprotéinémie, l'hyperlipidémie, en particulier l'hyperlipidémie mixte, l'hypercholestérolémie, la dysbêtalipoprotéinémie, l'hypertriglycéridémie y compris le trouble sporadique et familial (hypertriglycéridémie héréditaire), la migraine, l'obésité, l'arthrite, l'hypertension, l'arythmie, l'ulcère, les troubles de l'apprentissage, de la mémoire et de l'attention, les troubles de la cognition, les maladies neurodégénératives, les maladies démyélinisantes, la dépendance aux médicaments et aux substances chimiques comprenant la cocaïne, les amphétamines, l'éthanol et la nicotine, la dyskinésie tardive, l'accident ischémique, l'épilepsie, l'accident vasculaire, la dépression, le stress, la pathologie psychotique, la schizophrénie, l'inflammation, les maladies auto-immunes ou le cancer ; ou
dans laquelle la maladie est la douleur, en particulier la douleur neuropathique, la douleur inflammatoire ou d'autres douleurs pathologiques, l'allodynie et/ou l'hyperalgésie, en particulier l'allodynie mécanique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2003035065 A
- WO 2007001939 A
- FR 2875230

### Non-patent literature cited in the description


- **WALKER, J.M. et al.** *Pharmacological Reviews,* 1990, vol. 42, 355
- **SNYDER, S.H. ; LARGENT, B.L.** *J. Neuropsychiatry,* 1989, vol. 1, 7
- **QUIRION, R. et al.** *Trends Pharmacol. Sci.,* 1992, vol. 13, 85-86
- **HANNER, M. et al.** *Proc. Natl. Acad Sci.,* 1996, vol. 93, 8072-8077
- **MAIER et al.** *J Med Chem,* 2002, vol. 45, 438-448
- **MAIER et al.** *J Med Chem,* 2002, vol. 45, 4923-4930
- **DEHAVEN-HUDKINS, D. L. ; L.C. FLEISSNER ; F. Y. FORD-RICE.** Characterization of the binding of [3H](+)pentazocine to σ recognition sites in guinea pig brain. *Eur. J. Pharmacol.,* 1992, vol. 227, 371-378
- **RONSISVALLE G ; PREZZAVENTO O ; MARRAZZO A ; VITTORIO F ; OUSQUET E ; DI TORO R ; SPAMPINATO S.** Synthesis and binding affinity of cis-(-)- and cis-(+)-N-ethyleneamino-N-nordeoxymetazocine and cis-(-)-N-normetazocine analogues at sigma1, sigma2 and kappa opioid receptors. *Eur J Pharm Sci.,* 2001, vol. 12 (3), 277-84